# EUROPEAN PATENT SPECIFICATION

(11) **EP 2 408 934 B1**
(45) Date of publication and mention of the grant of the patent: **12.11.2014**
(21) Application number: 10752999.2
(22) Date of filing: 18.03.2010
(51) Int. Cl.: C12Q 1/68, A61K 39/00, C07K 16/18, A61K 31/14, A61P 37/00, C12N 5/078

(54) **PEPTIDASE INHIBITOR 16 (PI16) AS A BIOMARKER FOR REGULATORY T (TREG) CELLS AND USES THEREOF**
PEPTIDASE-HEMMER 16 (PI16) ALS BIOMARKER FÜR T-REGULATORZELLEN (TREG-ZELLEN) UND VERWENDUNG
INHIBITEUR DE PEPTIDASE 16 (PI16) COMME BIOMARQUEUR DES CELLULES T RÉGULATRICES (TREG) ET SES UTILISATIONS

(30) Priority: 18.03.2009 US 161255 P
(43) Date of publication of application: 25.01.2012
(60) Divisional application: 14186006.4
(73) Proprietor: Adelaide Research & Innovation Pty Ltd., Adelaide, SA 5005 (AU); Transbio Ltd, Bundoora, Victoria 3083 (AU)
(72) Inventor: BARRY, Simon, Crafers West, S.A. 5152 (AU); SADLON, Timothy John, Mile End, S.A. 5031 (AU); PEDERSON, Stephen Martin, Unley Park, S.A. 5061 (AU); WILKINSON, Bridget Gabrielle, Somerton Park, S.A. 5044 (AU); KRUMBIEGEL, Doreen, Edwardstown, S.A. 5039 (AU); NICHOLSON, Ian Cameron, Highbury, S.A. 5089 (AU); ZOLA, Heddy, Evandale, S.A. 5069 (AU)
(74) Representative: Brasnett, Adrian Hugh
(86) International application number: PCT/AU2010/000311
(87) International publication number: WO 2010/105298

(56) References cited:
- WO-A1-03/093474
- WO-A2-2007/140472
- WO-A2-2007/140472
- IAN C. NICHOLSON ET AL: "PI16 is expressed by a subset of human memory Treg with enhanced migration to CCL17 and CCL20", CELLULAR IMMUNOLOGY, vol. 275, no. 1-2, 1 January 2012 (2012-01-01), pages 12-18, XP55033944, ISSN: 0008-8749, DOI: 10.1016/j.cellimm.2012.04.002
- T. J. SADLON ET AL: "Genome-Wide Identification of Human FOXP3 Target Genes in Natural Regulatory T Cells", THE JOURNAL OF IMMUNOLOGY, vol. 185, no. 2, 16 June 2010 (2010-06-16) , pages 1071-1081, XP55033945, ISSN: 0022-1767, DOI: 10.4049/jimmunol.1000082
- BRESATZ, S. ET AL.: 'Isolation, propagation and characterization of cord blood derived CD4+ CD25+ regulatory T cells' JOURNAL OF IMMUNOLOGICAL METHODS. vol. 327, 2007, pages 53 - 62, XP022266390
- LIU, H. ET AL.: 'CD4+CD25+ regulatory T cells in health and disease' CLINICAL AND EXPERIMENTAL PHARMACOLOGY AND PHYSIOLOGY. vol. 33, 2006, pages 519 - 524, XP003026411

## Description

### Field of the Invention

The present invention relates to nucleic acid or protein markers of regulatory T (Treg) cells and uses thereof.

### Sequence Listing

A Sequence Listing of nucleotide and amino acid sequences referenced in this application as "SEQ ID NO: 1-286" is submitted in computer readable form along with this application.

### Background of the Invention

T cells belong to a group of white blood cells known as lymphocytes, and play a central role in cell-mediated immunity and, to a lesser degree the adaptive immune response. Generally, T cells are distinguished from other lymphocytes (e.g., B cells and natural killer cells) by the presence of T cell receptors (TCRs). T cells have diverse roles, which are accomplished by differentiation of distinct populations of T cells, recognizable by discrete gene expression profiles. Exemplary T cell populations include Thelper cells (T_{H} cells), cytotoxic T cells (CTLs) and regulatory T cells (Treg cells).

Treg cells are characterized by expression of both CD4 and CD25 and the forkhead/winged transcription factor FoxP3. First characterized in mice, in which they constitute about 6-10% of lymph node and splenic CD4⁺ T cell populations, CD4⁺CD25⁺ cells represent about 5-10% of human CD4⁺ T cells (Wing and Sakaguchi, 2010). As discussed in more detail below, Treg cells have the ability to suppress the activity of CD4⁺ T cells and CD8⁺ T cells.

Treg cells can be divided into several subsets (Bluestone *et al.,* 2000). One subset of Treg cells develops in the thymus (also known as natural Treg (nTreg) cells), and these thymic-derived Treg cells function by a cytokine-independent mechanism, which involves cell to cell contact (Shevach, 2002). These cells are essential for the induction and maintenance of self-tolerance and for the prevention of autoimmunity (Shevach, 2000; Salomon *et al.,* 2000; Sakaguchi *et al.,* 2001). These regulatory cells prevent the activation and proliferation of autoreactive T cells that have escaped thymic deletion or recognize extrathymic antigens, and, as a consequence are critical for homeostasis and immune regulation, as well as for protecting the host against the development of autoimmunity (Suri-Payer *et al.,* 1996; Asano, 1996; Willerford *et al.,* 1995; Salomon *et al.,* 2000).

Treg cells can also be generated by the activation of mature, peripheral CD4⁺ T cells (these cells are known as induced Treg (iTreg) cells). These cells can be generated *ex vivo,* e.g., by exposure to growth factors, and *in vivo,* e.g., in the gastrointestinal tract. Studies have indicated that peripherally derived Treg cells mediate their inhibitory activities by producing immunosuppressive cytokines, such as transforming growth factor-beta (TGF-β) and IL-10 (Kingsley 2002; Nakamura 2001). After antigen-specific activation, these Treg cells can non-specifically suppress proliferation of either CD4⁺ or CD8⁺ T cells (Baecher-Allan 2001). Studies have shown that CD4⁺CD25⁺ cells are able to inhibit anti-CD3 stimulation of T cells when co-cultured with autologous antigen presenting cells (APC) (e.g., Stephens 2001; Taams 2001).

While both nTreg cells and iTreg cells have regulatory activity, a recent study indicates that iTreg cells lose this activity *in vivo* in a study of graft-versus-host disease (GVHD; Koenecke *et al.,* 2009). In contrast nTreg cells maintain their regulatory activity and prevented development of GVHD. Thus, it is desirable to be able to identify/isolate populations of nTreg cells.

The immunomodulatory activity of Treg cells can be contact dependent, or the Treg cells may kill CD4⁺ and CD8⁺ T cells in a perforin- or granzyme-dependent manner or by the secretion of immunosuppressive cytokines, e.g., IL-10 and/or TGF-β (as reviewed in Dejaco 2005).

### Treg Cells and Autoimmunity/Tolerance

Depletion of Treg cells from various mouse strains has been shown to lead to a variety of autoimmune diseases that are tissue specific, including thyroiditis, oophoritis, gastritis or inflammatory bowel disease (Asano, 1996; Suri-Payer, 1998; and McHugh, 2002). Furthermore, human patients having a mutation in the FoxP3 gene fail to produce Treg cells and develop autoimmune polyendocrinopathy (especially type I diabetes and hypothyroidism) and enteropathy (summarized as immunodysregulation, polyendocrinopathy, enteropathy X-linked (IPEX) syndrome). A polymorphism in the FoxP3 is associated with autoimmune diabetes. Moreover, mice deficient in FoxP3 develop an IPEX like syndrome (see, Dejaco *et al.,* 2005).

The level of Treg cells is reduced in subjects suffering from a variety of disorders as shown in Table 1. Moreover, lower levels of these cells are associated with higher disease activity and/or poorer prognosis.

**Table 1: Autoimmune diseases associated with reduced levels of circulating CD4⁺CD25⁺ cells.**

| **Disease** | **Level of CD4+CD25+ cells (control) and significance** | **Reference** |
|---|---|---|
| Juvenile idiopathic arthritis | 1.2 (1.6) *** | De Kleer (2004) |
| Juvenile idiopathic arthritis | 0.4 (1.2) *** | Cao *et al.,* (2004). |
| Rheumatoid arthritis | 0.7 (1.2) * | Cao *et al., supra.* |
| Rheumatoid arthritis | 1.2(3.7) * | Liu *et al.,* (2004). |
| Psoriatic arthritis | 0.6 (1.2) * | Cao *et al., supra.* |
| HCV mixed cryoglobulinemia | 2.6 (7.9) ** | Boyer *et al.,* (2004) |
| Autoimmune liver disease | 2.5 (6.8) *** | Longhi *et al.,* (2004). |
| Systemic lupus erythematodes (SLE) | 1.8 (3.7) * | Cao *et al., supra.* |
| SLE | 0.9 (2.6) * | Crispin *et al.,* (2003). |

| | | |
|---|---|---|
| *, p<0.05; **, p<0.01, ***, p<0.001. | | |

Increased levels of CD4⁺CD25⁺ T cells are observed at sites of inflammation, e.g., in subjects suffering from juvenile idiopathic arthritis, rheumatoid arthritis, sponyloarthritis and infections (as reviewed in Dejaco *et al.,* 2005). These cells are considered to modulate local immune responses, e.g., to prevent collateral tissue damage.

Adoptive transfer of CD4⁺CD25⁺ T cells prevents the development of these diseases and, in some models can cure the disease after initiation (Mottet *et al.,* 2003). Suri-Payer *et al.,* (1998) also found that CD4⁺CD25⁺ T cells could prevent autoimmunity induced by autoantigen-specific T cell clones. Transfer of CD4⁺CD25⁻ T cells into nude mice also leads to development of autoimmune disease, which can be prevented by co-transfer of CD4⁺CD25⁺ T cells (Sakaguchi *et al.,* 1995).

Tang *et al.,* (2004) demonstrated that Treg cells are also useful for the treatment of autoimmune diabetes. The authors isolated Treg cells from non-obese diabetic (NOD) mice and expanded TCR transgenic cells specific for an autoantigen. Adoptive transfer of these cells to NOD mice reversed diabetes in newly transgenic mice.

Studies by Trenado (2002) also demonstrated that infusion of *ex vivo* activated and expanded CD4⁺CD25⁺ T cells significantly inhibit rapidly-lethal graft-versus-host disease (GVHD) in mice. Treg cells have also been shown to suppress allograft rejection in rodents with long term surviving cardiac (Van Maurik, 2002) or pancreatic islet (Gregori, 2001) allografts.

Based on the foregoing, it will be apparent to the skilled artisan that Treg cells are attractive for treatment or prevention of autoimmune disease or for inducing tolerance in a subject or that the detection of circulating levels of Treg cells is useful for the diagnosis or prognosis of those disorders. However, difficulties have arisen in translating the results of animal models to the human situation as a result of insufficient markers that permit isolation of Treg cells. To date, the only marker that clearly distinguishes Treg cells from other T cells is FoxP3. WO2007/140472 describes how CD127 expression inversely correlates with FOXP3 and suppressive function of CD4+ Treg cells. FOXP3 is an intracellular protein and, as a consequence, is not useful as a marker for isolating viable Treg cells. Accordingly, there is a need in the art for new markers, preferably cell surface markers of Treg cells that permit detection and/or isolation of those cells, e.g., for diagnositc and/or therapeutic and/or prophylactic purposes.

### Treg Cells and Inducing an Immune Response

Treg cells also exist in markedly higher proportions within tumor-infiltrating lymphocytes, peripheral blood lymphocytes, and/or regional lymph node lymphocytes of patients with cancer. The frequency of cells is related to tumor progression and inversely correlated with the efficacy of treatment. Accordingly, the ability of Treg cells to suppress anti-self immune responses appears to suppress the ability of the immune system to kill tumor cells.

Wang *et al.,* (2004) isolated a CD4⁺CD25⁺ tumor-infiltrating lymphocyte (TIL) from a human melanoma patient. This TIL recognized a tumor/self-antigen, LAGE-1. CD4⁺CD25⁺ TILs have also been isolated from ascites of patients with ovarian cancer, and these cells were shown to be capable of suppressing T cell activity (Curiel *et al.,* 2004).

Depleting populations of Treg has also been demonstrated to improve significantly the clearance of injected tumor cells. For example, Jones *et al.,* (2002), depletion of CD25 expressing T cells using monoclonal antibody therapy facilitated long-term CD4+ T cell-mediated immunity against melanoma cells. The authors demonstrated that following anti-CD25 treatment, mice developed an immune response against a self-antigen (tyrosinase) that accompanies inhibition of tumor growth in mice.

Goforth *et al.,* (2008) also demonstrated that poly lactic-co-glycolic acid (PLGA) polymer particles loaded with antigenic tumor lysate and immune stimulatory CpG oligonucleotides efficiently activated antigen-presenting cells and were incorporated into lysosomal compartments of macrophages and dendritic cells. Vaccination with the immune stimulatory antigen loaded particles (ISAPs) resulted in remarkable T cell proliferation, but only modestly suppressed tumor growth of established melanoma. When CD25⁺ cells were suppressed with anti-CD25 antibody, ISAP vaccination induced complete antigen-specific immunity in a prophylactic model. These findings suggest that it may be necessary or desirable to suppress Treg cell activity prior to and/or during vaccination, particularly against self-antigens.

It will be apparent from the foregoing discussion that depletion of Treg cells provides an attractive means for improving an immune response, e.g., against a self antigen or a non-self antigen. However, as discussed above, insufficient markers that permit removal of Treg cells has hampered therapeutic strategies targeting these cells. For example, while the cell surface marker CD25 is highly expressed on Treg cells and has been traditionally used to isolate or target these cells, this protein is also expressed on other T cell populations (e.g., activated T cells) in addition to activated B cells, some thymocytes, myeloid precursors, and oligodendrocytes. Accordingly, there is a need in the art for new markers, preferably cell surface markers of Treg cells that permit removal or destruction of Treg cells, e.g., for diagnostic, prognostic, therapeutic and/or prophylactic purposes.

### Summary of Invention

In a first aspect, the invention provides an *in vitro* method for detecting a regulatory T (Treg) cell (i.e., cells expressing CD4, CD25 and FoxP3) comprising determining the level of expression of a nucleic acid encoding PI16 or a PI16 protein in/on a T cell in a sample, wherein an increased level of expression of the nucleic acid or protein compared to another cell type is indicative of a Treg cell.

In a second aspect, the invention provides an isolated population of cells enriched for regulatory T (Treg) cells (i.e., cells expressing CD4, CD25 and FoxP3) expressing nucleic acid encoding PI16 or PI16 protein.

In a third aspect, the invention provides an isolated population of cells or a population of cells according to the claims for use in treating or preventing a condition associated with reduced regulatory T (Treg) cell numbers or activity, and/or inducing immunosuppression, and/or reducing CTL or Thelper cell activity in a subject.

The invention is further defined by the accompanying claims.

The invention in its broadest sense is defined by the claims. Only embodiments and examples falling under the claims constitute embodiments and examples of the invention. Embodiments and examples not falling under the claims constitute embodiments and examples of the disclosure.

### Further disclosures

Disclosed is a method for detecting a regulatory T (Treg) cell comprising determining the level of expression of a nucleic acid or protein set forth in Table 2 and/or Table 3, or a nucleic acid or protein having at least about 70% identity thereto, in/on a T cell, wherein an increased level of expression of a nucleic acid or protein set forth in Table 2 or nucleic acid or protein having at least about 70% identity thereto compared to another cell type, or a reduced level of expression of a nucleic acid or protein set forth in Table 3 or a nucleic acid or protein having at least about 70% identity thereto compared to another cell type is indicative of a Treg cell.

In one example, the method comprises determining the level of expression of a nucleic acid or protein set forth in Table 4 or a nucleic acid or protein having at least about 70% identity thereto in/on a T cell, wherein an increased level of expression of said nucleic acid or protein compared to another cell type is indicative of a Treg cell.

The protein is PI16 or the nucleic acid encodes PI16 in the present invention. In other aspects of the disclosure, the protein is Interleukin 1 receptor, type I (IL1R1) (CD121) or the nucleic acid encodes Interleukin 1 receptor, type I (IL1R1) (CD121). In other aspects of the disclosure, the protein is PKD1L3 or the nucleic acid encodes PKD1L3. In other aspects of the disclosure, the protein is PTPRB or the nucleic acid encodes PTPRB. In other aspects of the disclosure, the protein is CD146 or the nucleic acid encodes CD146. In other aspects of the disclosure, the protein is C18orf1 or the nucleic acid encodes C18orf1. In other aspects of the disclosure, the protein is AQP3 or the nucleic acid encodes AQP3. In other aspects of the disclosure, the protein is RGMB or the nucleic acid encodes RGMB. In other aspects of the disclosure, the protein is CD49f or the nucleic acid encodes CD49f. In other aspects of the disclosure, the protein is PERP or the nucleic acid encodes PERP. In other aspects of the disclosure, the protein is LSR or the nucleic acid encodes LSR.

In one example, the level of expression of the nucleic acid or protein is increased or reduced compared to the level of expression of the nucleic acid or protein in a T cell other than a Treg cell and, preferably, in a Thelper cell, preferably a stimulated Thelper cell or a resting Thelper cell. Preferably, the cell other than a Treg cell is a T cell expressing CD4 and expressing low levels of CD25 or levels of CD25 no greater than background (e.g., the level of an isotype control antibody).

In one example, the method detects an activated Treg cell and comprises determining the level of expression of a nucleic acid or protein set forth in Table 5 and/or Table 6, or a nucleic acid or protein having at least about 70% identity thereto, in/on a T cell, wherein an increased level of expression of a nucleic acid or protein set forth in Table 5 or a nucleic acid or protein having at least about 70% identity thereto compared to a non-activated Treg cell, or a reduced level of expression of a nucleic acid or protein set forth in Table 6 or a nucleic acid or protein having at least about 70% identity thereto compared to a non-activated Treg cell is indicative of an activated Treg cell.

Preferably, the protein is Interleukin 1 receptor, type I (IL1R1) (CD121) or the nucleic acid encodes Interleukin 1 receptor, type I (IL1R1) (CD121).

In one example, the method comprises initially isolating a population of cells comprising Treg cells, e.g., using a method as described herein, and then identifying an activated Treg cell.

In one example, the level of expression of a protein set forth in Table 2 and/or Table 3 and/or Table 4 and/or Table 5, or a protein having at least about 70% identity thereto, in/on the T cell is determined by contacting the cell with a compound that binds to said protein for a time and under conditions sufficient for a compound-protein complex to form and detecting the level of said complex, wherein the level of said complex is indicative of the level of said protein on said T cell. In this respect, any compound that binds specifically to the protein is suitable for performance of the invention.

Preferred compounds include antibodies and proteins comprising an antibody variable region.

In one example, the method additionally comprises detecting a cell that expresses CD4 and/or CD25. Optionally, the method additionally comprises detecting a cell that expresses a low or undetectable level of CD127 or a cell that is CD127⁻.

In one example, the method detects an iTreg cell and/or a nTreg cell. In one example, the method detects an iTreg cell. Preferably, the method detects a nTreg cell.

In one example, the Treg cell is a naive Treg cell, an effector memory Treg cell or a memory Treg cell.

Alternatively, or in addition, the method comprises detecting a cell that expresses low or undetectable levels of markers of T cells other than Treg cells, e.g., CD 19 and/or CD20 and/or CD 14 and/or CD56.

The present inventors have also demonstrated that the marker PI16 is useful for identifying nTreg cells, such as memory nTreg cells, e.g., resting (or non-activated) memory nTreg cells.

In one example, the cell expresses PI16, CD4 and CD25 and, optionally low or undetectable levels of CD127. Preferably, the cell additionally expresses CD45R0 and/or CD27 and/or CD95 and/or CCR6. Preferably, the cell expresses low levels of CD45RA (or is CD45RA⁻), CD146 (or is CD146⁻) and CXCR5 (or is CXCR5⁻))

Accordingly, the methods described herein according to any embodiment or example shall be taken to apply *mutatis mutandis* to the detection of PI16 nucleic acid or protein to identify or isolate nTreg cells, such as memory nTreg cells, e.g., resting (or non-activated) memory nTreg cells or to diagnose, prognose or treat conditions associated with those cells.

In one example, the method is performed using a sample from a subject, e.g., a blood sample or fraction thereof (e.g., plasma or serum or buffy coat fraction or peripheral blood mononuclear cell fraction) or a thymus or part thereof. Alternatively, the method is performed using one or more isolated cells or a lysate or extract thereof.

Another example provides a method for isolating a regulatory T (Treg) cell, said method comprising detecting a Treg cell by performing the method as described herein according to any embodiment and isolating the detected Treg cell.

Another example provides a method for isolating a population of cells enriched for regulatory T (Treg) cells, said method comprising:
(i) contacting a population of cells comprising Treg cells with a compound that binds to a protein set forth in Table 2 and/or 4 or a protein having at least about 70% identity thereto for a time and under conditions sufficient for said compound to bind to a cell and isolating cells to which the compound is bound; and/or
(ii) contacting said population with a compound that binds to a protein set forth in Table 3 or a protein having at least about 70% identity thereto for a time and under conditions sufficient for said compound to bind to a cell and isolating cells to which the compound binds to a reduced level compared to other cells in the population.

Preferably, (i) comprises isolating cells to which the compound binds to an increased level compared to other cells in the population.

The protein is PI16 or the nucleic acid encodes PI16 in the invention. In other disclosed aspects, the protein is Interleukin 1 receptor, type I (IL1R1) (CD121) or the nucleic acid encodes Interleukin 1 receptor, type I (IL1R1) (CD121), the protein is PKD1L3 or the nucleic acid encodes PKD1L3, the protein is PTPRB or the nucleic acid encodes PTPRB, the protein is CD146 or the nucleic acid encodes CD146, the protein is C18orfl or the nucleic acid encodes C18orfl, the protein is AQP3 or the nucleic acid encodes AQP3, the protein is RGMB or the nucleic acid encodes RGMB, the protein is CD49f or the nucleic acid encodes CD49f, the protein is PERP or the nucleic acid encodes PERP, the protein is LSR or the nucleic acid encodes LSR.

Another example provides a method for isolating a population of cells enriched for activated regulatory T (Treg) cells, said method comprising
(i) contacting said population with a compound that binds to a protein set forth in Table 5 or a protein having at least about 70% identity thereto for a time and under conditions sufficient for said compound to bind to a cell and isolating a cell(s) to which the compound is bound; and/or
(ii) contacting said population with a compound that binds to a protein set forth in Table 6 or a protein having at least about 70% identity thereto for a time and under conditions sufficient for said compound to bind to a cell and isolating cells to which the compound binds to a reduced level compared to other cells in the population.

Preferably, the protein is Interleukin 1 receptor, type I (IL1R1) (CD121) or the nucleic acid encodes Interleukin 1 receptor, type I (IL1R1) (CD121).

In an example, the compound that binds to the protein is an antibody or a protein comprising an antibody variable region.

The skilled artisan will be aware of suitable methods for isolating cells making use of compounds that bind to proteins, such as fluorescence-activated cell sorting (FACS) or magnetic cell separation cell techniques, e.g., MACS or techniques using Dynabeads™.

In one example, the population of cells is a population of T cells and/or a population of cells expressing CD4 and/or CD25. Optionally, the cells express low or undetectable levels of CD127 or a cell that is CD127⁻.

In one example, the method detects an iTreg cell and/or a nTreg cell. In one example, the method detects an iTreg cell. Preferably, the method detects a nTreg cell.

In one example, the Treg cell is a naive Treg cell, an effector memory Treg cell or a memory Treg cell.

Alternatively, or in addition, the method comprises depleting the population of cells and/or the isolated cells of cells expressing CD 19 and/or CD20 and/or CD 14 and/or CD56.

In one example, the enriched population is isolated from a sample from a subject, e.g., a blood sample or fraction thereof (e.g., plasma or serum or buffy coat fraction or peripheral blood mononuclear cell fraction) or a thymus or part thereof. Accordingly, the disclosure also encompasses a method additionally comprising providing or obtaining a sample from a subject. Such a sample may have been isolated previously from a subject, e.g., the method is performed *in vitro* or *ex vivo.* The population of cells can also be an isolated population of cells, e.g., produced using tissue culture techniques.

In one example, the method additionally comprises culturing the isolated cells, e.g., to increase the number of Treg cells. Alternatively, or in addition, in the case of non-activated Treg cells, the method can comprise activating the Treg cells.

In an example, the method additionally comprises formulating the isolated Treg cells with a pharmaceutically acceptable carrier or excipient to thereby produce a pharmaceutical composition.

The skilled artisan will appreciate that a method for identifying Treg cells in a sample from a subject is useful for diagnosing or prognosing a condition associated with Treg cells, e.g., by assessing the number of Treg cells in the sample. Such assessment can be made using standard techniques, e.g., FACS, MACS, immunohistochemistry or immunofluorescence. Accordingly, an example of the present invention provides a method for diagnosing and/or prognosing a Treg-associated condition in a subject, said method comprising performing a method as described herein according to the claims to detect a Treg cell in a sample from a subject wherein detection of Treg cell(s) or failure to detect Treg cells indicates that the subject suffers from a Treg-associated condition.

Suitable conditions are described herein, including in the Background of Invention section and shall be taken to apply *mutates mutandis* to the embodiments and examples herein in relation or diagnosis/prognosis/treatment/prevention of a condition associated with Treg cells.

In one example, the method comprises:
(i) determining or estimating the number of Treg cells in the sample or a portion thereof;
(ii) comparing the number of Treg cells determined or estimated at (i) to the number of Treg cells in a sample from a normal and/or healthy subject;
wherein an increased or decreased number of Treg cells at (i) compared to the number of Treg cells in a sample from a normal and/or healthy subject indicates that the subject suffers from a Treg-associated condition.

In one example, the subject is receiving treatment for the condition and wherein:
(i) a similar number of Treg cells at (i) compared to the number of Treg cells in a sample from a normal and/or healthy subject indicates that the subject is responding to treatment for said Treg-associated condition;
(ii) an increased or decreased number of Treg cells at (i) compared to the number of Treg cells in a sample from a normal and/or healthy subject indicates that the subject is not responding to treatment for said Treg-associated condition;
(iii) an increased or decreased number of Treg cells compared to the number of Treg cells in a sample from the subject prior to treatment indicates that the subject is responding to treatment for said Treg-associated condition; or
(iv) a similar number of Treg cells at (i) compared to the number of Treg cells in a sample from the subject prior to treatment indicates that the subject is not responding to treatment for said Treg-associated condition.

In one example, the method comprises contacting a sample with a compound that binds to a protein set forth in Table 2 and/or Table 4 for a time and under conditions sufficient for the compound to bind to a cell expressing said protein and determining the number of cells to which the compound has bound. Preferably, the compound is labeled with a detectable marker to facilitate detection. Preferred compounds include antibodies and proteins comprising an antibody variable region.

The skilled artisan will also appreciate that the provision of markers of Treg cells provides the basis for methods for diagnosing and/or prognosing a Treg-associated condition without necessarily assessing the number of cells in a sample, e.g., by detecting the level of the marker(s) in a sample, e.g., using an immunoassay. Accordingly, the disclosure additionally provides a method for diagnosing and/or prognosing a Treg-associated condition in a subject, said method comprising:
(i) detecting the level of a nucleic acid or protein set forth in Table 2 or a nucleic acid or protein having at least about 70% identity thereto in a sample from a subject;
(ii) comparing the level at (i) to the level of the nucleic acid or protein in a normal and/or healthy subject,
wherein an increased or reduced level of the nucleic acid or protein at (i) compared to the level in the normal and/or healthy subject indicates that the subject suffers from a Treg-associated condition.

Preferably, the method comprises detecting the level of a protein set forth in Table 4.

In one example, the subject is receiving treatment for said condition and wherein
(i) a similar level of the nucleic acid or protein at (i) compared to the level of the nucleic acid or protein in a sample from a normal and/or healthy subject indicates that the subject is responding to treatment for said Treg-associated condition;
(ii) an increased or decreased level of the nucleic acid or protein at (i) compared to the level of the nucleic acid or protein in a sample from a normal and/or healthy subject indicates that the subject is not responding to treatment for said Treg-associated condition;
(iii) an increased or decreased level of the nucleic acid or protein compared to the level of the nucleic acid or protein in a sample from the subject prior to treatment indicates that the subject is responding to treatment for said Treg-associated condition; or
(iv) a similar level of the nucleic acid or protein at (i) compared to the level of the nucleic acid or protein in a sample from the subject prior to treatment indicates that the subject is not responding to treatment for said Treg-associated condition.

In one example, the method comprises contacting a sample with a compound that binds to a protein set forth in Table 2 and/or Table 4 for a time and under conditions sufficient for a compound-protein complex to form and determining the level of said complex. Preferably, the compound is labeled with a detectable marker to facilitate detection.

Preferred compounds include antibodies or proteins comprising an antibody variable region.

Preferred Treg-associated conditions for diagnosis/prognosis using a method as described herein according to any embodiment include the following
- An autoimmune disease, e.g., juvenile idiopathic arthritis, rheumatoid arthritis, psoriatic arthritis, HCV mixed cryoglobulinemia, autoimmune liver disease or SLE, which is/are diagnosed/prognosed by detecting reduced levels of Treg cells or a reduced level of a protein expressed at a higher level on Treg cells in a sample, e.g., a blood sample or a fraction thereof;
- Inflammation, e.g., caused by juvenile idiopathic arthritis, rheumatoid arthritis, sponylarthritis or infection, which is/are diagnosed/prognosed by detecting increased levels of Treg cells or an increased level of a protein expressed at a higher level on Treg cells in, e.g., a sample from the site of inflammation or predicted inflammation (e.g., synovial fluid);
- Cancer(s) (e.g., cancers refractory to treatment with a vaccine or antigen-specific T cells), which is/are diagnosed/prognosed by detecting increased levels of Treg cells or an increased level of a protein expressed at a higher level on Treg cells in a sample, e.g., a sample of the cancer or in a blood sample or a fraction thereof.

Preferably, the condition is an autoimmune disease, preferably, rheumatoid arthritis. The present invention also contemplates autoimmune diseases, such as, type 1 diabetes, multiple scerosis or inflammatory bowel disease.

Preferably, the condition is a cancer. Preferably, the cancer is a hematological cancer. Preferably, the cancer is a breast cancer. Preferably, the cancer is a colorectal cancer. Preferably, the cancer is a prostate cancer. Other examples of suitable cancers include brain cancer (e.g., glioma), gastric cancer, cervical cancer, melanoma and lymphoma (e.g., Hodgkin's lymphoma).

Additional suitable conditions are described herein, including in the Background of Invention section, and shall be taken to apply *mutatis mutandis* to the present example of the invention.

The skilled artisan will appreciate that methods described herein for isolating a Treg cell also provide the basis for increasing Treg cell numbers in a subject, e.g., by adoptive transfer or cell therapy. Increasing Treg cells numbers is useful for, for example, treating or preventing a condition associated with reduced Treg numbers and/or inducing immunosuppression in a subject and/or reducing CTL or Thelper cell activity in a subject. Accordingly, another example provides a method of treating or preventing a condition associated with reduced Treg cell numbers or activity, and/or inducing immunosuppression, and/or reducing CTL or Thelper cell activity in a subject, said method comprising:
(i) isolating a population of Treg cells by performing a method as described herein according to any embodiment; and
(ii) administering the cells at (i) to the subject.

In one example, the subject suffers from or is at risk of developing a condition associated with reduced Treg numbers and/or activity and/or requires a reduction in CTL or Thelper cell activity (e.g., the subject suffers from or is at risk of developing an autoimmune disease) and/or the subject requires immunosuppression (e.g., is undergoing or about to undergo a transplant or suffers from graft-versus-host disease).

Preferably, the cells are from the subject to be treated, i.e., an autologous transplant, or from a related subject of the same species (e.g., a HLA matched subject), i.e., an allogeneic transplant. The present invention also encompasses xenogeneic transplants.

Preferably an effective amount, e.g., a therapeutically or prophylactically effective amount of cells is administered.

The identification of cell surface proteins preferentially expressed by Treg cells also provides the means for depleting or reducing the number of those cells in a subject, e.g., to reduce or prevent Treg cell-mediated suppression of a subject's immune system. Accordingly, another example provides a method of treating or preventing a condition associated with regulatory T (Treg) cell-mediated suppression of a subject's immune system, said method comprising administering to a subject in need thereof a compound that reduces expression and/or activity of a protein or nucleic acid set forth in Table 2 and/or that administering to a subject in need thereof a compound that increases expression and/or activity of a protein or nucleic acid set forth in Table 3.

Another example of the present invention provides for the use of a population of cells in medicine or in the manufacture of a medicament for administration to a subject in need thereof, e.g., to treat a Treg-associated condition.

The present disclosure also provides a population of cells enriched for Treg cells expressing one or more of the nucleic acids or proteins set forth in Table 2 and/or expressing a reduced level of one or more of the nucleic acids or proteins set forth in Table 3.

Preferably, the population of cells is enriched for Treg cells expressing one or more of the nucleic acids or proteins set forth in Table 4.

The protein is PI16 or the nucleic acid encodes PI16 in the present invention. In other aspects of the disclosure, the protein is Interleukin 1 receptor, type I (IL1R1) (CD121) or the nucleic acid encodes Interleukin 1 receptor, type I (IL1R1) (CD121). In other aspects of the disclosure, the protein is PKD1L3 or the nucleic acid encodes PKD1L3. In other aspects of the disclosure, the protein is PTPRB or the nucleic acid encodes PTPRB. In other aspects of the disclosure, the protein is CD146 or the nucleic acid encodes CD146. In other aspects of the disclosure, the protein is C18orfl or the nucleic acid encodes C180rf1. In other aspects of the disclosure, the protein is AQP3 or the nucleic acid encodes AQP3. In other aspects of the disclosure, the protein is RGMB or the nucleic acid encodes RGMB. In other aspects of the disclosure, the protein is CD49f or the nucleic acid codes CD49f. In other aspects of the disclosure, the protein is PERP or the nucleic acid encodes PERP. In other aspects of the disclosure, the protein is LSR or the nucleic acid encodes LSR.

In one example, the population of cells is enriched for iTreg cells and/or nTreg cells, more preferably, nTreg cells.

In one example, the population of cells is enriched for naive Treg cells, effector memory Treg cells or memory Treg cells.

Preferably, the population is additionally enriched for cells expressing one or more of CD4 and/or CD25. Optionally, the population is additionally enriched for cells expressing low or undetectable levels of CD127 or that are CD127⁻.

In one example, the population of cells is enriched for cells expressing CD4, CD25 and PI16. Optionally, the population of cells is also enriched for cells expressing low or undetectable levels of CD127. Preferably, the population is additionally enriched for cells expressing one or more of CD45R0 and/or CD27 and/or CD95 and/or CCR6. Preferably, the population is additionally enriched for cells expressing low or undetectable levels of CD127 and/or CD45RA and/or CD146 and/or CXCR5.

Preferably, such a population of cells is enriched for nTreg cells, e.g., memory nTreg cells, such as resting memory nTreg cells.

In one example, the population of cells is enriched for cells expressing CD4, CD25, CD45R0, CD27, CD95, CCR6 and PI16 and expressing low or undetectable levels of CD45RA, CD127, CD146 and CXCR5.

In one example, the population of cells is enriched for cells expressing CD4, CD25 and AQP3. Optionally, the population of cells is also enriched for cells expressing low or undetectable levels of CD127.

In one example, the population of cells is enriched for cells expressing CD4, CD25 and C18orfl. Optionally, the population of cells is also enriched for cells expressing low or undetectable levels of CD127.

In one example, the population of cells is enriched for cells expressing CD4, CD25 and CD121. Optionally, the population of cells is also enriched for cells expressing low or undetectable levels of CD127.

In one example, the population of cells is enriched for cells expressing CD4, CD25 and CD146. Optionally, the population of cells is also enriched for cells expressing low or undetectable levels of CD 127.

In one example, the population of cells is enriched for cells expressing CD4, CD25 and CD49f. Optionally, the population of cells is also enriched for cells expressing low or undetectable levels of CD 127.

In one example, the population of cells is enriched for cells expressing CD4, CD25 and LSR. Optionally, the population of cells is also enriched for cells expressing low or undetectable levels of CD127.

In one example, the population of cells is enriched for cells expressing CD4, CD25 and PERP. Optionally, the population of cells is also enriched for cells expressing low or undetectable levels of CD127.

In one example, the population of cells is enriched for cells expressing CD4, CD25 and PKD1L3. Optionally, the population of cells is also enriched for cells expressing low or undetectable levels of CD 127.

In one example, the population of cells is enriched for cells expressing CD4, CD25 and PTPRB. Optionally, the population of cells is also enriched for cells expressing low or undetectable levels of CD127.

### Brief Description of the Drawings

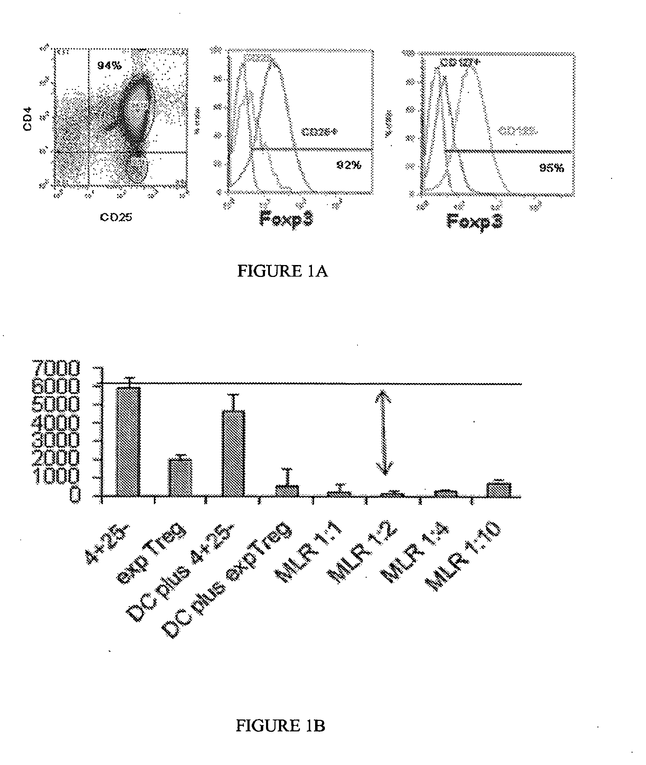

Figure 1A includes graphical representations showing (left-hand panel) isolation of Treg cells (upper ellipse) and Thelper cells (lower ellipse) using FACS analysis based on CD4 and CD25 expression levels; and (middle and right-hand panels) phenotypic analysis of expanded Treg cells showing that these cells maintained a Treg phenotype upon expansion, with between 80- 90% of the expanded cells staining CD4⁺, CD25^{hi}, CD127^{-/dim} and FoxP3⁺.
Figure 1B is a graphical representation showing that post expansion Treg cells retained function as they were able to robustly suppress the proliferation of CD4⁺CD25⁻ cells *in vitro* in an unmatched donor mixed leukocyte suppression assay.

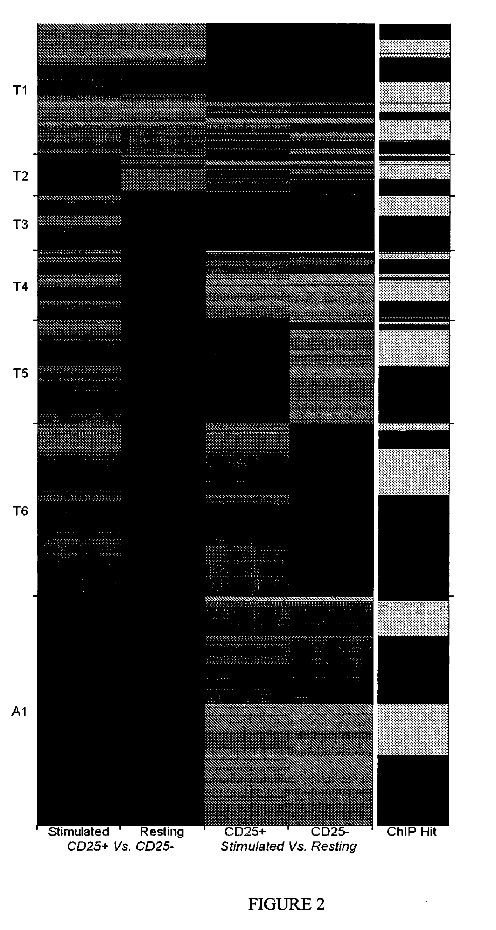

Figure 2 is a diagrammatic representation showing a heatmap of differentially expressed genes in resting or activated Treg vs Thelpers, some of which are listed in Table 7.

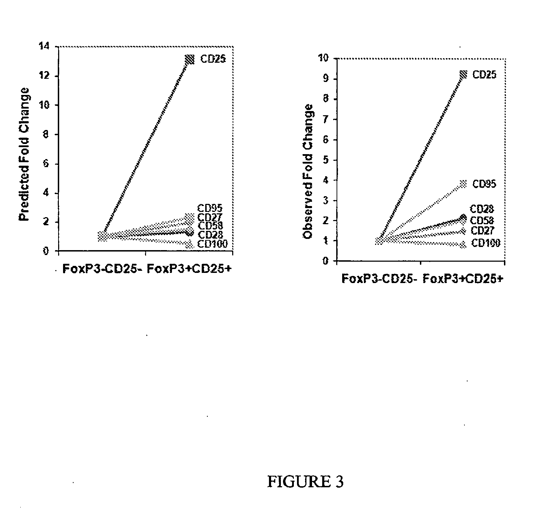

Figure 3 includes two graphical representations showing the level of expression of six genes identified as differentially expressed in Treg cells using microarray analysis in FoxP3⁻CD25⁻ cells (non-Treg) and FoxP3⁺CD25⁺ cells (Treg). The left-hand panel shows the difference in gene expression expected from microarray analysis, and the right-hand panel shows the difference in gene expression observed using qPCR.

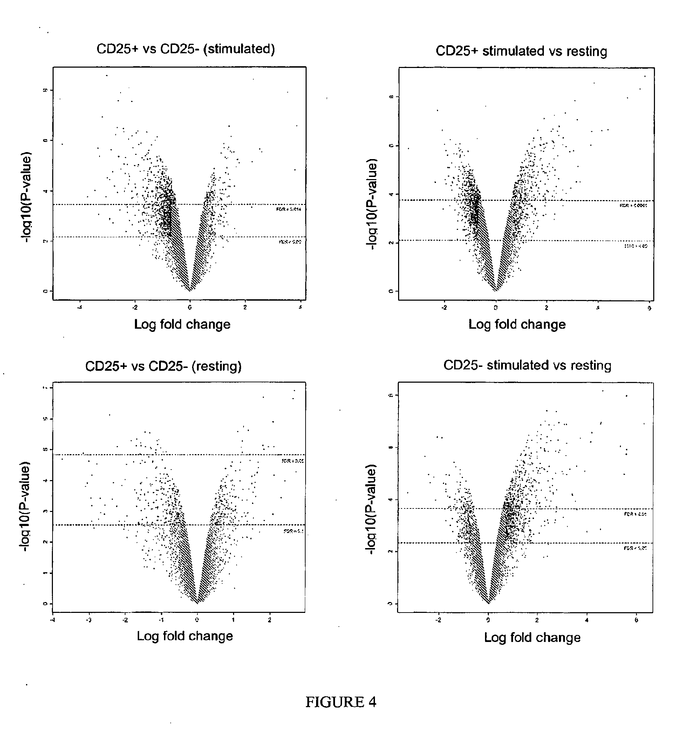

Figure 4 is a series of volcano plots showing genes selected for inclusion in the heatmap (black dots) and the FDR estimates used for selection. FDR estimates for the first tier of genes are the upper line in each plot, and estimates for the second tier are the lower line in each plot. A log fold-change threshold of 0.7 was applied to second tier genes from each comparison, with the exception of CD25⁺ v CD25⁻ where a threshold of 0.5 was applied.

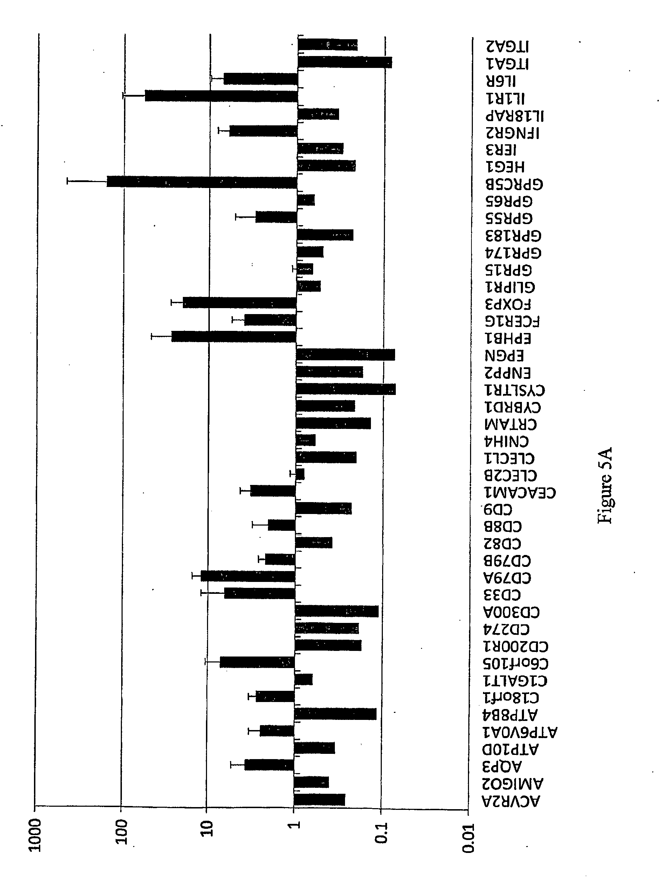

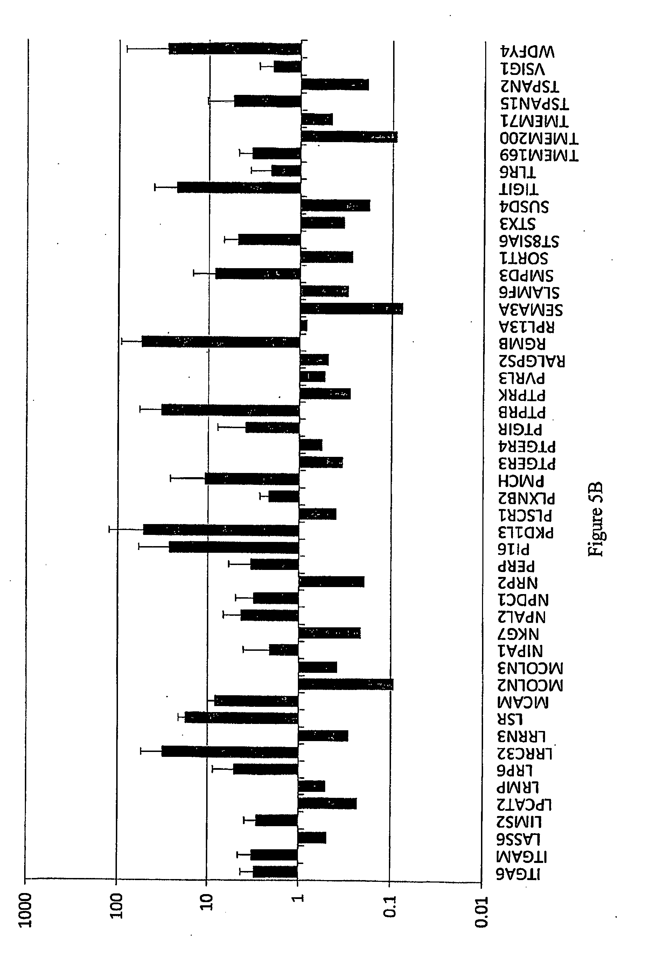

Figures 5A and 5B are graphical representations showing mean relative fold change (log fold change is depicted on the Y axis) in the level of expression of the genes indicated on the X-axis in stimulated Treg cells compared to stimulated Thelper cells. Results were obtained using low density array analysis as described herein. N=4. Error bars are indicative of the range in the data obtained.

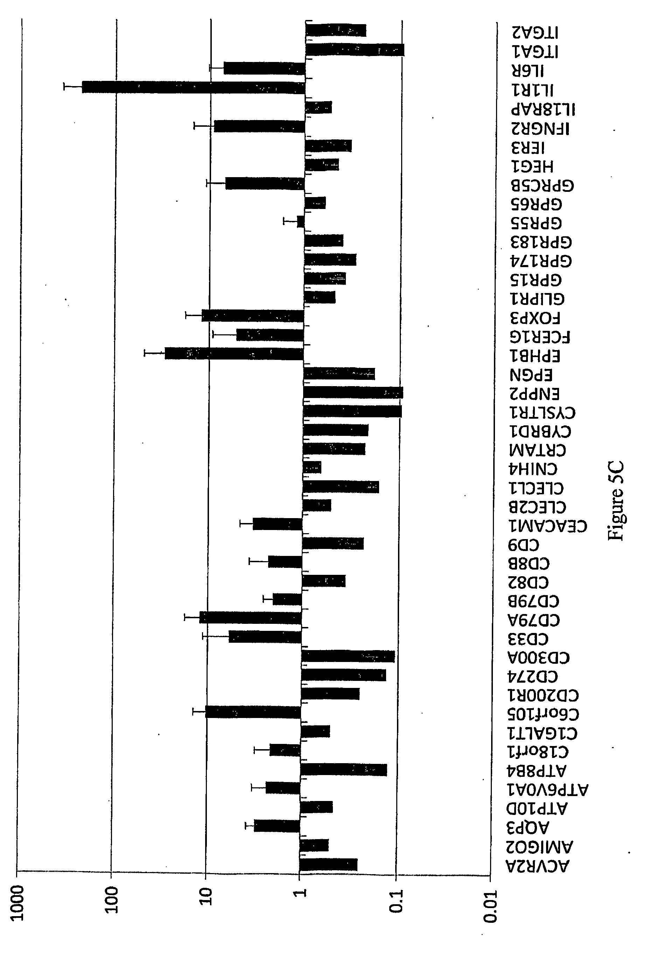

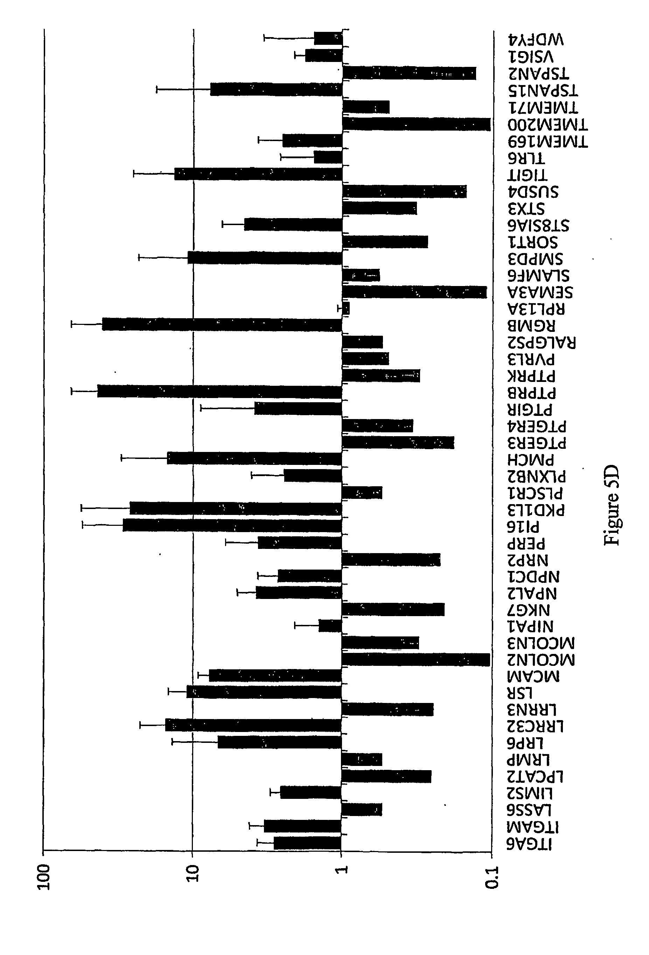

Figure 5C and 5D are graphical representations showing mean relative fold change (log fold change is depicted on the Y axis) in the level of expression of the genes indicated on the X-axis in resting Treg cells compared to resting Thelper cells. Results were obtained using low density array analysis as described herein. N=4. Error bars are indicative of the range in the data obtained.

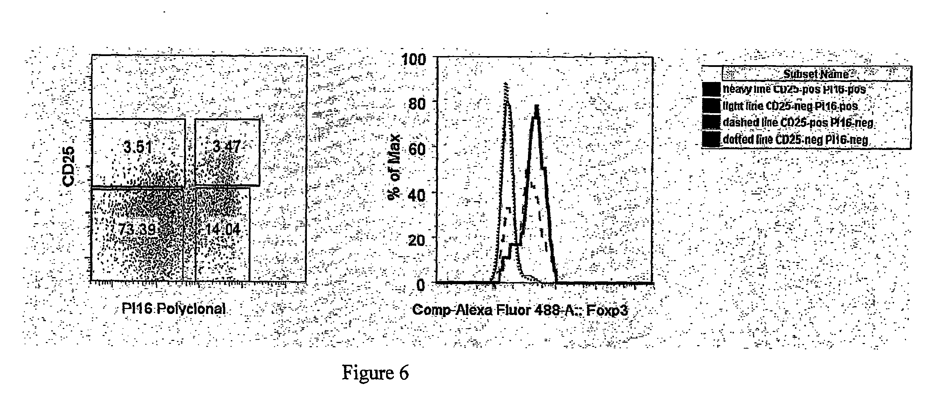

Figure 6 is a series of graphical representations showing staining of freshly isolated adult CD4⁺ T cells with a polyclonal PI16 antibody (Abnova) together with CD25 or FoxP3. CD25 and PI16 staining is shown in the left hand panel with boxes indicating CD25 positive or negative or PI16 positive or negative. The right hand panel indicates FoxP3 expression levels for each boxed cell population. Results reveal that CD4⁺CD25⁺PI16⁺ cells show the highest FoxP3 expression levels whereas the CD4⁺CD25⁺PI16- cells show a lower mean expression level. CD4⁺CD25⁻ cells are FoxP3⁻.

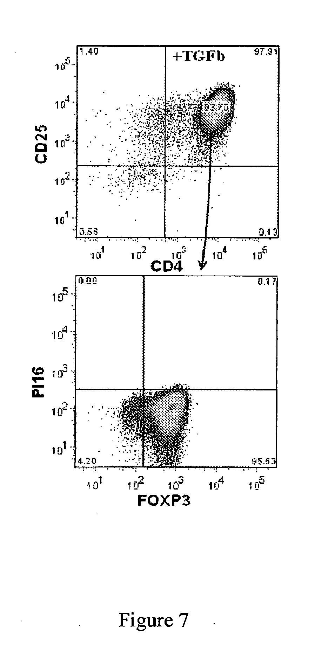

Figure 7 is a series of graphical representations showing the expression of PI16 on induced Treg (iTreg). Cord blood CD4⁺CD25⁻ cells were expanded in the presence of recombinant human TGFβ-1 and CD3/CD28 T cell expander beads for a total of eight days before beads were removed and analyzed for CD25, FoxP3 and PI16 expression. The induced Treg expressed FoxP3 but are not positive for PI16 suggesting that PI16 may serve as a marker for natural but not induced Treg.

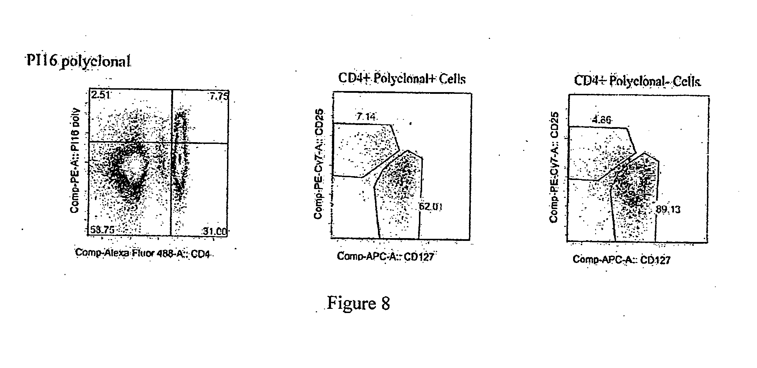

Figure 8 is a series of graphical representations showing the proportion of PI16⁺ cells (identified with PI16 polyclonal antibody) that are in the population of cells isolated based on Treg cell surface markers (CD4⁺CD25^{bright}CD127⁻).

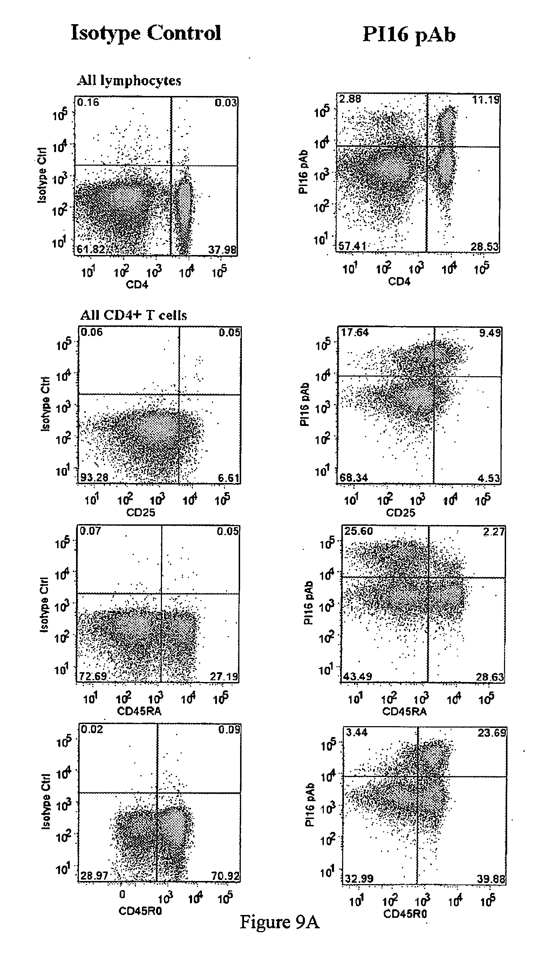

Figure 9A is a series of graphical representations showing detection of T cell populations by an anti-PI16 polyclonal antibody (right hand panels). Results obtained with an Isotype control antibody are depicted in the left hand panels. In the top panels lymphocytes were labeled with antibodies to PI16 and to CD4 and the results show that PI16 is expressed in CD4⁺ lymphocytes. The panel second from the top shows results when CD4⁺ lymphocytes were labeled with antibodies against PI16 and CD25. These results indicate that a population of CD4⁺CD25^{hi/bright} cells (which include a Treg cell population) express PI16. The third panel from the top and the bottom panel show results of assays in which CD4+ lymphocytes were labeled with antibodies against PI16 and CD45RA or CD45R0. Results indicate that PI16 is expressed by memory T cells (CD45R0⁺CD45RA^{-/low}).

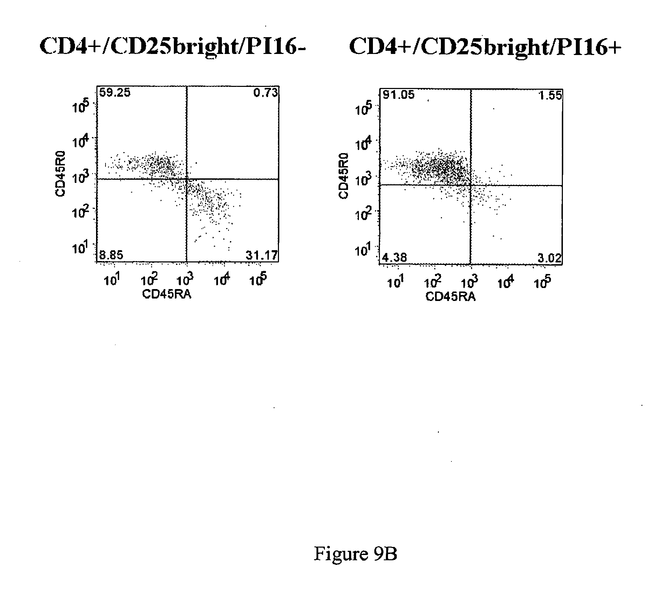

Figure 9B shows results of assays in which a population of CD4⁺CD25^{hi/bright}PI16⁺ Treg cells and a population of CD4⁺CD25^{hi/bright}PI16⁻ Treg cells were labeled with antibodies against CD45R0 or CD45RA. Results indicate that PI16 is expressed by memory Treg cells (CD45R0⁺CD45RA^{-/low}).

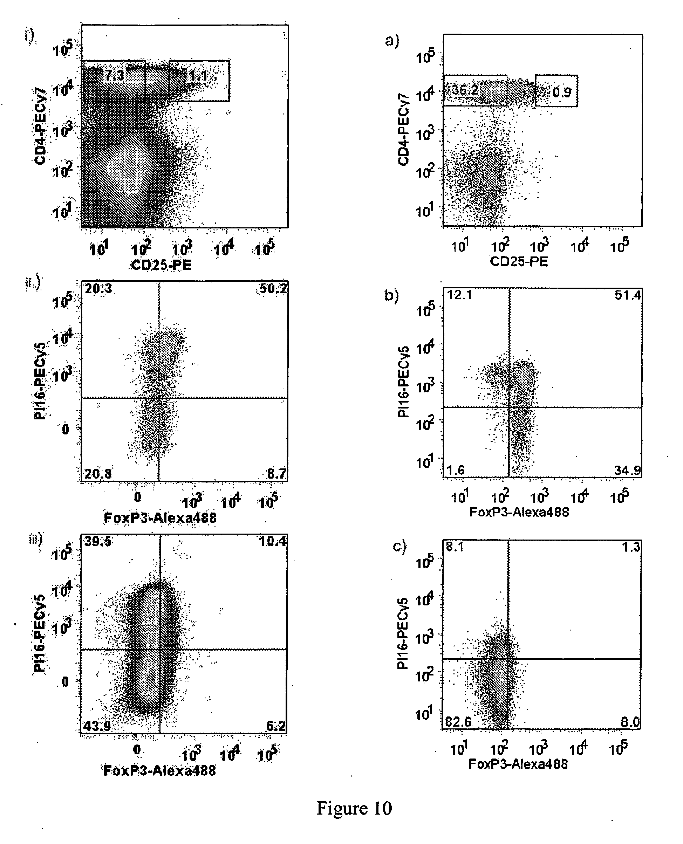

Figure 10 is a series of graphical representations showing expression of PI16 and FoxP3 expression in nTreg cells or Thelper cells.

The left hand panels show that when CD4⁺ cells were gated on the top 1% CD25⁺ cells (as depicted in Panel i) >85% of the FoxP3⁺ cells are PI16⁺ (as shown in Panel ii). CD25⁺ CD4 cells were also isolated according to the gates shown (Panel i) and then stimulated overnight with anti CD3/CD28 beads and stained for PI16 and FoxP3 expression. Sorted stimulated CD25⁺ cells stained robustly with the anti PI16 antibody, and this population contains >60% of the FoxP3⁺ cells (Panel iii).

The right hand panels show that when CD4⁺ cells were gated on CD25⁻ (as depicted in Panel a) few FOXP3 cells are detected (as shown in Panel b). CD25⁻ CD4 cells were also isolated according to the gates shown (Panel a) and then stimulated overnight with anti CD3/CD28 beads and stained for PI16 and FoxP3 expression. The majority of the activated CD25- cells do not express PI16 or FoxP3 (Panel c). Representative data from n=3 donors.

### Key to Sequence Listing

SEQ ID NO: 1 - Nucleotide coding sequence for human sphingomyelin phosphodiesterase 3 (SMPD3).

SEQ ID NO: 2 - Amino acid sequence of human sphingomyelin phosphodiesterase 3 (SMPD3).

SEQ ID NO: 3 - Nucleotide coding sequence for isoform alpha 1 of human chromosome 18 open reading frame 1 (C18orf1).

SEQ ID NO: 4 - Amino acid sequence of isoform alpha 1 of human chromosome 18 open reading frame 1 (C18orf1).

SEQ ID NO: 5 - Nucleotide coding sequence for isoform alpha 2 of human chromosome 18 open reading frame 1 (C18orf1).

SEQ ID NO: 6 - Amino acid sequence for isoform alpha 2 of human chromosome 18 open reading frame 1 (C18orfl).

SEQ ID NO: 7 - Nucleotide coding sequence for isoform beta 1 of human chromosome 18 open reading frame 1 (C18orf1).

SEQ ID NO: 8 - Amino acid sequence for isoform beta 1 of human chromosome 18 open reading frame 1 (C18orf1).

SEQ ID NO: 9 - Nucleotide coding sequence for isoform beta 2 of human chromosome 18 open reading frame 1 (C18orf1).

SEQ ID NO: 10 - Amino acid sequence for isoform beta 2 of human chromosome 18 open reading frame 1 (C18orf1).

SEQ ID NO: 11 - Nucleotide coding sequence for isoform gamma 1 of human chromosome 18 open reading frame 1 (C18orf1).

SEQ ID NO: 12 - Amino acid sequence for isoform gamma 1 of human chromosome 18 open reading frame 1 (C18orf1).

SEQ ID NO: 13 - Nucleotide coding sequence for isoform gamma 2 of human chromosome 18 open reading frame 1 (C18orf1).

SEQ ID NO: 14 - Amino acid sequence for isoform gamma 2 of human chromosome 18 open reading frame 1 (C18orfl).

SEQ ID NO: 15 - Nucleotide coding sequence for human interleukin 1 receptor, type I (IL1R1).

SEQ ID NO: 16 - Amino acid sequence of human interleukin 1 receptor, type I (IL1R1).

SEQ ID NO: 17 - Nucleotide coding sequence for isoform 1 of human CD79A.

SEQ ID NO: 18 - Amino acid sequence for isoform 1 of human CD79A.

SEQ ID NO: 19 - Nucleotide coding sequence for isoform 2 of human CD79A.

SEQ ID NO: 20 - Amino acid sequence for isoform 2 of human CD79A.

SEQ ID NO: 21 - Nucleotide coding sequence for isoform (a) of human protein tyrosine phosphatase, receptor type B (PTPRB).

SEQ ID NO: 22 - Amino acid sequence for isoform (a) of human protein tyrosine phosphatase, receptor type B (PTPRB).

SEQ ID NO: 23 - Nucleotide coding sequence for isoform (b) of human protein tyrosine phosphatase, receptor type B (PTPRB).

SEQ ID NO: 24 - Amino acid sequence for isoform (b) of human protein tyrosine phosphatase, receptor type B (PTPRB).

SEQ ID NO: 25 - Nucleotide coding sequence for human interferon gamma receptor 2 (IFNGR2).

SEQ ID NO: 26 - Amino acid sequence of human interferon gamma receptor 2 (IFNGR2).

SEQ ID NO: 27 - Nucleotide coding sequence for human peptidase inhibitor 16 (PI16). SEQ ID NO: 28 - Amino acid sequence of human peptidase inhibitor 16 (PI16).

SEQ ID NO: 29 -Nucleotide coding sequence for isoform 1 of human CD33.

SEQ ID NO: 30 - Amino acid sequence for isoform 1 of human CD33.

SEQ ID NO: 31 - Nucleotide coding sequence for isoform 2 of human CD33.

SEQ ID NO: 32 - Amino acid sequence for isoform 2 of human CD33.

SEQ ID NO: 33 - Nucleotide coding sequence for human melanoma cell adhesion molecule (MCAM).

SEQ ID NO: 34 - Amino acid sequence of human melanoma cell adhesion molecule (MCAM).

SEQ ID NO: 35 - Nucleotide coding sequence for human integrin alpha M (ITGAM).

SEQ ID NO: 36 - Amino acid sequence of human integrin alpha M (ITGAM).

SEQ ID NO: 37 -Nucleotide coding sequence for human aquaporin 3 (AQP3).

SEQ ID NO: 38 - Amino acid sequence of human aquaporin 3 (AQP3).

SEQ ID NO: 39 - Nucleotide coding sequence for isoform A of human integrin alpha 6 (ITGA6).

SEQ ID NO: 40 - Amino acid sequence for isoform A of human integrin alpha 6 (ITGA6).

SEQ ID NO: 41 - Nucleotide coding sequence for isoform B of human integrin alpha 6 (ITGA6).

SEQ ID NO: 42 - Amino acid sequence for isoform B of human integrin alpha 6 (ITGA6).

SEQ ID NO: 43 - Nucleotide coding sequence for isoform 1 of human lipolysis stimulated lipoprotein receptor (LSR).

SEQ ID NO: 44 - Amino acid sequence for isoform 1 of human lipolysis stimulated lipoprotein receptor (LSR).

SEQ ID NO: 45 - Nucleotide coding sequence for isoform 2 of human lipolysis stimulated lipoprotein receptor (LSR).

SEQ ID NO: 46 - Amino acid sequence for isoform 2 of human lipolysis stimulated lipoprotein receptor (LSR).

SEQ ID NO: 47 - Nucleotide coding sequence for isoform 3 of human lipolysis stimulated lipoprotein receptor (LSR).

SEQ ID NO: 48 - Amino acid sequence for isoform 3 of human lipolysis stimulated lipoprotein receptor (LSR).

SEQ ID NO: 49 - Nucleotide coding sequence for isoform 1 of human carcinoembryonic antigen-related cell adhesion molecule 1 (CEACAM1).

SEQ ID NO: 50 - Amino acid sequence for isoform 1 of human carcinoembryonic antigen-related cell adhesion molecule 1 (CEACAM1).

SEQ ID NO: 51 - Nucleotide coding sequence for isoform 2 of human carcinoembryonic antigen-related cell adhesion molecule 1 (CEACAM1).

SEQ ID NO: 52 - Amino acid sequence for isoform 2 of human carcinoembryonic antigen-related cell adhesion molecule 1 (CEACAM1).

SEQ ID NO: 53 - Nucleotide coding sequence for human neural proliferation, differentiation and control 1 protein (NPDC1).

SEQ ID NO: 54 - Amino acid sequence of human proliferation, differentiation and control 1 protein (NPDC1).

SEQ ID NO: 55 - Nucleotide coding sequence for human EPH receptor B1 (EPHB1).

SEQ ID NO: 56 - Amino acid sequence of human EPH receptor B1 (EPHB1).

SEQ ID NO: 57 - Nucleotide coding sequence for human toll-like receptor 6 (TLR6).

SEQ ID NO: 58 - Amino acid sequence of human toll-like receptor 6 (TLR6).

SEQ ID NO: 59 - Nucleotide coding sequence for human plexin B2 (PLXNB2).

SEQ ID NO: 60 - Amino acid sequence of human plexin B2 (PLXNB2).

SEQ ID NO: 61 - Nucleotide coding sequence for human low density lipoprotein receptor-related protein 6 (LRP6).

SEQ ID NO: 62 - Amino acid sequence of human low density lipoprotein receptor-related protein 6 (LRP6).

SEQ ID NO: 63 - Nucleotide coding sequence for isoform 1 of human CD8B.

SEQ ID NO: 64 - Amino acid sequence for isoform 1 of human CD8B.

SEQ ID NO: 65 - Nucleotide coding sequence for isoform 2 of human CD8B.

SEQ ID NO: 66 - Amino acid sequence for isoform 2 of human CD8B.

SEQ ID NO: 67 - Nucleotide coding sequence for isoform 3 of human CD8B.

SEQ ID NO: 68 - Amino acid sequence for isoform 3 of human CD8B.

SEQ ID NO: 69 - Nucleotide coding sequence for isoform 4 of human CD8B.

SEQ ID NO: 70 - Amino acid sequence for isoform 4 of human CD8B.

SEQ ID NO: 71 - Nucleotide coding sequence for isoform 5 of human CD8B.

SEQ ID NO: 72 - Amino acid sequence for isoform 5 of human CD8B.

SEQ ID NO: 73 - Nucleotide coding sequence for human V-set and immunoglobulin domain containing 1 protein (VSIG1).

SEQ ID NO: 74 - Amino acid sequence of human V-set and immunoglobulin domain containing 1 protein (VSIG1).

SEQ ID NO: 75 - Nucleotide coding sequence for human p53 apoptosis effector related to PMP22 (PERP).

SEQ ID NO: 76 - Amino acid sequence of human p53 apoptosis effector related to PMP22 (PERP).

SEQ ID NO: 77 - Nucleotide coding sequence for isoform 1 of human CD79B.

SEQ ID NO: 78 - Amino acid sequence for isoform 1 of human CD79B.

SEQ ID NO: 79- Nucleotide coding sequence for isoform 2 of human CD79B.

SEQ ID NO: 80 - Amino acid sequence for isoform 2 of human CD79B.

SEQ ID NO: 81 - Nucleotide coding sequence for isoform 3 of human CD79B.

SEQ ID NO: 82 - Amino acid sequence for isoform 3 of human CD79B.

SEQ ID NO: 83 - Nucleotide coding sequence for human polycystic kidney disease 1-like 3 (PKD1L3).

SEQ ID NO: 84 - Amino acid sequence for human polycystic kidney disease 1-like 3 (PKD1L3).

SEQ ID NO: 85 - Nucleotide coding sequence for human T cell immunoreceptor with Ig and ITIM domains (TIGIT).

SEQ ID NO: 86 - Amino acid sequence for human T cell immunoreceptor with Ig and ITIM domains (TIGIT).

SEQ ID NO: 87 - Nucleotide coding sequence for human ST8 alpha-N-acetyl-neuraminide alpha-2,8-sialyltransferase 6 (ST8SIA6).

SEQ ID NO: 88 - Amino acid sequence for human ST8 alpha-N-acetyl-neuraminide alpha-2,8-sialyltransferase 6 (ST8SIA6).

SEQ ID NO: 89 - Nucleotide coding sequence for isoform 1 of human chromosome 6 open reading frame 105 (C6orf105).

SEQ ID NO: 90 - Amino acid sequence for isoform 1 of human chromosome 6 open reading frame 105 (C6orf105).

SEQ ID NO: 91 - Nucleotide coding sequence for isoform 2 of human chromosome 6 open reading frame 105 (C6orfl05).

SEQ ID NO: 92 - Amino acid sequence for isoform 2 of human chromosome 6 open reading frame 105 (C6orfl05).

SEQ ID NO: 93 - Nucleotide coding sequence for human tetraspanin 15 (TSPAN15). SEQ ID NO: 94 - Amino acid sequence for human tetraspanin 15 (TSPAN15).

SEQ ID NO: 95 - Nucleotide coding sequence for human NIPA-like domain containing 2 (NPAL2).

SEQ ID NO: 96 - Amino acid sequence for human NIPA-like domain containing 2 (NPAL2).

SEQ ID NO: 97 - Nucleotide coding sequence for human FRAS1 related extracellular matrix 3 (FREM3).

SEQ ID NO: 98 - Amino acid sequence for human FRAS1 related extracellular matrix 3 (FREM3).

SEQ ID NO: 99 - Nucleotide coding sequence for human WDFY family member 4 (WDFY4).

SEQ ID NO: 100 - Amino acid sequence for human WDFY family member 4 (WDFY4).

SEQ ID NO: 101 - Nucleotide coding sequence for human Fc fragment of IgE, high affinity I, receptor for; gamma polypeptide (FCER1G).

SEQ ID NO: 102 - Amino acid sequence for human Fc fragment of IgE, high affinity I, receptor for; gamma polypeptide (FCER1G).

SEQ ID NO: 103 - Nucleotide coding sequence for variant 1 of human transmembrane protein 169 (TMEM169).

SEQ ID NO: 104 - Amino acid sequence for variant 1 of human transmembrane protein 169 (TMEM169).

SEQ ID NO: 105 - Nucleotide coding sequence for variant 2 of human transmembrane protein 169 (TMEM169).

SEQ ID NO: 106 - Amino acid sequence for variant 2 of human transmembrane protein 169 (TMEM169).

SEQ ID NO: 107 - Nucleotide coding sequence for variant 3 of human transmembrane protein 169 (TMEM169).

SEQ ID NO: 108 - Amino acid sequence for variant 3 of human transmembrane protein 169 (TMEM169).

SEQ ID NO: 109 - Nucleotide coding sequence for variant 4 of human transmembrane protein 169 (TMEM169).

SEQ ID NO: 110 - Amino acid sequence for variant 4 of human transmembrane protein 169 (TMEM169).

SEQ ID NO: 111 - Nucleotide coding sequence for human mucolipin 2 (MCOLN2). SEQ ID NO: 112 - Amino acid sequence for human mucolipin 2 (MCOLN2).

SEQ ID NO: 113 - Nucleotide coding sequence for human C-type lectin-like 1 (CLECL1).

SEQ ID NO: 114 - Amino acid sequence for human C-type lectin-like 1 (CLECL1). SEQ ID NO: 115 - Nucleotide coding sequence for human lysophosphatidylcholine acyltransferase 2 (LPCAT2).

SEQ ID NO: 116 - Amino acid sequence for human lysophosphatidylcholine - acyltransferase 2 (LPCAT2).

SEQ ID NO: 117 - Nucleotide coding sequence for variant 1 of human leucine rich repeat neuronal 3 (LRRN3).

SEQ ID NO: 118 - Amino acid sequence for variant 1 of human leucine rich repeat neuronal 3 (LRRN3).

SEQ ID NO: 119 - Nucleotide coding sequence for variant 2 of human leucine rich repeat neuronal 3 (LRRN3).

SEQ ID NO: 120 - Amino acid sequence for variant 2 of human leucine rich repeat neuronal 3 (LRRN3).

SEQ ID NO: 121 - Nucleotide coding sequence for variant 3 of human leucine rich repeat neuronal 3 (LRRN3).

SEQ ID NO: 122 - Amino acid sequence for variant 3 of human leucine rich repeat neuronal 3 (LRRN3).

SEQ ID NO: 123 - Nucleotide coding sequence for human syntaxin 3 (STX3).

SEQ ID NO: 124 - Amino acid sequence for human syntaxin 3 (STX3).

SEQ ID NO: 125 - Nucleotide coding sequence for human HEG homolog 1 (HEG1).

SEQ ID NO: 126 - Amino acid sequence for human HEG homolog 1 (HEG1).

SEQ ID NO: 127 - Nucleotide coding sequence for human transmembrane protein 200A, KIAA1913 (TMEM200A).

SEQ ID NO: 128 - Amino acid sequence for human transmembrane protein 200A, KIAA1913 (TMEM200A).

SEQ ID NO: 129 - Nucleotide coding sequence for human G protein-coupled receptor 174 (GPR174).

SEQ ID NO: 130 - Amino acid sequence for human G protein-coupled receptor 174 (GPR174).

SEQ ID NO: 131 - Nucleotide coding sequence for human transmembrane protein 71 (TMEM71).

SEQ ID NO: 132 - Amino acid sequence for human transmembrane protein 71 (TMEM71).

SEQ ID NO: 133 - Nucleotide coding sequence for human ATPase, class V, type 10D (ATP10D).

SEQ ID NO: 134 - Amino acid sequence for human ATPase, class V, type 10D (ATP10D).

SEQ ID NO: 135 - Nucleotide coding sequence for human immediate early response 3 (IER3).

SEQ ID NO: 136 - Amino acid sequence for human immediate early response 3 (IER3).

SEQ ID NO: 137 - Nucleotide coding sequence for human lymphoid-restricted membrane protein (LRMP).

SEQ ID NO: 138 - Amino acid sequence for human lymphoid-restricted membrane protein (LRMP).

SEQ ID NO: 139 - Nucleotide coding sequence for human prostaglandin E receptor 4, subtype EP4 (PTGER4).

SEQ ID NO: 140 - Amino acid sequence for human prostaglandin E receptor 4, subtype EP4 (PTGER4).

SEQ ID NO: 141 - Nucleotide coding sequence for human cysteinyl leukotriene receptor 1 (CYSLTR1).

SEQ ID NO: 142 - Amino acid sequence of human cysteinyl leukotriene receptor 1 (CYSLTR1).

SEQ ID NO: 143 - Nucleotide coding sequence for human ATPase, class I, type 8B, member 4 (ATP8B4).

SEQ ID NO: 144 - Amino acid sequence of human ATPase, class I, type 8B, member 4 (ATP8B4).

SEQ ID NO: 145 - Nucleotide coding sequence for human integrin alpha 2 (ITGA2). SEQ ID NO: 146 - Amino acid sequence of human integrin alpha 2 (ITGA2).

SEQ ID NO: 147 - Nucleotide coding sequence for human CD300A.

SEQ ID NO: 148 - Amino acid sequence of human CD300A.

SEQ ID NO: 149 -Nucleotide coding sequence for human integrin alpha 1 (ITGA1). SEQ ID NO: 150 - Amino acid sequence of human integrin alpha 1 (ITGA1).

SEQ ID NO: 151 - Nucleotide coding sequence for human CD274.

SEQ ID NO: 152 - Amino acid sequence of human CD274.

SEQ ID NO: 153 - Nucleotide coding sequence for human CD9.

SEQ ID NO: 154 - Amino acid sequence of human CD9.

SEQ ID NO: 155 - Nucleotide coding sequence for human semaphorin 3A (SEMA3A). SEQ ID NO: 156 - Amino acid sequence of human semaphorin 3A (SEMA3A).

SEQ ID NO: 157 - Nucleotide coding sequence for isoform 1 of human neuropilin 2 (NRP2).

SEQ ID NO: 158 - Amino acid sequence for isoform 1 of human neuropilin 2 (NRP2). SEQ ID NO: 159 - Nucleotide coding sequence for isoform 2 of human neuropilin 2 (NRP2).

SEQ ID NO: 160 - Amino acid sequence for isoform 2 of human neuropilin 2 (NRP2). SEQ ID NO: 161 - Nucleotide coding sequence for isoform 3 of human neuropilin 2 (NRP2).

SEQ ID NO: 162 - Amino acid sequence for isoform 3 of human neuropilin 2 (NRP2). SEQ ID NO: 163 - Nucleotide coding sequence for isoform 4 of human neuropilin 2 (NRP2).

SEQ ID NO: 164 - Amino acid sequence for isoform 4 of human neuropilin 2 (NRP2). SEQ ID NO: 165 - Nucleotide coding sequence for isoform 5 of human neuropilin 2 (NRP2).

SEQ ID NO: 166 - Amino acid sequence for isoform 5 of human neuropilin 2 (NRP2). SEQ ID NO: 167 - Nucleotide coding sequence for isoform 6 of human neuropilin 2 (NRP2).

SEQ ID NO: 168 - Amino acid sequence for isoform 6 of human neuropilin 2 (NRP2). SEQ ID NO: 169 - Nucleotide coding sequence for human G protein-coupled receptor 183 (GPR183).

SEQ ID NO: 170 - Amino acid sequence of human G protein-coupled receptor 183 (GPR183).

SEQ ID NO: 171 - Nucleotide coding sequence for isoform 1 of human cytochrome b reductase 1 (CYBRD1).

SEQ ID NO: 172 - Amino acid sequence for isoform 1 of human cytochrome b reductase 1 (CYBRD1).

SEQ ID NO: 173 - Nucleotide coding sequence for isoform 2 of human cytochrome b reductase 1 (CYBRD1).

SEQ ID NO: 174 - Amino acid sequence for isoform 2 of human cytochrome b reductase 1 (CYBRD1).

SEQ ID NO: 175 - Nucleotide coding sequence for human activin A receptor, type IIA (ACVR2A).

SEQ ID NO: 176 - Amino acid sequence of human activin A receptor, type IIA (ACVR2A).

SEQ ID NO: 177 - Nucleotide coding sequence for human sortilin 1 (SORT1).

SEQ ID NO: 178 -Amino acid sequence of human sortilin 1 (SORT1).

SEQ ID NO: 179 - Nucleotide coding sequence for isoform a of human CD200 receptor 1 (CD200R1).

SEQ ID NO: 180 - Amino acid sequence for isoform a of human CD200 receptor 1 (CD200R1).

SEQ ID NO: 181 - Nucleotide coding sequence for isoform b of human CD200 receptor 1 (CD200R1).

SEQ ID NO: 182 - Amino acid sequence for isoform b of human CD200 receptor 1 (CD200R1).

SEQ ID NO: 183 - Nucleotide coding sequence for isoform c of human CD200 receptor 1 (CD20ORI).

SEQ ID NO: 184 - Amino acid sequence for isoform c of human CD200 receptor 1 (CD200R1).

SEQ ID NO: 185 - Nucleotide coding sequence for isoform d of human CD200 receptor 1 (CD200R1).

SEQ ID NO: 186 - Amino acid sequence for isoform d of human CD200 receptor 1 (CD200R1).

SEQ ID NO: 187 - Nucleotide coding sequence for human mucolipin 3 (MCOLN3).

SEQ ID NO: 188 - Amino acid sequence of human mucolipin 3 (MCOLN3).

SEQ ID NO: 189 - Nucleotide coding sequence for human tetraspanin 2 (TSPAN2).

SEQ ID NO: 190 - Amino acid sequence of human tetraspanin 2 (TSPAN2).

SEQ ID NO: 191 - Nucleotide coding sequence for human epithelial mitogen homolog (EPGN).

SEQ ID NO: 192 - Amino acid sequence of human epithelial mitogen homolog (EPGN).

SEQ ID NO: 193 - Nucleotide coding sequence for isoform 1 of human phosphatidic acid phosphatase type 2A (PPAP2A).

SEQ ID NO: 194- Amino acid sequence for isoform 1 of human phosphatidic acid phosphatase type 2A (PPAP2A).

SEQ ID NO: 195 - Nucleotide coding sequence for isoform 2 of human phosphatidic acid phosphatase type 2A (PPAP2A).

SEQ ID NO: 196- Amino acid sequence for isoform 2 of human phosphatidic acid phosphatase type 2A (PPAP2A).

SEQ ID NO: 197 - Nucleotide coding sequence for isoform 1 of human adhesion molecule, interacts with CXADR antigen 1 (AMICA1).

SEQ ID NO: 198 - Amino acid sequence for isoform 1 of human adhesion molecule, interacts with CXADR antigen 1 (AMICA1).

SEQ ID NO: 199 - Nucleotide coding sequence for isoform 2 of human adhesion molecule, interacts with CXADR antigen 1 (AMICA1).

SEQ ID NO: 200 - Amino acid sequence for isoform 2 of human adhesion molecule, interacts with CXADR antigen 1 (AMICA1).

SEQ ID NO: 201 - Nucleotide coding sequence for isoform 1 of human prostaglandin E receptor 3 (PTGER3).

SEQ ID NO: 202 - Amino acid sequence for isoform 1 of human prostaglandin E receptor 3 (PTGER3).

SEQ ID NO: 203 - Nucleotide coding sequence for isoform 2 of human prostaglandin E receptor 3 (PTGER3).

SEQ ID NO: 204 - Amino acid sequence for isoform 2 of human prostaglandin E receptor 3 (PTGER3).

SEQ ID NO: 205 - Nucleotide coding sequence for isoform 3 of human prostaglandin E receptor 3 (PTGER3).

SEQ ID NO: 206 - Amino acid sequence for isoform 3 of human prostaglandin E receptor 3 (PTGER3).

SEQ ID NO: 207 - Nucleotide coding sequence for isoform 4 of human prostaglandin E receptor 3 (PTGER3).

SEQ ID NO: 208 - Amino acid sequence for isoform 4 of human prostaglandin E receptor 3 (PTGER3).

SEQ ID NO: 209 - Nucleotide coding sequence for isoform 5 of human prostaglandin E receptor 3 (PTGER3).

SEQ ID NO: 210 - Amino acid sequence for isoform 5 of human prostaglandin E receptor 3 (PTGER3).

SEQ ID NO: 211 - Nucleotide coding sequence for isoform 6 of human prostaglandin E receptor 3 (PTGER3).

SEQ ID NO: 212- Amino acid sequence for isoform 6 of human prostaglandin E receptor 3 (PTGER3).

SEQ ID NO: 213 - Nucleotide coding sequence for isoform 7 of human prostaglandin E receptor 3 (PTGER3).

SEQ ID NO: 214 - Amino acid sequence for isoform 7 of human prostaglandin E receptor 3 (PTGER3).

SEQ ID NO: 215 - Nucleotide coding sequence for isoform 8 of human prostaglandin E receptor 3 (PTGER3).

SEQ ID NO: 216 - Amino acid sequence for isoform 8 of human prostaglandin E receptor 3 (PTGER3).

SEQ ID NO: 217 - Nucleotide coding sequence for isoform 9 of human prostaglandin E receptor 3 (PTGER3).

SEQ ID NO: 218 - Amino acid sequence for isoform 9 of human prostaglandin E receptor 3 (PTGER3).

SEQ ID NO: 219 - Nucleotide coding sequence for isoform 10 of human prostaglandin E receptor 3 (PTGER3).

SEQ ID NO: 220 - Amino acid sequence for isoform 10 of human prostaglandin E receptor 3 (PTGER3).

SEQ ID NO: 221 - Nucleotide coding sequence for isoform a of human protein tyrosine phosphatase, receptor type, K (PTPRK).

SEQ ID NO: 222 - Amino acid sequence for isoform a of human protein tyrosine phosphatase, receptor type, K (PTPRK).

SEQ ID NO: 223 - Nucleotide coding sequence for isoform b of human protein tyrosine phosphatase, receptor type, K (PTPRK).

SEQ ID NO: 224 - Amino acid sequence for isoform b of human protein tyrosine phosphatase, receptor type, K (PTPRK).

SEQ ID NO: 225 -Nucleotide coding sequence for isoform 1 of human CD82.

SEQ ID NO: 226 - Amino acid sequence for isoform 1 of human CD82.

SEQ ID NO: 227 - Nucleotide coding sequence for isoform 2 of human CD82.

SEQ ID NO: 228 - Amino acid sequence for isoform 2 of human CD82.

SEQ ID NO: 229 - Nucleotide coding sequence for human phospholipid scramblase 1 (PLSCR1).

SEQ ID NO: 230 - Amino acid sequence of human phospholipid scramblase 1 (PLSCR1).

SEQ ID NO: 231 - Nucleotide coding sequence for human LAG1 homolog, ceramide synthase 6 (LASS6).

SEQ ID NO: 232 - Amino acid sequence of human LAG1 homolog, ceramide synthase 6 (LASS6).

SEQ ID NO: 233 - Nucleotide coding sequence for human G protein-coupled receptor 15 (GPR15).

SEQ ID NO: 234 - Amino acid sequence of human G protein-coupled receptor 15 (GPR 15).

SEQ ID NO: 235 - Nucleotide coding sequence for human GLI pathogenesis-related 1 protein (GLIPR1).

SEQ ID NO: 236 - Amino acid sequence of human GLI pathogenesis-related 1 protein (GLIPR1).

SEQ ID NO: 237 - Nucleotide coding sequence for human cytotoxic and regulatory T cell molecule (CRTAM).

SEQ ID NO: 238 - Amino acid sequence of human cytotoxic and regulatory T cell molecule (CRTAM).

SEQ ID NO: 239 - Nucleotide coding sequence for human C-type lectin domain family 2, member B (CLEC2B).

SEQ ID NO: 240 - Amino acid sequence of human C-type lectin domain family 2, member B (CLEC2B).

SEQ ID NO: 241 - Nucleotide coding sequence for isoform 1 of human ectonucleotide pyrophosphatase/phosphodiesterase 2 (ENPP2).

SEQ ID NO: 242 - Amino acid sequence for isoform 1 of human ectonucleotide pyrophosphatase/phosphodiesterase 2 (ENPP2).

SEQ ID NO: 243 - Nucleotide coding sequence for isoform 2 of human ectonucleotide pyrophosphatase/phosphodiesterase 2 (ENPP2).

SEQ ID NO: 244 - Amino acid sequence for isoform 2 of human ectonucleotide pyrophosphatase/phosphodiesterase 2 (ENPP2).

SEQ ID NO: 245 - Nucleotide coding sequence for isoform 3 of human ectonucleotide pyrophosphatase/phosphodiesterase 2 (ENPP2).

SEQ ID NO: 246 - Amino acid sequence for isoform 3 of human ectonucleotide pyrophosphatase/phosphodiesterase 2 (ENPP2).

SEQ ID NO: 247 - Nucleotide coding sequence for human SLAM family member 6 (SLAMF6).

SEQ ID NO: 248 - Amino acid sequence of human SLAM family member 6 (SLAMF6).

SEQ ID NO: 249 - Nucleotide coding sequence for human core 1 synthase, glycoprotein-N-acetylgalactosamine 3-beta-galactosyltransferase, 1 (C1GALT1).

SEQ ID NO: 250 - Amino acid sequence of human core 1 synthase, glycoprotein-N-acetylgalactosamine 3-beta-galactosyltransferase, 1 (C1GALT1).

SEQ ID NO: 251 - Nucleotide coding sequence for human adhesion molecule with Ig-like domain 2 (AMIGO2).

SEQ ID NO: 252 - Amino acid sequence of human adhesion molecule with Ig-like domain 2 (AMIGO2).

SEQ ID NO: 253 - Nucleotide coding sequence for human cornichon homolog 4 (CNIH4).

SEQ ID NO: 254 - Amino acid sequence of human cornichon homolog 4 (CNIH4).

SEQ ID NO: 255 - Nucleotide coding sequence for human poliovirus receptor-related 3 (PVRL3).

SEQ ID NO: 256 - Amino acid sequence of human poliovirus receptor-related 3 (PVRL3).

SEQ ID NO: 257 - Nucleotide coding sequence for human G protein-coupled receptor 65 (GPR65).

SEQ ID NO: 258 - Amino acid sequence of human G protein-coupled receptor 65 (GPR65). SEQ ID NO: 259 - Nucleotide coding sequence for isoform a of human sushi domain containing 4 (SUSD4).

SEQ ID NO: 260 - Amino acid sequence for isoform a of human sushi domain containing 4 (SUSD4).

SEQ ID NO: 261 - Nucleotide coding sequence for isoform b of human sushi domain containing 4 (SUSD4).

SEQ ID NO: 262 - Amino acid sequence for isoform b of human sushi domain containing 4 (SUSD4).

SEQ ID NO: 263 - Nucleotide coding sequence for human Ral GEF with PH domain and SH3 binding motif 2 (RALGPS2).

SEQ ID NO: 264 - Amino acid sequence of human Ral GEF with PH domain and SH3 binding motif 2 (RALGPS2).

SEQ ID NO: 265 - Nucleotide coding sequence for human interleukin 18 receptor accessory protein (IL18RAP).

SEQ ID NO: 266 - Amino acid sequence of human interleukin 18 receptor accessory protein (IL18RAP).

SEQ ID NO: 267 - Nucleotide coding sequence for human RGM domain family member B.

SEQ ID NO: 268 - Amino acid sequence of human RGM domain family member B. SEQ ID NO: 269 - Nucleotide coding sequence for human ATPase, H+ transporting, lysosomal VO subunit al (ATP6VOA1) isoform a.

SEQ ID NO: 270 - Amino acid sequence of human ATPase, H+ transporting, lysosomal V0 subunit al (ATP6VOA1) isoform a.

SEQ ID NO: 271 - Nucleotide coding sequence for human ATPase, H+ transporting, lysosomal VO subunit al (ATP6V0A1) isoform b.

SEQ ID NO: 272 - Amino acid sequence of human ATPase, H+ transporting, lysosomal VO subunit al (ATP6V0A1) isoform b.

SEQ ID NO: 273 - Nucleotide coding sequence for human ATPase, H+ transporting, lysosomal V0 subunit al (ATP6V0A1) isoform c.

SEQ ID NO: 274 - Amino acid sequence of human ATPase, H+ transporting, lysosomal V0 subunit al (ATP6V0A1) isoform c.

SEQ ID NO: 275 - Nucleotide coding sequence for human interleukin 6 receptor (IL6R) isoform 1.

SEQ ID NO: 276 - Amino acid sequence of human interleukin 6 receptor (IL6R) isoform 1.

SEQ ID NO: 277 - Nucleotide coding sequence for human interleukin 6 receptor (IL6R) isoform 2.

SEQ ID NO: 278 - Amino acid sequence of human interleukin 6 receptor (IL6R) isoform 2.

SEQ ID NO: 279 - Nucleotide coding sequence for human natural killer cell group 7 sequence (NKG7).

SEQ ID NO: 280 - Amino acid sequence of human natural killer cell group 7 sequence (NKG7).

SEQ ID NO: 281 - Nucleotide coding sequence for human CD4.

SEQ ID NO: 282 - Amino acid sequence of human CD4.

SEQ ID NO: 283 -Nucleotide coding sequence for human CD25.

SEQ ID NO: 284 - Amino acid sequence of human CD25.

SEQ ID NO: 285 - Nucleotide coding sequence for human FoxP3.

SEQ ID NO: 286 - Amino acid sequence of human FoxP3.

### Selected Definitions

The term "and/or", e.g., "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As used herein, the term "effective amount" shall be taken to mean a sufficient quantity of a compound to bind to the target protein *in vivo* and, preferably, to reduce or inhibit or prevent Treg cell activity *in vivo,* compared to the same level in a subject or cell, tissue or organ thereof prior to administration and/or compared to a subject or cell, tissue or organ thereof from a subject of the same species to which the compound has not been administered. Preferably, the term "effective amount" means a sufficient quantity of the compound to reduce, prevent, or ameliorate a Treg-mediated condition and/or to kill Treg cells in a subject. The skilled artisan will be aware that such an amount will vary depending on, for example, the specific compounds administered and/or the particular subject and/or the type or severity or level of disease. Accordingly, this term is not to be construed to limit the invention to a specific quantity, e.g., weight or amount of compound(s) rather the present invention encompasses any amount of the compound(s) sufficient to achieve the stated result in a subject.

As used herein, the term "therapeutically effective amount" shall be taken to mean a sufficient quantity of a compound to reduce or inhibit one or more symptoms of a clinical disease associated with or caused by Treg cells to a level that is below that observed and accepted as clinically diagnostic or clinically characteristic of that disease. The skilled artisan will be aware that such an amount will vary depending on, for example, the specific compound(s) administered and/or the particular subject and/or the type or severity or level of disease. Accordingly, this term is not to be construed to limit the invention to a specific quantity, e.g., weight or amount of compound(s), rather the present invention encompasses any amount of the compound(s) sufficient to achieve the stated result in a subject.

As used herein, the term "prophylactically effective amount" shall be taken to mean a sufficient quantity of a compound to prevent or inhibit or delay the onset of one or more detectable symptoms of a clinical condition associated with or caused by Treg cells. The skilled artisan will be aware that such an amount will vary depending on, for example, the specific compound(s) administered and/or the particular subject and/or the type or severity or level of disease and/or predisposition (genetic or otherwise) to the disease. Accordingly, this term is not to be construed to limit the invention to a specific quantity, e.g., weight or amount of compound(s), rather the present invention encompasses any amount of the compound(s) sufficient to achieve the stated result in a subject.

As used herein, the term "immune response" refers to the concerted action of lymphocytes, antigen presenting cells, phagocytic cells, granulocytes, and soluble macromolecules produced by the above cells or the liver (including antibodies, cytokines, and complement) that results in selective damage to, destruction of, or elimination from the body of a subject of cancerous cells, metastatic tumor cells, malignant melanoma, invading pathogens, cells or tissues infected with pathogens, or, in cases of autoimmunity or pathological inflammation, normal cells or tissues of a subject.

As used herein, the term "specifically binds" shall be taken to mean a compound reacts or associates more frequently, more rapidly, with greater duration and/or with greater affinity with a particular cell or substance than it does with alternative cells or substances. For example, a compound that specifically binds to a target protein is a compound that binds that protein or an epitope or immunogenic fragment thereof with greater affinity, avidity, more readily, and/or with greater duration than it binds to unrelated protein and/or epitopes or immunogenic fragments thereof. It is also understood by reading this definition that, for example, a compound that specifically binds to a first target may or may not specifically bind to a second target. As such, "specific binding" does not necessarily require exclusive binding or non-detectable binding of another molecule, this is encompassed by the term "selective binding". Generally, but not necessarily, reference to binding means specific binding.

As used herein, the terms "treating", "treat" or "treatment" include administering a therapeutically effective amount of a compound described herein sufficient to reduce or eliminate at least one symptom of a specified disease or condition.

As used herein, the terms "preventing", "prevent" or "prevention" include administering a therapeutically effective amount of an inhibitor(s) and/or agent(s) described herein sufficient to stop or hinder the development of at least one symptom of a specified disease or condition.

As used herein, the term "Treg" or "regulatory T cell" shall be understood to mean a T cell expresses at least CD4 and CD25 and FOXP3, and that is capable of reducing or suppressing the activity of a T cell other than a Treg cell and/or killing a T cell. This term includes T cells which produce low levels of IL-2, IL-4, IL-5, and IL-1, and acts to suppress activation of the immune system. Regulatory T cells actively suppress the proliferation and cytokine production of Th1, Th2, or naive T cells which have been stimulated in culture with an activating signal (e.g., antigen and antigen presenting cells or with a signal that mimics antigen in the context of MHC, e.g., anti-CD3 antibody, plus anti-CD28 antibody). This term also encompasses mutant forms of Treg cells, e.g., a cell which is derived from Treg cells but exhibits at least one difference at the phenotypic or functional or structural level. For example, the mutant or variant may have been altered to express a therapeutic protein or a protein that alters or enhances Treg cell function. Such changes can occur either spontaneously or as a result of a directed manipulation, such as would occur if a cell was deliberately transformed (for example, in order to effect the creation of a cell line) or transfected.

As used herein, the term "enriched" or "enrich" in the context of a cell population shall be taken to mean that the number or percentage of Treg cells is greater than the number or percentage in a naturally occurring cell population. For example, a population enriched in Treg cells is made up of at least about 0.02% of said cells, or at least about 0.05% of said cells or at least about 0.1 % of said cells or at least about 0.2% of said cells or at least about 0.5% of said cells or at least about 0.5% of said cells or at least about 0.8% of said cells or at least about 1% of said cells or at least about 2% of said cells or at least about 3% of said cells or at least about 4% of said cells or at least about 5% of said cells or at least about 10% of said cells or at least about 15% of said cells or at least about 20% of said cells or at least about 25% of said cells or at least about 30% of said cells or at least about 40% of said cells or at least about 50% of said cells or at least about 60% of said cells or at least about 70% of said cells or at least about 80% of said cells or at least about 85% of said cells or at least about 90% of said cells or at least about 95% of said cells or at least about 97% of said cells or at least about 98% of said cells or at least about 99% of said cells.

As used herein, the term "non-Treg cell" or "cell other than a Treg cell" includes T cells which function to eliminate antigen (e.g., by producing cytokines which modulate the activation of other cells or by cytotoxic activity). This term includes Thelper cells (e.g., Th1 and Th2 cells) and cytotoxic T cells. In this respect, Thelper cells preferably express CD4 and express low or undetectable levels of CD25. CTL cells preferably express CD8 and low or undetectable levels of CD4. Preferably, a non-Treg cell does not express both CD4 and CD25. Preferably, a non-Treg cell does not express FoxP3 (however, it not a requirement that expression of FoxP3 is detected to determine whether or not a cell is a Treg cell).

The term "Treg-associated condition" shall be taken to encompass any disease or disorder or state in which modulation of Treg numbers and/or activity may provide a beneficial effect. This term encompasses conditions associated with regulatory T cell-mediated suppression of a subject's immune system, e.g., conditions associated with or caused by an excessive immune response (e.g., by a Thelper cell or a CTL or other cell regulated by one or more T cells). Accordingly, this term encompasses inflammatory conditions and/or autoimmune conditions. Exemplary Treg-associated conditions include, an inflammatory disorder of the nervous system (e.g., multiple sclerosis), or a mucosal inflammatory disease (e.g., inflammatory bowel disease, asthma or tonsillitis), or an inflammatory skin disease (e.g., dermatitis, psoriasis or contact hypersensitivity) or autoimmune arthritis (e.g., rheumatoid arthritis). Preferred Treg associated conditions include rheumatoid arthritis. In one embodiment, the inflammatory disorder is an allergic inflammatory disorder. An immune response against a graft or graft versus host disease or host versus graft disease is also a Treg-associated condition. This term also includes cancer, e.g., in which Treg cells suppress the activity of immune cells against cancerous cells, thereby permitting the disease to develop.

As used herein, the term "subject" shall be taken to mean any subject comprising Treg cells, preferably a mammal. Exemplary subjects include but are not limited to human, primate, livestock (e.g. sheep, cow, horse, donkey, pig), companion animals (e.g. dogs, cats), laboratory test animals (e.g. mice, rabbits, rats, guinea pigs, hamsters), captive wild animal (e.g. fox, deer). Preferably the mammal is a human or primate. Most preferably the mammal is a human.

The term "sample" shall be taken to encompass the recited sample (e.g., a blood sample) and any fraction thereof (e.g., plasma, serum or buffy coat) or cells derived therefrom (e.g., peripheral blood mononuclear cells) or process forms thereof.

### General

This specification contains nucleotide and amino acid sequence information prepared using PatentIn Version 3.5. Each nucleotide sequence is identified in the sequence listing by the numeric indicator <210> followed by the sequence identifier (e.g. <210>1, <210>2, <210>3, etc). The length and type of sequence (DNA, protein (PRT), etc), and source organism for each nucleotide sequence, are indicated by information provided in the numeric indicator fields <211>, <212> and <213>, respectively. Nucleotide sequences referred to in the specification are defined by the term "SEQ ID NO:", followed by the sequence identifier (e.g. SEQ ID NO: 1 refers to the sequence in the sequence listing designated as <400>1).

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

The present invention is not to be limited in scope by the specific embodiments described herein, which are intended for the purpose of exemplification only. Functionally-equivalent products, compositions and methods are clearly within the scope of the invention, as described herein.

Any embodiment herein shall be taken to apply *mutatis mutandis* to any other embodiment unless specifically stated otherwise.

Unless specifically defined otherwise, all technical and scientific terms used herein shall be taken to have the same meaning as commonly understood by one of ordinary skill in the art (for example, in cell culture, molecular genetics, immunology, immunohistochemistry, protein chemistry, and biochemistry).

Unless otherwise indicated, the recombinant protein, cell culture, and immunological techniques utilized in the present invention are standard procedures, well known to those skilled in the art. Such techniques are described and explained throughout the literature in sources such as, Perbal, (1984), Sambrook *et al.,* (1989), Brown (1991), Glover and Hames (1995 and 1996), Ausubel *et al.,* (1988, including all updates until present), Harlow and Lane (1988), Coligan *et al.,* (including all updates until present) and Zola (1987).

### Detailed Description of the Preferred Embodiments

### Treg Markers and Encoding Nucleic Acids

Preferred Treg protein markers and nucleic acids encoding same are set out in the claims, with reference to the tables below.

**Table 2: Protein biomarkers and nucleic acids encoding same that are upregulated on/in Treg cells.**

| **Gene name** | **Entrez Accession Number** | **Nucleotide SEQ ID NO:** | **Amino acid SEQ ID NO:** |
|---|---|---|---|
| Peptidase inhibitor 16 (PI 16) | 221476 | 27 | 28 |
| Interleukin 1 receptor, type I (IL1R1) (CD121) | 3554 | 15 | 16 |
| Polycystic kidney disease 1-like 3 (PKD1L3) | 342372 | 83 | 84 |
| Isoform (a) of protein tyrosine phosphatase, receptor type B (PTPRB) | 5787 | 21 | 22 |
| Isoform (b) of protein tyrosine phosphatase, receptor type B (PTPRB) | 5787 | 23 | 24 |
| Melanoma cell adhesion molecule (MCAM) (CD146) | 4162 | 33 | 34 |
| Isoform alpha 1 of human chromosome 18 open reading frame 1 (C18orfl) | 753 | 3 | 4 |
| Isoform alpha 2 of human chromosome 18 open reading frame 1 (C18orfl) | 753 | 5 | 6 |
| Isoform beta 1 of human chromosome 18 open reading frame 1 (C18orfl) | 753 | 7 | 8 |
| Isoform beta 2 of human chromosome 18 open reading frame 1 (C18orfl) | 753 | 9 | 10 |
| Isoform gamma 1 of human chromosome 18 open reading frame 1 (C18orfl) | 753 | 11 | 12 |
| Isoform gamma 2 of human chromosome 18 open reading frame 1 (C18orfl) | 753 | 13 | 14 |
| Aquaporin 3 (AQP3) | 360 | 37 | 38 |
| RGMB | 285704 | 267 | 268 |
| Isoform A of integrin alpha 6 (ITGA6) (CD49f) | 3655 | 39 | 40 |
| Isoform B of integrin alpha 6 (ITGA6) (CD49f) | 3655 | 41 | 42 |
| p53 apoptosis effector related to PMP22 (PERP) | 64065 | 75 | 76 |
| Isoform 1 of lipolysis stimulated lipoprotein receptor (LSR) | 51599 | 43 | 44 |
| Isoform 2 of lipolysis stimulated lipoprotein receptor (LSR) | 51599 | 45 | 46 |
| Isoform 3 of lipolysis stimulated lipoprotein receptor (LSR) | 51599 | 47 | 48 |
| WDFY family member 4 (WDFY4) | 57705 | 99 | 100 |
| ATP6V0A1 isoform a | 535 | 269 | 270 |
| ATP6V0A1 isoform b | 535 | 271 | 272 |
| ATP6V0A1 isoform c | 535 | 273 | 274 |
| ST8 alpha-N-acetyl-neuraminide alpha-2,8-sialyltransferase 6 (ST8SIA6) | 338596 | 87 | 88 |
| Isoform 1 of human chromosome 6 open reading frame 105 (C6orf105) | 84830 | 89 | 90 |
| Isoform 2 of human chromosome 6 open reading frame 105 (C6orf105) | 84830 | 91 | 92 |
| NIPA-like domain containing 2 (NPAL2) | 79815 | 95 | 96 |
| Variant 1 of transmembrane protein 169 (TMEM169) | 92691 | 103 | 104 |
| Variant 2 of transmembrane protein 169 (TMEM169) | 92691 | 105 | 106 |
| Variant 3 of transmembrane protein 169 (TMEM169) | 92691 | 107 | 108 |
| Variant 4 of transmembrane protein 169 (TMEM169) | 92691 | 109 | 110 |
| FRAS1 related extracellular matrix 3 (FREM3) | 166752 | 97 | 98 |
| EPH receptor B1 (EPHB1) | 2047 | 55 | 56 |
| Fc fragment of IgE, high affinity I, receptor for; gamma polypeptide (FCER1G) | 2207 | 101 | 102 |
| Tetraspanin 15 (TSPAN15) | 23555 | 93 | 94 |
| Plexin B2 (PLXNB2) | 23654 | 59 | 60 |
| V-set and immunoglobulin domain containing 1 protein (VSIG1) | 340547 | 73 | 74 |
| Neural proliferation, differentiation and control 1 protein (NPDC1) | 56654 | 53 | 54 |
| IL6R isoform 1 | 3570 | 275 | 276 |
| IL6R isoform 2 | 3570 | 277 | 278 |
| T cell immunoreceptor with Ig and ITIM domains (TIGIT) | 201633 | 85 | 86 |
| Isoform 1 of CD79A | 973 | 17 | 18 |
| Isoform 2 of CD79A | 973 | 19 | 20 |
| Interferon gamma receptor 2 (IFNGR2) | 3460 | 25 | 26 |
| Isoform 1 of CD33 | 945 | 29 | 30 |
| Isoform 2 of CD33 | 945 | 31 | 32 |
| Integrin alpha M (ITGAM) (CD11b) | 3684 | 35 | 36 |
| Isoform 1 of carcinoembryonic antigen-related cell adhesion molecule 1 (CEACAM1) (CD66) | 634 | 49 | 50 |
| Isoform 2 of carcinoembryonic antigen-related cell adhesion molecule 1 (CEACAM1) (CD66) | 634 | 51 | 52 |
| Isoform 1 of CD79B | 974 | 77 | 78 |
| Isoform 2 of CD79B | 974 | 79 | 80 |
| Isoform 3 of CD79B | 974 | 81 | 82 |
| Isoform 1 of CD8B | 926 | 63 | 64 |
| Isoform 2 of CD8B | 926 | 65 | 66 |
| Isoform 3 of CD8B | 926 | 67 | 68 |
| Isoform 4 of CD8B | 926 | 69 | 70 |
| Isoform 5 of CD8B | 926 | 71 | 72 |
| Toll-like receptor 6 (TLR6) | 10333 | 57 | 58 |
| Low density lipoprotein receptor-related protein 6 (LRP6) | 4040 | 61 | 62 |
| Sphingomyelin phosphodiesterase 3 (SMPD3) | 55512 | 1 | 2 |

**Table 3: Protein biomarkers and nucleic acids encoding same that are downregulated on/in Treg cells.**

| **Gene name** | **Entrez Accession Number** | **Nucleotide SEQ ID NO:** | **Amino acid SEQ ID NO:** |
|---|---|---|---|
| ATPase, class I, type 8B, member 4 (ATP8B4) | 79895 | 143 | 144 |
| Mucolipin 2 (MCOLN2) | 255231 | 111 | 112 |
| Cysteinyl leukotriene receptor 1 (CYSLTR1) | 10800 | 141 | 142 |
| Integrin alpha 2 (ITGA2) | 3673 | 145 | 146 |
| G protein-coupled receptor 183 (GPR183) | 1880 | 169 | 170 |
| C-type lectin-like 1 (CLECL1) | 160365 | 113 | 114 |
| Cytotoxic and regulatory T cell molecule (CRTAM) | 56253 | 237 | 238 |
| CD9 | 928 | 153 | 154 |
| Isoform 1 of ectonucleotide pyrophosphatase/phosphodiesterase 2 (ENPP2) | 5168 | 241 | 242 |
| Isoform 2 of ectonucleotide pyrophosphatase/phosphodiesterase 2 (ENPP2) | 5168 | 243 | 244 |
| Isoform 3 of ectonucleotide pyrophosphatase/phosphodiesterase 2 (ENPP2) | 2168 | 245 | 246 |
| HEG homolog 1 (HEG1) | 57493 | 125 | 126 |
| Immediate early response 3 (IER3) | 8870 | 135 | 136 |
| CD274 | 29126 | 151 | 152 |
| Integrin alpha 1 (ITGA1) | 3672 | 149 | 150 |
| NKG7 | 4818 | 279 | 280 |
| SLAM family member 6 (SLAMF6) | 114836 | 247 | 248 |
| Sortilin 1 (SORT1) | 6272 | 177 | 178 |
| Tetraspanin 2 (TSPAN2) | 10100 | 189 | 190 |
| Lysophosphatidylcholine acyltransferase 2 (LPCAT2) | 54947 | 115 | 116 |
| Phospholipid scramblase 1 (PLSCR1) | 5359 | 229 | 230 |
| semaphorin 3A (SEMA3A) | 10371 | 155 | 156 |
| CD300A | 11314 | 147 | 148 |
| Isoform 1 of neuropilin 2 (NRP2) | 8828 | 157 | 158 |
| Isoform 2 of neuropilin 2 (NRP2) | 8828 | 159 | 160 |
| Isoform 3 of neuropilin 2 (NRP2) | 8828 | 161 | 162 |
| Isoform 4 of neuropilin 2 (NRP2) | 8828 | 163 | 164 |
| Isoform 5 of neuropilin 2 (NRP2) | 8828 | 165 | 166 |
| Isoform 6 of neuropilin 2 (NRP2) | 8828 | 167 | 168 |
| Mucolipin 3 (MCOLN3) | 55283 | 187 | 188 |
| Activin A receptor, type IIA (ACVR2A) | 92 | 175 | 176 |
| Epithelial mitogen homolog (EPGN) | 255324 | 191 | 192 |
| Syntaxin 3 (STX3) | 6809 | 123 | 124 |
| Variant 1 of leucine rich repeat neuronal 3 (LRRN3) | 54674 | 117 | 118 |
| Variant 2 of leucine rich repeat neuronal 3 (LRRN3) | 54674 | 119 | 120 |
| Variant 3 of leucine rich repeat neuronal 3 (LRRN3) | 54674 | 121 | 122 |
| Isoform a of CD200 receptor 1 (CD200R1) | 131450 | 179 | 180 |
| Isoform b of CD200 receptor 1 (CD200R1) | 131450 | 181 | 182 |
| Isoform c of CD200 receptor 1 (CD200R1) | 131450 | 183 | 184 |
| Isoform d of CD200 receptor 1 (CD200R1) | 131450 | 185 | 186 |
| isoform 1 of cytochrome b reductase 1 (CYBRD1) | 79901 | 171 | 172 |
| isoform 2 of cytochrome b reductase 1 (CYBRD1) | 79901 | 173 | 174 |
| Transmembrane protein 71 (TMEM71) | 137835 | 131 | 132 |
| LAG1 homolog, ceramide synthase 6 (LASS6) | 253782 | 231 | 232 |
| Transmembrane protein 200A, KIAA1913 (TMEM200A) | 114801 | 127 | 128 |
| isoform 1 of CD82 | 3732 | 225 | 226 |
| isoform 2 of CD82 | 3732 | 227 | 228 |
| Poliovirus receptor-related 3 (PVRL3) | 25945 | 255 | 256 |
| Isoform a of protein tyrosine phosphatase, receptor type, K (PTPRK) | 5796 | 221 | 222 |
| Isoform b of protein tyrosine phosphatase, receptor type, K (PTPRK) | 5796 | 223 | 224 |
| C-type lectin domain family 2, member B (CLEC2B) | 9976 | 239 | 240 |
| Lymphoid-restricted membrane protein (LRMP) | 4033 | 137 | 138 |
| Adhesion molecule with Ig-like domain 2 (AMIGO2) | 347902 | 251 | 252 |
| ATPase, class V, type 10D (ATP10D) | 57205 | 133 | 134 |
| Ral GEF with PH domain and SH3 binding motif 2 (RALGPS2) | 55103 | 263 | 264 |
| Isoform 1 of prostaglandin E receptor 3 (PTGER3) | 5733 | 201 | 202 |
| Isoform 2 of prostaglandin E receptor 3 (PTGER3) | 5733 | 203 | 204 |
| Isoform 3 of prostaglandin E receptor 3 (PTGER3) | 5733 | 205 | 206 |
| Isoform 4 of prostaglandin E receptor 3 (PTGER3) | 5733 | 207 | 208 |
| Isoform 5 of prostaglandin E receptor 3 (PTGER3) | 5733 | 209 | 210 |
| Isoform 6 of prostaglandin E receptor 3 (PTGER3) | 5733 | 211 | 212 |
| Isoform 7 of prostaglandin E receptor 3 (PTGER3) | 5733 | 213 | 214 |
| Isoform 8 of prostaglandin E receptor 3 (PTGER3) | 5733 | 215 | 216 |
| Isoform 9 of prostaglandin E receptor 3 (PTGER3) | 5733 | 217 | 218 |
| Isoform 10 of prostaglandin E receptor 3 (PTGER3) | 5733 | 219 | 220 |
| G protein-coupled receptor 174 (GPR174) | 84636 | 129 | 130 |
| Isoform a of sushi domain containing 4 (SUSD4) | 55061 | 259 | 260 |
| Isoform a of sushi domain containing 4 (SUSD4) | 55061 | 261 | 262 |
| Interleukin 18 receptor accessory protein (IL18RAP) | 8807 | 265 | 266 |
| Isoform 1 of phosphatidic acid phosphatase type 2A (PPAP2A) | 8611 | 193 | 194 |
| Isoform 2 of phosphatidic acid phosphatase type 2A (PPAP2A) | 8611 | 195 | 196 |
| Isoform 1 of adhesion molecule, interacts with CXADR antigen 1 (AMICA1) | 120425 | 197 | 198 |
| Isoform 2 of adhesion molecule, interacts with CXADR antigen 1 (AMICA1) | 120425 | 199 | 200 |
| GLI pathogenesis-related 1 protein (GLIPR1) | 11010 | 235 | 236 |
| G protein-coupled receptor 65 (GPR65) | 8477 | 257 | 258 |
| G protein-coupled receptor 15 (GPR15) | 2838 | 233 | 234 |
| Prostaglandin E receptor 4, subtype EP4 (PTGER4) | 5734 | 139 | 140 |
| Cornichon homolog 4 (CNIH4) | 29097 | 253 | 254 |
| Core 1 synthase, glycoprotein-N-acetylgalactosamine 3-beta-galactosyltransferase, 1 (C1GALT1) | 56913 | 249 | 250 |

**Table 4: Preferred cell surface proteins and nucleic acids encoding same that are upregulated on/in Treg cells.**

| **Gene name** | **Entrez Accession Number** | **Nucleotide SEQ ID NO:** | **Amino acid SEQ ID NO:** |
|---|---|---|---|
| Peptidase inhibitor 16 (PI 16) | 221476 | 27 | 28 |
| Interleukin 1 receptor, type I (IL1R1) (CD121) | 3554 | 15 | 16 |
| Polycystic kidney disease 1-like 3 (PKD1L3) | 342372 | 83 | 84 |
| Isoform (a) of protein tyrosine phosphatase, receptor type B (PTPRB) | 5787 | 21 | 22 |
| Isoform (b) of protein tyrosine phosphatase, receptor type B (PTPRB) | 5787 | 23 | 24 |
| Melanoma cell adhesion molecule (MCAM) (CD146) | 4162 | 33 | 34 |
| Isoform alpha 1 of human chromosome 18 open reading frame 1 (C18orfl) | 753 | 3 | 4 |
| Isoform alpha 2 of human chromosome 18 open reading frame 1 (C18orfl) | 753 | 5 | 6 |
| Isoform beta 1 of human chromosome 18 open reading frame 1 (C18orfl) | 753 | 7 | 8 |
| Isoform beta 2 of human chromosome 18 open reading frame 1 (C18orfl) | 753 | 9 | 10 |
| Isoform gamma 1 of human chromosome 18 open reading frame 1 (C18orfl) | 753 | 11 | 12 |
| Isoform gamma 2 of human chromosome 18 open reading frame 1 (C18orfl) | 753 | 13 | 14 |
| Aquaporin 3 (AQP3) | 360 | 37 | 38 |
| RGMB | 285704 | 267 | 268 |
| Isoform A of integrin alpha 6 (ITGA6) (CD49f) | 3655 | 39 | 40 |
| Isoform B of integrin alpha 6 (ITGA6) (CD49f) | 3655 | 41 | 42 |
| p53 apoptosis effector related to PMP22 (PERP) | 64065 | 75 | 76 |
| Isoform 1 of lipolysis stimulated lipoprotein receptor (LSR) | 51599 | 43 | 44 |
| Isoform 2 of lipolysis stimulated lipoprotein receptor (LSR) | 51599 | 45 | 46 |
| Isoform 3 of lipolysis stimulated lipoprotein receptor (LSR) | 51599 | 47 | 48 |
| WDFY family member 4 (WDFY4) | 57705 | 99 | 100 |
| ATP6VOA1 isoform a | 535 | 269 | 270 |
| ATP6VOA1 isoform b | 535 | 271 | 272 |
| ATP6VOA1 isoform c | 535 | 273 | 274 |
| ST8 alpha-N-acetyl-nemaminide alpha-2,8-sialyltransferase 6 (ST8SIA6) | 338596 | 87 | 88 |
| Isoform 1 of human chromosome 6 open reading frame 105 (C6orf105) | 84830 | 89 | 90 |
| Isoform 2 of human chromosome 6 open reading frame 105 (C6orf105) | 84830 | 91 | 92 |
| NIPA-like domain containing 2 (NPAL2) | 79815 | 95 | 96 |
| Variant 1 of transmembrane protein 169 (TMEM169) | 92691 | 103 | 104 |
| Variant 2 of transmembrane protein 169 (TMEM169) | 92691 | 105 | 106 |
| Variant 3 of transmembrane protein 169 (TMEM169) | 92691 | 107 | 108 |
| Variant 4 of transmembrane protein 169 (TMEM169) | 92691 | 109 | 110 |
| FRAS1 related extracellular matrix 3 (FREM3) | 166752 | 97 | 98 |
| IL6R isoform 1 | 3570 | 275 | 276 |
| IL6R isoform 2 | 3570 | 277 | 278 |
| T cell immunoreceptor with Ig and ITIM domains (TIGIT) | 201633 | 85 | 86 |

**Table 5: Protein biomarkers and nucleic acids encoding same that are upregulated on/in activated Treg cells.**

| **Gene name** | **Entrez Accession Number** | **Nucleotide SEQ ID NO:** | **Amino acid SEQ ID NO:** |
|---|---|---|---|
| Interleukin 1 receptor, type I (IL1R1) (CD121) | 3554 | 15 | 16 |
| Cysteinyl leukotriene receptor 1 (CYSLTR1) | 10800 | 141 | 142 |
| Integrin alpha 2 (ITGA2) | 3673 | 145 | 146 |
| G protein-coupled receptor 183 (GPR183) | 1880 | 169 | 170 |
| Cytotoxic and regulatory T cell molecule (CRTAM) | 56253 | 237 | 238 |
| Isoform 1 of ectonucleotide pyrophosphatase/phosphodiesterase 2 (ENPP2) | 5168 | 241 | 242 |
| Isoform 2 of ectonucleotide pyrophosphatase/phosphodiesterase 2 (ENPP2) | 5168 | 243 | 244 |
| Isoform 3 of ectonucleotide pyrophosphatase/phosphodiesterase 2 (ENPP2) | 2168 | 245 | 246 |
| Adhesion molecule with Ig-like domain 2 (AMIGO2) | 347902 | 251 | 252 |
| Poliovirus receptor-related 3 (PVRL3) | 25945 | 255 | 256 |
| Interleukin 18 receptor accessory protein (IL18RAP) | 8807 | 265 | 266 |
| T cell immunoreceptor with Ig and ITIM domains (TIGIT) | 201633 | 85 | 86 |
| HEG homolog 1 (HEG1) | 57493 | 125 | 126 |
| G protein-coupled receptor 174 (GPR174) | 84636 | 129 | 130 |
| Immediate early response 3 (IER3) | 8870 | 135 | 136 |
| Prostaglandin E receptor 4, subtype EP4 (PTGER4) | 5734 | 139 | 140 |

**Table 6: Protein biomarkers and nucleic acids encoding same that are downregulated on/in activated Treg cells.**

| **Gene name** | **Entrez Accession Number** | **Nucleotide SEQ ID NO:** | **Amino acid SEQ ID NO:** |
|---|---|---|---|
| IL6R isoform 1 | 3570 | 275 | 276 |
| IL6R isoform 2 | 3570 | 277 | 278 |
| Isoform A of integrin alpha 6 (ITGA6) | 3655 | 39 | 40 |
| Isoform B of integrin alpha 6 (ITGA6) | 3655 | 41 | 42 |
| ST8 alpha-N-acetyl-neuraminide alpha-2,8-sialyltransferase 6 (ST8SIA6) | 338596 | 87 | 88 |
| NIPA-like domain containing 2 (NPAL2) | 79815 | 95 | 96 |
| Variant 1 of transmembrane protein 169 (TMEM169) | 92691 | 103 | 104 |
| Variant 2 of transmembrane protein 169 (TMEM169) | 92691 | 105 | 106 |
| Variant 3 of transmembrane protein 169 (TMEM169) | 92691 | 107 | 108 |
| Variant 4 of transmembrane protein 169 (TMEM169) | 92691 | 109 | 110 |
| V-set and immunoglobulin domain containing 1 protein (VSIG1) | 340547 | 73 | 74 |
| Isoform 1 of CD79B | 974 | 77 | 78 |
| Isoform 2 of CD79B | 974 | 79 | 80 |
| Isoform 3 of CD79B | 974 | 81 | 82 |
| Toll-like receptor 6 (TLR6) | 10333 | 57 | 58 |
| Sphingomyelin phosphodiesterase 3 (SMPD3) | 55512 | 1 | 2 |
| SLAM family member 6 (SLAMF6) | 114836 | 247 | 248 |
| Syntaxin 3 (STX3) | 6809 | 123 | 124 |
| Lymphoid-restricted membrane protein (LRMP) | 4033 | 137 | 138 |

The claims are limited to PI16.

A preferred protein or nucleic acid comprises a sequence at least about 75% amino acid sequence identity to the amino acid sequence set forth in any one or more of Tables 2-6, preferably at least about 80% sequence identity, preferably at least about 85%, more preferably at least about 90%, even more preferably at least about 95% and still more preferably at least about 99%. The present invention is not to be restricted to the use of the exemplified *Homo sapiens* nucleic acids or proteins because, as will be known to those skilled in the art, it is possible to identify naturally-occurring variants and/or mutants of said nucleic acids and/or proteins using standard techniques, including *in silico* analysis, e.g., using BLAST.

The % identity of a nucleic acid or polypeptide is determined by GAP (Needleman and Wunsch, 1970) analysis (GCG program) with a gap creation penalty=5, and a gap extension penalty=0.3. The query sequence is at least 50 residues in length, and the GAP analysis aligns the two sequences over a region of at least 50 residues. Even more preferably, the query sequence is at least 100 residues in length and the GAP analysis aligns the two sequences over a region of at least 100 residues. Most preferably, the two sequences are aligned over their entire length.

In one example, reference herein to a protein or nucleic acid shall be taken to be a reference to a human sphingomyelin phosphodiesterase 3 (SMPD3) or nucleic acid encoding same.

In one example, reference herein to a protein or nucleic acid shall be taken to be a reference to a human chromosome 18 open reading frame 1 (C18orfl) protein or nucleic acid encoding same.

In one example, reference herein to a protein or nucleic acid shall be taken to be a reference to a human interleukin 1 receptor, type I (IL 1R1) or nucleic acid encoding same.

In one example, reference herein to a protein or nucleic acid shall be taken to be a reference to a human CD79A or nucleic acid encoding same.

In one example, reference herein to a protein or nucleic acid shall be taken to be a reference to a human protein tyrosine phosphatase, receptor type B (PTPRB) or nucleic acid encoding same.

In one example, reference herein to a protein or nucleic acid shall be taken to be a reference to a human interferon gamma receptor 2 (IFNGR2) or nucleic acid encoding same.

In one example, reference herein to a protein or nucleic acid shall be taken to be a reference to a human peptidase inhibitor 16 (PI16) or nucleic acid encoding same.

In one example, reference herein to a protein or nucleic acid shall be taken to be a reference to a human CD33 or nucleic acid encoding same.

In one example, reference herein to a protein or nucleic acid shall be taken to be a reference to a human melanoma cell adhesion molecule (MCAM) or nucleic acid encoding same.

In one example, reference herein to a protein or nucleic acid shall be taken to be a reference to a human integrin alpha M (ITGAM) or nucleic acid encoding same.

In one example, reference herein to a protein or nucleic acid shall be taken to be a reference to a human aquaporin 3 (AQP3) or nucleic acid encoding same.

In one example, reference herein to a protein or nucleic acid shall be taken to be a reference to a human integrin alpha 6 (ITGA6) or nucleic acid encoding same.

In one example, reference herein to a protein or nucleic acid shall be taken to be a reference to a human lipolysis stimulated lipoprotein receptor (LSR) or nucleic acid encoding same.

In one example, reference herein to a protein or nucleic acid shall be taken to be a reference to a human carcinoembryonic antigen-related cell adhesion molecule 1 (CEACAM1) or nucleic acid encoding same.

In one example reference herein to a protein or nucleic acid shall be taken to be a reference to a neural proliferation, differentiation and control 1 protein (NPDC1) or nucleic acid encoding same.

In one example, reference herein to a protein or nucleic acid shall be taken to be a reference to an EPH receptor B1 (EPHB1) or nucleic acid encoding same.

In one example, reference herein to a protein or nucleic acid shall be taken to be a reference to a human toll-like receptor 6 (TLR6) or nucleic acid encoding same.

In one example, reference herein to a protein or nucleic acid shall be taken to be a reference to a human plexin B2 (PLXNB2) or nucleic acid encoding same.

In one example, reference herein to a protein or nucleic acid shall be taken to be a reference to a human low density lipoprotein receptor-related protein 6 (LRP6) or nucleic acid encoding same.

In one example, reference herein to a protein or nucleic acid shall be taken to be a reference to a human CD8Bor nucleic acid encoding same.

In one example, reference herein to a protein or nucleic acid shall be taken to be a reference to a human V-set and immunoglobulin domain containing 1 protein (VSIG1) or nucleic acid encoding same.

In one example, reference herein to a protein or nucleic acid shall be taken to be a reference to a human p53 apoptosis effector related to PMP22 (PERP) or nucleic acid encoding same.

In one example, reference herein to a protein or nucleic acid shall be taken to be a reference to a human CD79Bor nucleic acid encoding same.

In one example, reference herein to a protein or nucleic acid shall be taken to be a reference to a human polycystic kidney disease 1-like 3 (PKD1L3) or nucleic acid encoding same.

In one example, reference herein to a protein or nucleic acid shall be taken to be a reference to a human T cell immunoreceptor with Ig and ITIM domains (TIGIT) or nucleic acid encoding same.

In one example, reference herein to a protein or nucleic acid shall be taken to be a reference to a human ST8 alpha-N-acetyl-neuraminide alpha-2,8-sialyltransferase 6 (ST8SIA6) or nucleic acid encoding same.

In one example, reference herein to a protein or nucleic acid shall be taken to be a reference to a human chromosome 6 open reading frame 105 (C6orf105) protein or nucleic acid encoding same.

In one example, reference herein to a protein or nucleic acid shall be taken to be a reference to a human tetraspanin 15 (TSPAN15) or nucleic acid encoding same.

In one example, reference herein to a protein or nucleic acid shall be taken to be a reference to a human NIPA-like domain containing 2 (NPAL2) or nucleic acid encoding same.

In one example, reference herein to a protein or nucleic acid shall be taken to be a reference to a human FRAS1 related extracellular matrix 3 (FREM3) or nucleic acid encoding same.

In one example, reference herein to a protein or nucleic acid shall be taken to be a reference to a human WDFY family member 4 (WDFY4) or nucleic acid encoding same.

In one example, reference herein to a protein or nucleic acid shall be taken to be a reference to a human Fc fragment of IgE, high affinity I, receptor for; gamma polypeptide (FCER1G) or nucleic acid encoding same.

In one example, reference herein to a protein or nucleic acid shall be taken to be a reference to a human transmembrane protein 169 (TMEM169) or nucleic acid encoding same.

Preferably, any of the foregoing proteins is expressed at a higher level than in a Thelper cell.

In one example, reference herein to a protein or nucleic acid shall be taken to be a reference to a human mucolipin 2 (MCOLN2) or nucleic acid encoding same.

In one example, reference herein to a protein or nucleic acid shall be taken to be a reference to a human C-type lectin-like 1 (CLECL1) or nucleic acid encoding same.

In one example, reference herein to a protein or nucleic acid shall be taken to be a reference to a human lysophosphatidylcholine acyltransferase 2 (LPCAT2) or nucleic acid encoding same.

In one example, reference herein to a protein or nucleic acid shall be taken to be a reference to a human leucine rich repeat neuronal 3 (LRRN3) or nucleic acid encoding same.

In one example, reference herein to a protein or nucleic acid shall be taken to be a reference to a human syntaxin 3 (STX3) or nucleic acid encoding same.

In one example, reference herein to a protein or nucleic acid shall be taken to be a reference to a human HEG homolog 1 (HEG1) or nucleic acid encoding same.

In one example, reference herein to a protein or nucleic acid shall be taken to be a reference to a human transmembrane protein 200A, KIAA1913 (TMEM200A) or nucleic acid encoding same.

In one example, reference herein to a protein or nucleic acid shall be taken to be a reference to a human G protein-coupled receptor 174 (GPR174) or nucleic acid encoding same.

In one example, reference herein to a protein or nucleic acid shall be taken to be a reference to a human transmembrane protein 71 (TMEM71) or nucleic acid encoding same.

In one example, reference herein to a protein or nucleic acid shall be taken to be a reference to a human ATPase, class V, type 10D (ATP10D) or nucleic acid encoding same.

In one example, reference herein to a protein or nucleic acid shall be taken to be a reference to a human immediate early response 3 (IER3) or nucleic acid encoding same.

In one example, reference herein to a protein or nucleic acid shall be taken to be a reference to a human lymphoid-restricted membrane protein (LRMP) or nucleic acid encoding same.

In one example, reference herein to a protein or nucleic acid shall be taken to be a reference to a human prostaglandin E receptor 4, subtype EP4 (PTGER4) or nucleic acid encoding same.

In one example, reference herein to a protein or nucleic acid shall be taken to be a reference to a human cysteinyl leukotriene receptor 1 (CYSLTR1) or nucleic acid encoding same.

In one example reference herein to a protein or nucleic acid shall be taken to be a reference to a human ATPase, class I, type 8B, member 4 (ATP8B4) or nucleic acid encoding same.

In one example, reference herein to a protein or nucleic acid shall be taken to be a reference to a human integrin alpha 2 (ITGA2) or nucleic acid encoding same.

In one example, reference herein to a protein or nucleic acid shall be taken to be a reference to a human CD300A or nucleic acid encoding same.

In one example, reference herein to a protein or nucleic acid shall be taken to be a reference to a human integrin alpha 1 (ITGA1) or nucleic acid encoding same.

In one example, reference herein to a protein or nucleic acid shall be taken to be a reference to a human CD274 or nucleic acid encoding same.

In one example, reference herein to a protein or nucleic acid shall be taken to be a reference to a human CD9 or nucleic acid encoding same.

In one example, reference herein to a protein or nucleic acid shall be taken to be a reference to a human semaphorin 3A (SEMA3A) or nucleic acid encoding same.

In one example, reference herein to a protein or nucleic acid shall be taken to be a reference to a human neuropilin 2 (NRP2) or nucleic acid encoding same.

In one example, example reference herein to a protein or nucleic acid shall be taken to be a reference to a human G protein-coupled receptor 183 (GPR183) or nucleic acid encoding same.

In one example, reference herein to a protein or nucleic acid shall be taken to be a reference to a human cytochrome b reductase 1 (CYBRD1) or nucleic acid encoding same.

In one example, reference herein to a protein or nucleic acid shall be taken to be a reference to a human activin A receptor, type IIA (ACVR2A) or nucleic acid encoding same.

In one example, reference herein to a protein or nucleic acid shall be taken to be a reference to a human sortilin 1 (SORT1) or nucleic acid.

In one example, reference herein to a protein or nucleic acid shall be taken to be a reference to a human CD200 receptor 1 (CD200R1) or nucleic acid encoding same.

In one example, reference herein to a protein or nucleic acid shall be taken to be a reference to a human mucolipin 3 (MCOLN3) or nucleic acid encoding same.

In one example, reference herein to a protein or nucleic acid shall be taken to be a reference to a tetraspanin 2 (TSPAN2) or nucleic acid encoding same.

In one example, reference herein to a protein or nucleic acid shall be taken to be a reference to a human epithelial mitogen homolog (EPGN) or nucleic acid encoding same.

In one example, reference herein to a protein or nucleic acid shall be taken to be a reference to a human phosphatidic acid phosphatase type 2A (PPAP2A) or nucleic acid encoding same.

In one example, reference herein to a protein or nucleic acid shall be taken to be a reference to a human adhesion molecule, interacts with CXADR antigen 1 (AMICA1) or nucleic acid encoding same.

In one example, reference herein to a protein or nucleic acid shall be taken to be a reference to a human prostaglandin E receptor 3 (PTGER3) or nucleic acid encoding same.

In one example, reference herein to a protein or nucleic acid shall be taken to be a reference to a human protein tyrosine phosphatase, receptor type, K (PTPRK) or nucleic acid encoding same.

In one example, reference herein to a protein or nucleic acid shall be taken to be a reference to a human CD82 or nucleic acid encoding same.

In one example, reference herein to a protein or nucleic acid shall be taken to be a reference to a human phospholipid scramblase 1 (PLSCR1) or nucleic acid.

In one example, reference herein to a protein or nucleic acid shall be taken to be a reference to a human LAG1 homolog, ceramide synthase 6 (LASS6) or nucleic acid encoding same.

In one example, reference herein to a protein or nucleic acid shall be taken to be a reference to a human G protein-coupled receptor 15 (GPR15) or nucleic acid encoding same.

In one example, reference herein to a protein or nucleic acid shall be taken to be a reference to a human GLI pathogenesis-related 1 protein (GLIPR1) or nucleic acid encoding same.

In one example, reference herein to a protein or nucleic acid shall be taken to be a reference to a human cytotoxic and regulatory T cell molecule (CRTAM) or nucleic acid encoding same.

In one example, reference herein to a protein or nucleic acid shall be taken to be a reference to a human C-type lectin domain family 2, member B (CLEC2B) or nucleic acid encoding same.

In one example, reference herein to a protein or nucleic acid shall be taken to be a reference to a human ectonucleotide pyrophosphatase/phosphodiesterase 2 (ENPP2) or nucleic acid encoding same.

In one example, reference herein to a protein or nucleic acid shall be taken to be a reference to a human SLAM family member 6 (SLAMF6) or nucleic acid encoding same.

In one example, reference herein to a protein or nucleic acid shall be taken to be a reference to a human core 1 synthase, glycoprotein-N-acetylgalactosamine 3-beta-galactosyltransferase, 1 (C1GALT1) or nucleic acid encoding same.

In one example, reference herein to a protein or nucleic acid shall be taken to be a reference to a human adhesion molecule with Ig-like domain 2 (AMIG02) or nucleic acid encoding same.

In one example, reference herein to a protein or nucleic acid shall be taken to be a reference to a human cornichon homolog 4 (CNIH4) or nucleic acid encoding same.

In one example, reference herein to a protein or nucleic acid shall be taken to be a reference to a human poliovirus receptor-related 3 (PVRL3) or nucleic acid encoding same.

In one example, reference herein to a protein or nucleic acid shall be taken to be a reference to a RGM domain family member B or nucleic acid encoding same.

In one example, reference herein to a protein or nucleic acid shall be taken to be a reference to a ATPase, H+ transporting, lysosomal V0 subunit al (ATP6V0A1) or nucleic acid encoding same.

In one example, reference herein to a protein or nucleic acid shall be taken to be a reference to an interleukin 6 receptor (IL6R) or nucleic acid encoding same.

In one example, reference herein to a protein or nucleic acid shall be taken to be a reference to a natural killer cell group 7 sequence (NKG7) or nucleic acid encoding same.

In one example, reference herein to a protein or nucleic acid shall be taken to be a reference to a human G protein-coupled receptor 65 (GPR65) or nucleic acid encoding same.

In one example, reference herein to a protein or nucleic acid shall be taken to be a reference to a human sushi domain containing 4 (SUSD4) or nucleic acid encoding same.

In one example, reference herein to a protein or nucleic acid shall be taken to be a reference to a human Ral GEF with PH domain and SH3 binding motif 2 (RALGPS2) or nucleic acid encoding same.

In one example, reference herein to a protein or nucleic acid shall be taken to be a reference to a human interleukin 18 receptor accessory protein (IL18RAP) or nucleic acid encoding same.

In one example, a marker set forth in any one of Tables 2, 4 or 5 are expressed on a Treg cell (i.e., are positive for expression) or are expressed at a high or "hi" level on a Treg cell.

As used herein, the term "positive expression" or "+" shall be taken to mean expression above the level of background, e.g., as detected using an isotype control compound, e.g., antibody.

As used herein, the term "isotype control compound" shall be taken to mean a compound, preferably an antibody of the same isotype as that used to detect expression of a protein, however having no relevant specificity to a protein and conjugated to the same detectable moiety as the compound used to detect expression of the protein. Such a control aids in distinguishing non-specific "background" binding from specific binding.

Reference to a "high" or "hi" level of expression means the 50% of cells, preferably 40%, 30% or more preferably 20%, more preferably 10% of cells in a population of cells expressing the highest level of the recited marker, e.g., as determined using FACS analysis.

In one example, a marker set forth in any one of Tables 3 or 6 are expressed on a Treg cell at a low or "lo" level.

As used herein, the term "negative expression" or "-"shall be taken to mean expression equal to or less than the level of background expression, e.g., as detected using an isotype control compound, e.g., antibody.

Reference to a "low" or "lo" level of expression shall be taken to mean the 50% or 60% or 70% or 80% or 90% of cells in a population of cells with lowest level of expression of the recited nucleic acid or protein in a population of cells.

The present disclosure also encompasses the detection of any combination of nucleic acids or proteins set forth in any one or more of Tables 2-6. For example, any embodiment described herein shall be taken to apply *mutatis mutandis* to detection of any two or more nucleic acids and/or proteins individually or collectively set forth in any one or more of Tables 2-6. Similarly, the present invention shall be taken to encompass detection of any combination of protein and nucleic acid markers individually or collectively set forth in any one or more of Tables 2-6 according to the claims.

By "individually" is meant that the disclosure encompasses the recited nucleic acids or proteins or groups nucleic acids and/or proteins separately, and that, notwithstanding that individual nucleic acid(s) and/or protein(s) or groups of nucleic acids and/or proteins may not be separately listed herein the accompanying claims may define such nucleic acid(s) and/or protein(s) or groups of nucleic acids and/or proteins separately and divisibly from each other.

By "collectively" is meant that the disclosure encompasses any number or combination of the recited nucleic acids and/or proteins or groups of nucleic acids and/or proteins, and that, notwithstanding that such numbers or combinations of nucleic acid(s) and/or proteins(s) or groups of nucleic acids and/or proteins may not be specifically listed herein the accompanying claims may define such combinations or sub-combinations separately and divisibly from any other combination of nucleic acid(s) and/or protein(s) or groups of nucleic acids and/or proteins.

The present disclosure also contemplates detection of any individual or collection of proteins or nucleic acids described herein according to any embodiment together with any other marker, e.g., of a Treg cell. Exemplary additional proteins or nucleic acids include, CD4, CD25, FoxP3, cytotoxic T-lymphocyte-associated antigen 4 (CTLA-4), CD62 ligand (CD62L), CD134 (OX40), glucocorticoid-induced tumor necrosis factor receptor (GITR), membrane-bound TGF-β, CD96, programmed cell death ligand 1 (PD-L1), α₄β₇ integrin, α₄β₁ integrin, CD45R0, CD27, CD95, CCR6. Preferred additional proteins or nucleic acids are CD4 and/or CD25 and/or FoxP3. Preferably, CD25 is expressed at a high level.

In another example, a method for detecting or isolating Treg cells additionally comprises detecting a low or undetectable level of expression of a nucleic acid or protein expressed by a non-Treg cell. Exemplary nucleic acids and/or proteins include CD19 and/or CD20 and/or CD14 and/or CD56.

### Detection/Isolation/Diagnostic/Therapeutic Compounds

Also described is a variety of reagents useful in detecting/isolating Treg cells and/or diagnosing/prognosing/treating/preventing Treg-mediated conditions. Compounds include antibodies, proteins comprising antibody variable regions, peptides, nucleic acid-based reagents, and small molecules. Any compound for treating a subject can be tested *in vitro* and/or *in vivo* using models of Treg activity and/or Treg-associated disease, e.g., as described herein.

### Protein Compounds

### Antibodies and Proteins Comprising Variable Regions

Preferably, a method as described herein according to the claims detects a protein and/or isolates a population enriched for Treg cells using an antibody or a protein comprising a variable region or Fv of an antibody and/or involves administering an antibody or protein comprising a variable region or Fv thereof.

The skilled artisan will be aware that an "antibody" includes an immune protein capable of specifically binding to one or a few closely related antigens (e.g., PI16) by virtue of an antigen binding site contained within at least one variable region. This term includes four chain antibodies (e.g., two light chains and two heavy chains), recombinant or modified antibodies (e.g., chimeric antibodies, humanized antibodies, primatized antibodies, de-immunized antibodies, half antibodies, bispecific antibodies) and single domain antibodies such as domain antibodies and heavy chain only antibodies (e.g., camelid antibodies or cartilaginous fish immunoglobulin new antigen receptors (IgNARs)). An antibody generally comprises constant domains, which can be arranged into a constant region or constant fragment or fragment crystallisable (Fc). Preferred forms of antibodies comprise a four-chain structure as their basic unit. Full-length antibodies comprise two heavy chains (~50-70 kD) covalently linked and two light chains (~23 kD each). A light chain generally comprises a variable region and a constant domain and in mammals is either a κ light chain or a λ light chain. A heavy chain generally comprises a variable region and one or two constant domain(s) linked by a hinge region to additional constant domain(s). Heavy chains of mammals are of one of the following types α, δ, ε, γ, or µ. Each light chain is also covalently linked to one of the heavy chains. For example, the two heavy chains and the heavy and light chains are held together by inter-chain disulfide bonds and by non-covalent interactions. The number of inter-chain disulfide bonds can vary among different types of antibodies. Each chain has an N-terminal variable region (V_{H} or V_{L} wherein each are ~110 amino acids in length) and one or more constant domains at the C- terminus. The constant domain of the light chain (C_{L} which is ~110 amino acids in length) is aligned with and disulfide bonded to the first constant domain of the heavy chain (C_{H} which is-330-440 amino acids in length). The light chain variable region is aligned with the variable region of the heavy chain. The antibody heavy chain can comprise 2 or more additional C_{H} domains (such as, C_{H}2, C_{H}3 and the like) and can comprise a hinge region can be identified between the C_{H}1 and Cm constant domains. Antibodies can be of any type (e.g., IgG, IgE, IgM, IgD, IgA, and IgY), class (e.g., IgG₁, IgG₂, IgG₃, IgG₄, IgA₁ and IgA₂) or subclass. Preferably, the antibody is a murine (mouse or rat) antibody or a primate (preferably human) antibody. Preferably, the antibody is a monoclonal antibody. The term "antibody" also encompasses antibody derivatives, e.g., antibody conjugates.

As used herein, the term "protein comprising an antigen binding domain" shall be taken to mean any fragment or domain or protein comprising same of an antibody that retains the ability to bind to the target protein preferably specifically or selectively. This term also includes a polypeptide comprising a plurality of antigen binding domains of an antibody and/or a plurality of antigen binding domains wherein a domain is from one antibody and another domain is from another antibody. The domains may be in a single polypeptide chain or in multiple polypeptide chains (e.g., in the case of a diabody or higher order multimer). This term includes a Fab fragment, a Fab' fragment, a F(ab') fragment, a single chain antibody (SCA or SCAB), a diabody or higher order multimer amongst others. An "Fab fragment" consists of a monovalent antigen-binding fragment of an antibody molecule, and can be produced by digestion of a whole antibody molecule with the enzyme papain, to yield a fragment consisting of an intact light chain and a portion of a heavy chain. Such fragments can also be produced using recombinant means. An "Fab' fragment" of an antibody molecule can be obtained by treating a whole antibody molecule with pepsin, followed by reduction, to yield a molecule consisting of an intact light chain and a portion of a heavy chain. Two Fab' fragments are obtained per antibody molecule treated in this manner. Such fragments can also be produced using recombinant means. An "F(ab')2 fragment" of an antibody consists of a dimer of two Fab' fragments held together by two disulfide bonds, and is obtained by treating a whole antibody molecule with the enzyme pepsin, without subsequent reduction. Such fragments can also be produced using recombinant means. An "scFv fragment" is a genetically engineered fragment containing the variable region of a light chain and the variable region of a heavy chain expressed as two chains. A "single chain antibody" (SCA) is a genetically engineered single chain molecule containing the variable region of a light chain and the variable region of a heavy chain, linked by a suitable, flexible polypeptide linker. This term also encompasses domain antibodies (dAbs) comprising a single variable domain, a heavy chain only antibody (e.g., from camelid or cartilaginous fish) or a minibody or a flex minibody or a diabody or a triabody or a tetrabody or a higher order multimer or any protein discussed above fused to a constant region of an antibody or a Fc region of an antibody or a C_{H}2 and/or C_{H}3 region of an antibody.

The term "protein" shall be taken to include a single polypeptide chain, i.e., a series of contiguous amino acids linked by peptide bonds or a series of polypeptide chains covalently or non-covalently linked to one another (i.e., a polypeptide complex). For example, the series of polypeptide chains can be covalently linked using a suitable chemical or a disulphide bond. Examples of non-covalent bonds include hydrogen bonds, ionic bonds, Van der Waals forces, and hydrophobic interactions. A non-covalent bond contemplated by the present invention is the interaction between a V_{H} and a V_{L}, e.g., in some forms of diabody or a triabody or a tetrabody or a Fv.

The term "polypeptide chain" will be understood to mean from the foregoing paragraph to mean a series of contiguous amino acids linked by peptide bonds.

For some embodiments described herein antibodies can be obtained from commercial sources, as will be apparent to the skilled artisan. For example, antibodies against IL1R1 are available from R&D Systems, Inc. or AbCam Ltd; antibodies against CD79A are available from Becton Dickinson Inc; antibodies against PTPRB are available from Becton Dickinson Inc or Santa Cruz Biotechnology, Inc; antibodies against IFNGR2 are available from R&D Systems, Inc. or AbCam Ltd; antibodies against MCAM are available from R&D Systems, Inc. or AbCam Ltd; antibodies against LSR are available from LSR; antibodies against CD33 are available from Becton Dickinson Inc; antibodies against PI16 are available from R&D Systems, Inc or Abnova; antibodies against c18orf1 are available from Abnova or Santa Cruz Biotechnology, Inc.; antibodies against ITGAM are available from Becton Dickinson Inc; antibodies against CECAM are available from R&D Systems, Inc. or AbCam Ltd; antibodies against AQP3 are available from Santa Cruz Biotechnology, Inc.; antibodies against NPDC1 are available from AbCam Ltd or Santa Cruz Biotechnology, Inc.; antibodies against EPHB1 are available from AbCam Ltd or Santa Cruz Biotechnology, Inc.; antibodies against ITGA6 are available from AbCam Ltd or Santa Cruz Biotechnology, Inc.; antibodies against PLXNB2 are available from AbCam Ltd or Santa Cruz Biotechnology, Inc.; antibodies against CD79B are available from Becton Dickinson Inc; antibodies against CD8B are available from Becton Dickinson Inc or Santa Cruz Biotechnology, Inc; antibodies against TLR6 are available from AbCam Ltd or Santa Cruz Biotechnology, Inc.; antibodies against VSIG1 are available from R&D Systems, Inc. or Abnova; antibodies against LRP6 are available from &D Systems, Inc. or AbCam Ltd; antibodies against PERP are available from AbCam Ltd or Santa Cruz Biotechnology, Inc.; antibodies against PKD1L3 are available from AbCam Ltd; antibodies against FCER1G are available from Santa Cruz Biotechnology Inc; and antibodies against TSPAN15 are available from Santa Cruz Biotechnology Inc.

To generate antibodies, a protein or immunogenic fragment or epitope thereof or a cell expressing and displaying same, optionally formulated with any suitable or desired carrier, adjuvant, BRM, or pharmaceutically acceptable excipient, is conveniently administered in the form of an injectable composition. Injection may be intranasal, intramuscular, sub-cutaneous, intravenous, intradermal, intraperitoneal, or by other known route. For intravenous injection, it is desirable to include one or more fluid and nutrient replenishers. Means for preparing and characterizing antibodies are known in the art. (See, e.g., ANTIBODIES: A LABORATORY MANUAL, Cold Spring Harbor Laboratory, 1988).

Immunogenic peptides for generating polyclonal or monoclonal antibodies can be covalently coupled to an immunogenic carrier protein, such as Diphtheria toxoid (DT), Keyhole Limpet Hemocyanin (KLH), tetanus toxoid (TT) or the nuclear protein of influenza virus (NP), using one of several conjugation chemistries known in the art. This enhances the immunogenicity of peptides that are otherwise not highly immunogenic in animals e.g., mice, rats, chickens etc. Methods of preparing and/or carrier proteins are known in the art and described, for example, in U.S. 4,709,017, 5,843,711, 5,601,827, 5,917,017 and Lee, *et al.,* 1980).

The conjugate molecules so produced may be purified and employed in immunogenic compositions to elicit, upon administration to a host, an immune response to the protein and/or peptide which is potentiated in comparison to the protein or peptide alone.

The efficacy of the protein or immunogenic fragment or epitope thereof or cell expressing same in producing an antibody is established by injecting an animal, for example, a mouse, chicken, rat, rabbit, guinea pig, dog, horse, cow, goat or pig, with a formulation comprising the protein or immunogenic fragment or epitope thereof, and then monitoring the immune response to the protein, epitope or fragment. Both primary and secondary immune responses are monitored. The antibody titer is determined using any conventional immunoassay, such as, for example, ELISA, or radio-immunoassay.

The production of polyclonal antibodies may be monitored by sampling blood of the immunized animal at various points following immunization. A second, booster injection, may be given, if required to achieve a desired antibody titer. The process of boosting and titering is repeated until a suitable titer is achieved. When a desired level of immunogenicity is obtained, the immunized animal is bled and the serum isolated and stored, and/or the animal is used to generate monoclonal antibodies (Mabs).

Monoclonal antibodies are particularly preferred. The term "monoclonal antibody" refers to a homogeneous antibody population capable of binding to the same antigen(s) and, preferably, to the same epitopic determinant within the antigen(s). This term is not intended to be limited as regards to the source of the antibody or the manner in which it is made.

For the production of monoclonal antibodies (Mabs) any one of a number of known techniques may be used, such as, for example, the procedure exemplified in US4,196,265 or ANTIBODIES: A LABORATORY MANUAL, Cold Spring Harbor Laboratory, 1988, or Zola, 1997.

For example, a suitable animal is immunized with an effective amount of the protein or immunogenic fragment or epitope thereof or cell expressing same under conditions sufficient to stimulate antibody producing cells. Rodents such as rabbits, mice and rats are preferred animals, however, the use of sheep or frog cells is also possible. The use of rats may provide certain advantages, but mice or rabbits are preferred, with the BALB/c or C57/bl6 mouse being preferred as the most routinely used animal and one that generally gives a higher percentage of stable fusions. Alternatively, a mouse genetically-engineered to express human immunoglobulin proteins, and preferably not express murine immunoglobulin proteins, is immunized to produce an antibody. Such mice are known in the art and commercially available. For example, Regeneron, Inc. have produced the VelocImmune™ mouse in which human variable regions have been homologously recombined or knocked-in to the mouse genome to replace endogenous mouse variable region encoding genes. Such mice are described, for example, in WO2002/066630. Abgenix/Amgen, Inc. and Kirin Brewery/Medarex, Inc. have produced strains of mice in which the endogenous mouse immunoglobulin loci are inactivated or "knocked-out" and human immunoglobulin loci introduced using yeast artificial chromosomes. Examples of these mice are described or reviewed in Lonberg *et al.,* (1994); Lonberg, (1994); Tomizuka *et al.,* (2000) and Jakobovits *et al.,* (2007).

Following immunization, somatic cells with the potential for producing antibodies, specifically B lymphocytes (B cells), are selected for use in the MAb generating protocol. These cells may be obtained from biopsies of spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Spleen lymphocytes are obtained by homogenizing the spleen with a syringe. The B cells from the immunized animal are then fused with cells of an immortal myeloma cell, generally derived from the same species as the animal that was immunized with the immunogen. Any one of a number of myeloma cells may be used and these are known to those of skill in the art (e.g. murine P3-X63/Ag8, X63-Ag8.653, NS1/1 .Ag 4 1, Sp2/0-Agl4, FO, NSO/U, MPC-I 1, MPC11-X45-GTG 1.7 and S194/5XX0).

To generate hybrids of antibody-producing spleen or lymph node cells and myeloma cells, somatic cells are mixed with myeloma cells in the presence of an agent or agents (chemical or electrical) that promote the fusion of cell membranes. Fusion methods using Sendai virus have been described by Kohler and Milstein, (1975); and Kohler and Milstein, (1976). Methods using polyethylene glycol (PEG), such as 37% (v/v) PEG, are described in detail by Gefter *et al,* (1977). The use of electrically induced fusion methods is also appropriate.

Hybrids are amplified by culture in a selective medium comprising an agent that blocks the *de novo* synthesis of nucleotides in the tissue culture media. Exemplary and preferred agents are aminopterin, methotrexate and azaserine.

The amplified hybridomas are subjected to a functional selection for antibody specificity and/or titer, such as, for example, by immunoassay (e.g. radioimmunoassay, enzyme immunoassay, cytotoxicity assay, plaque assay, dot immunoassay, and the like).

The selected hybridomas are serially diluted and cloned into individual antibody-producing cell lines, which clones can then be propagated for an extended period, e.g., indefinitely to provide MAbs. The individual cell lines can be cultured *in vitro,* where the MAbs are naturally secreted into the culture medium from which they are readily obtained in high concentrations. MAbs produced by either means may be further purified, if desired, using filtration, centrifugation and various chromatographic methods such as HPLC or affinity chromatography.

Alternatively, ABL-MYC technology (NeoClone, Madison WI 53713, USA) is used to produce cell lines secreting monoclonal antibodies (mAbs) against a protein as described herein according to any embodiment or an epitope or immunogenic fragment thereof. This technology comprises infecting splenocytes from immunized mice with replication-incompetent retrovirus comprising the oncogenes v-abl and c-myc. Splenocytes are transplanted into naive mice which then develop ascites fluid containing cell lines producing monoclonal antibodies (mAbs) against a protein as described herein according to any embodiment or an epitope or immunogenic fragment thereof. The mAbs are purified from ascites using protein G or protein A, e.g., bound to a solid matrix, depending on the isotype of the mAb. The ABL-MYC technology is described generically in detail in Largaespada (1990); Weissinger *et al.,* (1991); Largaespada *et al,* (1992); Weissinger *et al,* (1994); Largaespada *et al,* (1996); and Kumar *et al,* (1999).

Antibodies can also be produced or isolated by screening a display library, e.g., a phage display library where, for example the phage express scFv fragments on the surface of their coat with a large variety of CDRs. For example, McCafferty *et al,* (1990), Clackson *et al,* (1991) and Marks *et al,* (1991) describe the isolation of murine and/or human antibodies, using phage libraries. Subsequent publications describe the production of high affinity (nM range) human antibodies by chain shuffling (Marks *et al,* 1992), as well as combinatorial infection and in vivo recombination as a strategy for constructing very large phage libraries (Waterhouse *et al,* 1993).

### Recombinant Antibody Production

The antibodies or fragments can also be produced recombinantly, using techniques and materials readily obtainable.

For example, DNA encoding an antibody or a protein comprising a variable region thereof, e.g., a Fab fragment is readily isolated and sequenced using conventional procedures (e.g., by using oligonucleotide probes that are capable of binding specifically to genes encoding the heavy and light chains of murine antibodies). A hybridoma cell serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra *et al,* (1993) and Pluckthun, (1992). Molecular cloning techniques to achieve these ends are known in the art and described, for example in Ausubel or Sambrook. A wide variety of cloning and in vitro amplification methods are suitable for the construction of recombinant nucleic acids. Examples of these techniques and instructions sufficient to direct persons of skill through many cloning exercises are found in Berger and Kimmel, Guide to Molecular Cloning Techniques, Methods in Enzymology volume 152 Academic Press, Inc., San Diego, Calif. (Berger); Sambrook et al., (1989) Molecular Cloning A Laboratory Manual (2nd ed.) Vol. 1 3, Cold Spring Harbor Laboratory, Cold Spring Harbor Press, N.Y., (Sambrook); and Current Protocols in Molecular Biology, F. M. Ausubel et al., eds., Current Protocols, a joint venture between Greene Publishing Associates, Inc. and John Wiley & Sons, Inc., (1994 Supplement) (Ausubel). Methods of producing recombinant immunoglobulins are also known in the art. See US4,816,567; and Queen *et al.,* (1989).

For recombinant production of an antibody or fragment, the nucleic acid encoding it is preferably isolated and inserted into a replicable vector for further cloning (amplification of the DNA) or for expression. DNA encoding the antibody is readily isolated or synthesized using conventional procedures (e.g., by using oligonucleotide probes that are capable of binding specifically to DNAs encoding the heavy and light chains of the antibody). Many vectors are available. Exemplary vectors are described herein. The vector components generally include, but are not limited to, one or more of the following: a signal sequence, a sequence encoding an antibody or fragment thereof (e.g., derived from the information provided herein), an enhancer element, a promoter, and a transcription termination sequence. The skilled artisan will be aware of suitable sequences for expression of an antibody. For example, exemplary signal sequences include prokaryotic secretion signals (e.g., alkaline phosphatase, penicillinase, Ipp, or heat-stable enterotoxin II), yeast secretion signals (e.g., invertase leader, α factor leader, or acid phosphatase leader) or mammalian secretion signals (e.g., herpes simplex gD signal). Exemplary promoters include those active in prokryotes (e.g., phoA promoter , β-lactamase and lactose promoter systems, alkaline phosphatase, a tryptophan (trp) promoter system, and hybrid promoters such as the tac promoter), and those active in mammalian cells (e.g., cytomegalovirus immediate early promoter (CMV), the human elongation factor 1-α promoter (EF1), the small nuclear RNA promoters (U1a and Ulb), α-myosin heavy chain promoter, Simian virus 40 promoter (SV40), Rous sarcoma virus promoter (RSV), Adenovirus major late promoter, β-actin promoter; hybrid regulatory element comprising a CMV enhancer/ β-actin promoter or an immunoglobulin promoter or active fragment thereof.).

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as Bacilli such as *B. subtilis* and *B. licheniformis, Pseudomonas* such as *P. aeruginosa,* and *Streptomyces.* One preferred *E. coli* cloning host is *E. coli* 294 (ATCC 31,446), although other strains such as *E. coli* B, *E. coli* X 1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Pichia pastoris* (EP 183,070); and filamentous fungi such as, e.g., *Neurospora, Penicillium, Tolypocladium,* and *Aspergillus* hosts such as *A. nidulans and A. niger.*

Suitable host cells for the expression of glycosylated antibody are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified.

Examples of useful mammalian host cell lines are monkey kidney CV1 line transformed by SV40 (COS-7, ATCC CRL 1651); human embryonic kidney line (293 or 293 cells subcloned for growth in suspension culture, Graham *et al.,* (1977) ; baby hamster kidney cells (BHK, ATCC CCL 10); Chinese hamster ovary cells (CHO, Urlaub *et al.,* (1980); mouse Sertoli cells (TM4, Mather (1980); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al.,* (1982); MRC 5 cells; FS4 cells; and PER.C6™ (Crucell NV)..

The host cells used to produce the antibody of this invention may be cultured in a variety of media. Commercially available media such as Ham's F10 (Sigma), Minimal Essential Medium ((MEM), (Sigma), RPM1-1640 (Sigma), and Dulbecco's Modified Eagle's Medium ((DMEM), Sigma) are suitable for culturing the host cells. In addition, any of the media described in Ham *et al.,* (1979), Barnes *et al.,* (1980), US4,767,704; US4,657,866; US4,927,762; US4,560,655; US5,122,469; WO 90/03430; WO 87/00195; may be used as culture media for the host cells.

### Chimeric antibodies

In one example an antibody is a chimeric antibody. The term "'chimeric antibody" refers to antibodies in which a portion of the heavy and/or light chain is identical with or homologous to corresponding sequences in antibodies derived from a particular species (e.g., murine, such as mouse) or belonging to a particular antibody class or subclass, while the remainder of the chain(s) is identical with or homologous to corresponding sequences in antibodies derived from another species (e.g., primate, such as human) or belonging to another antibody class or subclass, as well as fragments of such antibodies, so long as they exhibit the desired biological activity (US4,816,567; and Morrison *et al.,* 1984).

Typically chimeric antibodies utilize rodent or rabbit variable regions and human constant regions, in order to produce an antibody with predominantly human domains. For example, a chimeric antibody comprises a variable region from a mouse antibody as described herein fused to a human constant region. The production of such chimeric antibodies is known in the art, and may be achieved by standard means (as described, e.g., in Morrison, (1985); Oi *et al,* (1986); Gillies *et al,* (1989); US5,807,715; US4,816,567 and US4,816397).

The term "constant region" (CR) as used herein, refers to the portion of the antibody molecule which confers effector functions. The heavy chain constant region can be selected from any of the five isotypes: alpha, delta, epsilon, gamma or mu. Further, heavy chains of various subclasses (such as the IgG subclasses of heavy chains) are responsible for different effector functions and thus, by choosing the desired heavy chain constant region, antibodies with desired effector function can be produced. Preferred heavy chain constant regions are gamma 1 (IgG1), gamma 2 (IgG2), gamma 3 (IgG3) and gamma 4 (IgG4). Light chain constant regions can be of the kappa or lambda type, preferably of the kappa type.

As used herein, the term "complementarity determining regions" (*syn.* CDRs; i.e., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat *et al.,* (1991) and/or those residues from a "hypervariable loop" Chothia and Lesk (1987).

"Framework regions" (hereinafter FR) are those variable domain residues other than the CDR residues.

### Humanized and Human antibodies

The antibodies may be humanized antibodies or human antibodies.

The term "humanized antibody" shall be understood to refer to a chimeric molecule, generally prepared using recombinant techniques, having an epitope binding site derived from an immunoglobulin from a non-human species and the remaining immunoglobulin structure of the molecule based upon the structure and/or sequence of a human immunoglobulin. The antigen-binding site preferably comprises the complementarity determining regions (CDRs) from the non-human antibody grafted onto appropriate framework regions in the variable domains of a human antibody and the remaining regions from a human antibody. Antigen binding sites may be wild type or modified by one or more amino acid substitutions. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin (Jones *et al.,* 1989; Riechmann *et al.,* 1988; and Presta 1992).

Methods for humanizing non-human antibodies are known in the art. Humanization can be essentially performed following the method of Jones *et al., supra;* Riechmann *et al., supra;* Verhoeyen *et al.,* (1988), by substituting rodent CDRs or CDR sequences for the corresponding sequences of a human antibody. Other methods for humanizing an antibody are not excluded.

The term "human antibody" as used herein in connection with antibody molecules and binding proteins refers to antibodies having variable (e.g. VH, VL, CDR and FR regions) and constant antibody regions derived from or corresponding to sequences found in humans, e.g. in the human germline or somatic cells. The "human" antibodies can include amino acid residues not encoded by human sequences, e.g. mutations introduced by random or site directed mutations *in vitro* (in particular mutations which involve conservative substitutions or mutations in a small number of residues of the antibody, e.g. in 1, 2, 3, 4 or 5 of the residues of the antibody, preferably e.g. in 1, 2, 3, 4 or 5 of the residues making up one or more of the CDRs of the antibody). These "human antibodies" do not actually need to be produced by a human, rather, they can be produced using recombinant means and/or isolated from a transgenic animal (e.g., mouse) comprising nucleic acid encoding human antibody constant and/or variable regions (e.g., as described above).

Human antibodies can also be produced using various techniques known in the art, including phage display libraries (e.g., as described in Hoogenboom and Winter 1991; Marks *et al.,* 1991; US5,885,793).

Completely human antibodies which recognize a selected epitope can also be generated using a technique referred to as "guided selection." In this approach a selected non-human monoclonal antibody, e.g., a mouse antibody, is used to guide the selection of a completely human antibody recognizing the same epitope (Jespers *et al,* 1988).

### Multi-specific antibodies

Bispecific antibodies are antibodies that have binding specificities for at least two different epitopes. Exemplary bispecific antibodies may bind to two different epitopes of the target protein. Other such antibodies may combine a binding site for a protein described herein with a binding site for another protein. Alternatively, a region that binds a protein described herein may be combined with a region which binds to a triggering molecule on a leukocyte such as a T-cell receptor molecule (e.g., CD3), or Fc receptors for IgG (FcγR), such as FcγRI (CD64), FcγRII (CD32) and/or FcγRIII (CD16), so as to focus and localize cellular defense mechanisms to a Treg cell. Bispecific antibodies may also be used to localize cytotoxic agents to Treg cells. These antibodies possess a target protein-binding region and a region which binds the cytotoxic agent (e.g., saporin, anti-interferon-α., vinca alkaloid, ricin A chain, methotrexate or radioactive isotope hapten). Bispecific antibodies can be prepared as full length antibodies or antibody fragments (e.g., F(ab')2 bispecific antibodies). Exemplary bispecific antibodies and their method for production are described in WO96/16673, WO98/02463 and US5,821,337.

Methods for making bispecific antibodies are known in the art. Traditional production of full length bispecific antibodies is based on the co-expression of two immunoglobulin heavy chain-light chain pairs, where the two chains have different specificities (Millstein *et al,* 1983). Because of the random assortment of immunoglobulin heavy and light chains, these hybridomas (quadromas) produce a potential mixture of 10 different antibody molecules, of which only one has the correct bispecific structure. Purification of the correct molecule is usually done by affinity chromatography steps. Similar procedures are disclosed in WO93/08829, and in Traunecker *et al.,* (1991). Other approaches for producing bispecific antibodies are known in the art and described for example, in WO94/04690; US5,731,168; Suresh *et al,* (1986).

Bispecific antibodies include cross-linked or "heteroconjugate" antibodies. For example, one of the antibodies in the heteroconjugate can be coupled to avidin, the other to biotin. Such antibodies are known in the art and described, for example, in US4,676,980; WO91/00360; WO92/200373; and EP03089.

Techniques for generating bispecific antibodies from antibody fragments have also been described in the literature. For example, bispecific antibodies can be prepared using chemical linkage (Brennan *et al,* 1985) or using Fab'-SH fragments from *E. coli,* which can be chemically coupled to form bispecific antibodies (Shalaby *et al,* 1992). Other techniques make use of leucine zippers (Kostelny *et al,* 1992) or the "diabody" technology described by Hollinger et al, (1993).

Antibodies with more than two valencies are also contemplated . For example, trispecific antibodies can be prepared (Tutt *et al,* (1991).

The antibodies can be multivalent antibodies (which are other than of the IgM class) with three or more antigen binding sites (e.g., tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody can comprise an Fc region and three or more antigen binding sites amino-terminal to the Fc region.

### Mutations to Antibodies

Amino acid sequence modification(s) of the antibodies are described. For example, it may be desirable to improve the binding affinity and/or other biological properties of the antibody. Amino acid sequence variants of the antibody are prepared by introducing appropriate nucleotide changes into the encoding nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody, with an N-terminal methionyl residue or the antibody fused to a cytotoxic polypeptide. Other insertional variants of the antibody include the fusion to the N- or C-terminus of the antibody to an enzyme or a polypeptide which increases the serum half-life of the antibody.

Another type of variant is an amino acid substitution variant. These variants have at least one amino acid residue in the antibody replaced by a different residue. The sites of interest for substitutional mutagenesis include the CDRs, however FR alterations are also contemplated. Preferred substitutions are conservative substitutions.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more CDR residues of a parent antibody (e.g., a humanized or human antibody). Generally, the resulting variant(s) selected for further development will have improved biological properties relative to the parent antibody from which they are generated. A convenient way for generating such substitutional variants involves affinity maturation using phage display e.g., as described in US5,223,409.

Another type of amino acid variant of the antibody alters the original glycosylation pattern of the antibody. By altering is meant deleting one or more carbohydrate moieties found in the antibody, and/or adding one or more glycosylation sites that are not present in the antibody. Modified glycoforms of antibodies may be useful for a variety of purposes, including but not limited to enhancing or reducing effector function and/or modifying half life of the antibody (see, for example, WO2007/010401). Such alterations may result in a decrease or increase of CIq binding and CDC or of FcyR binding and/or ADCC. Substitutions can, for example, be made in one or more of the amino acid residues of the heavy chain constant region thereby causing an alteration in an effector function while retaining the ability to bind to the antigen as compared with the modified antibody, e.g., as described in US5,624,821 and US5,648,260. Engineered glycoforms may be generated by any method known to one skilled in the art, for example by using engineered or variant expression strains, by co-expression with one or more enzymes, for example β(1,4)-N-acetylglucosaminyltransferase III (GnTIII), by expressing an antibody or fragment thereof in various organisms or cell lines from various organisms, or by modifying carbohydrate(s) after the antibody or fragment has been expressed. Methods for generating engineered glycoforms are known in the art, and include but are not limited to those described in Umana *et al,* 1999; Davies *et al.,* 2007; Shields *et al,* 2002,; Shinkawa *et al.,* 2003,) US6,602,684; USSN10/277,370; USSN10/113,929; WO00/61739; WO01/292246; WO02/311140; WO02/30954; WO00/061739; EP01229125; US20030115614; Okazaki *et al.,* 2004.

Alternatively, or in addition, the antibodies or fragments can be expressed in a transfectoma which does not add the fucose unit normally attached to Asn at position 297 of the Fc region in order to enhance the affinity of the Fc region for Fc-Receptors which, in turn, will result in an increased ADCC of the antibodies in the presence of NK cells, e.g., Shield *et al.,* 2002.

To increase the serum half life of the antibody, one may incorporate a salvage receptor binding epitope into the antibody (especially an antibody fragment) as described in US5,739,277. As used herein, the term "salvage receptor binding epitope" refers to an epitope of the Fc region of an IgG molecule (e.g., IgG1, IgG2, IgG3, or IgG4) that is responsible for increasing the *in vivo* serum half-life of the IgG molecule.

### Purification of Antibodies

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody prepared from the cells can be purified using, for example, hydroxyl apatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark *et al.,* 1983). Protein G is recommended for all mouse isotypes and for human γ3 (Guss *et al.,* 1986). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered. Exemplary purification methods are described, for example, in Zola (1987).

Following any preliminary purification step(s), the mixture comprising the antibody of interest and contaminants may be subjected to low pH hydrophobic interaction chromatography.

### Protein Conjugates

Also described are derivatives of an antibody or protein as described herein, e.g., a conjugate (immunoconjugate) comprising an antibody or protein conjugated to a distinct moiety, e.g., a therapeutic agent or a detectable agent which is directly or indirectly bound to the antibody. Examples of other moieties include, but are not limited to, a cytotoxin, a radioisotope (e.g., ²¹²Bi, ¹³¹I, ⁹⁰Y, or ¹⁸⁶Re), an immunomodulatory agent, an anti-angiogenic agent, an anti-neovascularization and/or other vascularization agent, a toxin, an anti-proliferative agent, a pro-apoptotic agent, a chemotherapeutic agent and a therapeutic nucleic acid.

A cytotoxin includes any agent that is detrimental to (e.g., kills) cells. For a description of these classes of drugs which are known in the art, and their mechanisms of action, see Goodman *et al.,* 1990. Additional techniques relevant to the preparation of antibody immunotoxins are provided in for instance US5,194,594. Exemplary toxins include diphtheria A chain, nonbinding active fragments of diphtheria toxin, exotoxin A chain (from *Pseudornonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO93/21232.

Suitable therapeutic agents for forming immunoconjugates include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin, antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin), antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)).

The antibody may be conjugated to a "receptor" (such as streptavidin) for utilization in Treg cell pretargeting wherein the antibody-receptor conjugate is administered to the patient, followed by removal of unbound conjugate from the circulation using a clearing agent and then administration of a "ligand" (e.g., avidin) that is conjugated to a therapeutic agent (e.g., a radionucleotide).

The antibodies can be further modified to contain additional nonproteinaceous moieties that are known in the art and readily available. Preferably, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran or polyvinyl alcohol.

In one example, a compound as described herein comprises one or more detectable markers to facilitate detection and/or isolation. For example, the compound comprises a fluorescent label such as, for example, fluorescein (FITC), 5,6-carboxymethyl fluorescein, Texas red, nitrobenz-2-oxa-1,3- diazol-4-yl (NBD), coumarin, dansyl chloride, rhodamine, 4'-6-diamidino-2- phenylinodole (DAPI), and the cyanine dyes Cy3, Cy3.5, Cy5, Cy5.5 and Cy7, fluorescein (5-carboxyfluorescein-N-hydroxysuccinimide ester), rhodamine (5,6- tetramethyl rhodamine). The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm).

Alternatively, or in addition, the compound that binds to a protein or cell surface marker as described herein is labeled with, for example, a fluorescent semiconductor nanocrystal (as described, for example, in US6,306,610).

Alternatively, or in addition, the compound is labeled with, for example, a magnetic or paramagnetic compound, such as, iron, steel, nickel, cobalt, rare earth materials, neodymium-iron-boron, ferrous-chromium-cobalt, nickel-ferrous, cobalt-platinum, or strontium ferrite.

### Peptides or Polypeptides

The compound that binds to a protein as described herein is a peptide. For example, the peptide is derived from a ligand of a cell surface marker or protein as described herein (e.g., from a ligand binding region of the protein or marker). Other peptides or poplypeptides include, for example, T cell receptors.

Alternatively, a ligand is a peptide isolated from a random peptide library. To identify a suitable ligand, a random peptide library is generated and screened as described in US5,733,731, US5,591,646 and US5,834,318. Generally, such libraries are generated from short random oligonucleotides that are expressed either in vitro or in vivo and displayed in such a way to facilitate screening of the library to identify a peptide that. is capable of specifically binding to a protein or peptide of interest. Methods of display include, phage display, retroviral display, bacterial surface display, bacterial flagellar display, bacterial spore display, yeast surface display, mammalian surface display, and methods of in vitro display including, mRNA display, ribosome display and covalent display.

A peptide that is capable of binding a protein or peptide of interest is identified by a number of methods known in the art, such as, for example, standard affinity purification methods as described, for example in Scopes, 1994) purification using FACS analysis as described in US6,455,63, or purification using biosensor technology as described in Gilligan *et al,* 2002.

In another example, a peptide or polypeptide is a ligand of a protein as described herein. For example, a ligand for PI16 is prostate secretory protein (PSP) 94 (e.g., as described in US5428011). A ligand for PKD1L3 includes an acid (e.g., acetic acid) or a domain of PKD2L1 that interacts with PKD1L3. Ligands for PTPRB include contactin, tenascin C or an extracellular domain of VE-cadherin. A ligand for RGMB includes bone morphogenetic protein (BMP) 2 or BMP4. A ligand for AQP3 is, for example, syntaxin 4. Ligands for CD49f include tetraspanin or laminin. A ligand for LSR is, for example, receptor-associated protein (RAP).

Peptides or polypeptides can be labeled or conjugated as described herein. . Such labeling or conjugation permits detection, isolation or killing Treg cells.

### Small Molecules

A chemical small molecule library is also clearly contemplated for the identification of ligands that specifically bind to a protein or cell surface marker as described herein. Chemical small molecule libraries are available commercially or alternatively may be generated using methods known in the art, such as, for example, those described in US5,463,564.

### Nucleic acid detection/therapeutic reagents

### Probe/Primer Design and Production

As will be apparent to the skilled artisan, the specific probe or primer used in an assay of the present invention will depend upon the assay format used. Clearly, a probe or primer that is capable of specifically hybridizing to or detecting the marker of interest is preferred. Methods for designing probes and/or primers for, for example, PCR or hybridization are known in the art and described, for example, in Dieffenbach and Dveksler, 1995. Furthermore, several software packages are publicly available that design optimal probes and/or primers for a variety of assays, e.g. Primer 3 available from the Center for Genome Research, Cambridge, MA, USA. Probes and/or primers useful for detection of a marker associated Treg cells are assessed to determine those that do not form hairpins, self-prime or form primer dimers (e.g. with another probe or primer used in a detection assay).

Furthermore, a probe or primer (or the sequence thereof) is assessed to determine the temperature at which it denatures from a target nucleic acid (i.e. the melting temperature of the probe or primer, or Tm). Methods of determining Tm are known in the art and described, for example, in Santa Lucia, 1995 or Bresslauer *et al.,* 1986.

Methods for producing/synthesizing a probe or primer are known in the art. For example, oligonucleotide synthesis is described, in Gait, 1984. For example, a probe or primer may be obtained by biological synthesis (e.g. by digestion of a nucleic acid with a restriction endonuclease) or by chemical synthesis. For short sequences (up to about 100 nucleotides) chemical synthesis is preferable.

For longer sequences standard replication methods employed in molecular biology are useful, such as, for example, the use of M13 for single stranded DNA as described by Messing, 1983.

Other methods for oligonucleotide synthesis include, for example, phosphotriester and phosphodiester methods (Narang, *et al.,* 1979) and synthesis on a support (Beaucage, *et al,* 1981) as well as phosphoramidate technique, Caruthers, *et al.,* (1988), and others described in Narang (1987), and the references contained therein.

LNA synthesis is described, for example, in Nielsen *et al,* (1997); Singh and Wengel, (1998). PNA synthesis is described, for example, in Egholm *et al.,* (1992); Egholm *et al.,* (1993); and Orum *et al.,* (1993).

In one embodiment, a probe or primer useful for performance of the method of the invention comprises a nucleotide sequence comprising at least about 20 consecutive nucleotides of a nucleic set forth in any one of Tables 2-6.

The present invention additionally contemplates the use a probe or primer produced according to the methods described herein in the manufacture of a diagnostic reagent for diagnosing or determining a predisposition to a Treg-associated condition.

### Labeled Compounds

Also described is a compound which comprises one or more detectable markers to facilitate detection and/or isolation. For example, the compound comprises a fluorescent label such as, for example, fluorescein (FITC), 5,6-carboxymethyl fluorescein, Texas red, nitrobenz-2-oxa-1,3- diazol-4-yl (NBD), coumarin, dansyl chloride, rhodamine, 4'-6-diamidino-2-phenylinodole (DAPI), and the cyanine dyes Cy3, Cy3.5, Cy5, Cy5.5 and Cy7, fluorescein (5-carboxyfluorescein-N-hydroxysuccinimide ester), rhodamine (5,6-tetramethyl rhodamine). The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm).

Alternatively, or in addition, the compound that binds to a protein or cell surface marker as described herein is labeled with, for example, a fluorescent semiconductor nanocrystal (as described, for example, in US6,306,610).

Alternatively, or in addition, the compound is labeled with, for example, a magnetic or paramagnetic compound, such as, iron, steel, nickel, cobalt, rare earth materials, neodymium-iron-boron, ferrous-chromium-cobalt, nickel-ferrous, cobalt-platinum, or strontium ferrite.

### Cellular Compositions

According to the claims, Treg cells and/or progeny cells thereof are administered in the form of a composition. Preferably, such a composition comprises a pharmaceutically acceptable carrier and/or excipient.

Suitable carriers for this invention include those conventionally used, e.g., water, saline, aqueous dextrose, lactose, Ringer's solution, a buffered solution, hyaluronan and glycols are preferred liquid carriers, particularly (when isotonic) for solutions. Suitable pharmaceutical carriers and excipients include starch, cellulose, glucose, lactose, sucrose, gelatin, malt, rice, flour, chalk, silica gel, magnesium stearate, sodium stearate, glycerol monostearate, sodium chloride, glycerol, propylene glycol, water, ethanol, and the like.

In another example, a carrier is a media composition, e.g., in which a cell is grown or suspended. Preferably, such a media composition does not induce any adverse effects in a subject to whom it is administered.

Preferred carriers and excipients do not adversely affect the viability of a cell and/or the ability of a cell to reduce, prevent or delay a Treg-associated condition.

In one example, the carrier or excipient provides a buffering activity to maintain the cells and/or soluble factors at a suitable pH to thereby exert a biological activity, e.g., the carrier or excipient is phosphate buffered saline (PBS). PBS represents an attractive carrier or excipient because it interacts with cells and factors minimally and permits rapid release of the cells and factors, in such a case, the composition of the invention may be produced as a liquid for direct application to the blood stream or into a tissue or a region surrounding or adjacent to a tissue, e.g., by injection.

Treg cells and/or progeny cells thereof can also be incorporated or embedded within scaffolds that are recipient-compatible and which degrade into products that are not harmful to the recipient. These scaffolds provide support and protection for cells that are to be transplanted into the recipient subjects. Natural and/or synthetic biodegradable scaffolds are examples of such scaffolds. Other suitable scaffolds include polyglycolic acid scaffolds, e.g., as described by Vacanti, *et al.,* 1988; Cima, *et al.,* 1991; Vacanti*, et al.,* 1991; or synthetic polymers such as polyanhydrides, polyorthoesters, and polylactic acid.

Preferably, the composition comprises an effective amount or a therapeutically or prophylactically effective amount of cells. For example, the composition comprises about 1x10⁵ Treg cells/kg to about 1x10⁹ Treg cells/kg or about 1x10⁶ Treg cells/kg to about 1x10⁸ Treg cells/kg or about 1x10⁶ Treg cells/kg to about 1x10⁷ Treg cells/kg. The exact amount of cells to be administered is dependent upon a variety of factors, including the age, weight, and sex of the patient, and the extent and severity of the Treg-associated condition.

The cellular compositions of this invention can be administered to the subject by any recognized methods, either systemically or at a localized site. In one example, the most convenient time to administer the alloactivated cells to prevent GVHD in a transplant patient or in a cancer patient is during the time of surgery. To treat an autoimmune disease, the composition can be administered at the onset of symptoms and/or following onset of symptoms or even prior to the onset of symptoms (e.g., following detection of an autoimmune response). To keep the cells at the site until completion of the surgical procedure, it is convenient to administer the cells in a pharmaceutically compatible artificial gel, or in clotted plasma or by utilizing any other known controlled release mechanism (see above). When less invasive procedures are desired, the composition can be injected at a desired location through a needle. For deeper sites, the needle can be positioned using endoscopic ultrasound techniques, radioscintigraphy, or some other imaging technique, alone or in combination with the use of an appropriate scope or cannula. For such applications, the cell population is conveniently administered when suspended in isotonic saline or a neutral buffer.

### Isolation or Enrichment of Cells

One exemplary approach to enrich for the desired cells is magnetic bead cell sorting (MACS) or any other cell sorting method making use of magnetism, e.g., Dynabeads®. A conventional MACS procedure is described by Miltenyi *et al.,* 1990). In this procedure, cells are labeled with magnetic beads bound to an antibody or other compound that binds to a cell surface marker or protein and the cells are passed through a paramagnetic separation column or exposed to another form of magnetic field. The separation column is placed in a strong magnet, thereby creating a magnetic field within the column. Cells that are magnetically labeled are trapped in the column; cells that are not pass through. The trapped cells are then eluted from the column.

Cells of the invention can be enriched, for example, from a suitable sample using MACS to separate cells expressing a suitable protein. The sample is incubated with immunomagnetic beads that bind to the protein. Following incubation, samples are washed and resuspended and passed through a magnetic field to remove cells bound to the immunomagnetic beads, and cells bound to the beads collected. These techniques are equally applicable to negative selection, e.g., removal of cells expressing an undesirable marker, e.g., CD8, i.e., undesirable cells. Such a method involves contacting a population of cells with a magnetic particle labeled with a compound that binds to a cell surface marker expressed at detectable levels on the undesirable cell type(s). Following incubation, samples are washed and resuspended and passed through a magnetic field to remove cells bound to the immunomagnetic beads. The remaining cells depleted of the undesirable cell type(s) are then collected.

In another embodiment, a compound that binds to a protein or cell surface marker is immobilized on a solid surface and a population of cells is contacted thereto. Following washing to remove unbound cells, cells bound to the compound can be recovered, e.g., eluted, thereby isolating or enriching for cells expressing the protein to which the compound binds. Alternatively, cells that do not bind to the compound can be recovered if desired.

In a preferred embodiment, cells are isolated or enriched using fluorescence activated cell sorting (FACS). FACS is a known method for separating particles, including cells, based on the fluorescent properties of the particles and described, for example, in Kamarch, 1987. Generally, this method involves contacting a population of cells with compounds capable of binding to one or more proteins or cell surface markers, wherein compounds that bind to distinct markers are labeled with different fluorescent moieties, e.g., fluorophores. The cells are entrained in the center of a narrow, rapidly flowing stream of liquid. The flow is arranged so that there is a separation between cells relative to their diameter. A vibrating mechanism causes the stream of cells to break into individual droplets. The system is adjusted so that there is a low probability of more than one cell being in a droplet. Just before the stream breaks into droplets the flow passes through a fluorescence measuring station where the fluorescent character of interest of each cell is measured, e.g., whether or not a labeled compound is bound thereto. An electrical charging ring is placed at the point where the stream breaks into droplets. A charge is placed on the ring based on the immediately prior fluorescence intensity measurement and the opposite charge is trapped on the droplet as it breaks from the stream. The charged droplets then fall through an electrostatic deflection system that diverts droplets into containers based upon their charge, e.g., into one container if a labeled compound is bound to the cell and another container if not. In some systems the charge is applied directly to the stream and the droplet breaking off retains charge of the same sign as the stream. The stream is then returned to neutral after the droplet separates.

### Cell Culture

Following isolation cells of the invention can be maintained under standard cell culture conditions. For example, the cells can be maintained in Dulbecco Minimal Essential Medium (DMEM) or any other appropriate cell culture medium known in the art, e.g., as described above. Other appropriate media include, for example, MCDB, Minimal Essential Medium (MEM), IMDM, EXvivo™ and RPMI.

Cell cultures are preferably incubated at about 37°C in a humidified incubator. Cell culture conditions can vary considerably for the cells of the present invention. Preferably, the cells are maintained in an environment suitable for cell growth, e.g., comprising 5% O₂, 10% CO₂, 85% N₂ or comprising 10% CO₂ in air.

In one embodiment, Treg cells are grown in the presence of anti-CD3 antibodies to facilitate expansion, optionally in the presence of IL-2 and/or TGF-β. In one example, cells are cultured in the presence of beads (e.g., magnetic iron-dextran beads-Dynabeads) coated with antibodies to CD3 and CD28. The anti-CD28 antibody provides signals for augmented activation and growth of the hypo-proliferative Treg cells. CD4⁺CD25⁺ cells grown with the beads with low ratio (low anti-CD3 compared to anti-CD28) are much more stable, and less likely to be overgrown with conventional T cells. The beads can easily be removed by passing the cultured cells through a magnetic column. Cell sorting is not required.

Treg cells can also be cultured in the presence of cytokines, e.g., IL-4 and/or IL-7, which increase survival of T cells and/or IL-10, which is partially responsible for production of regulatory T cells and/or IL-15, which has been shown to synergize with IL-2 and induce proliferation of CD4⁺CD25⁺ T cells.

In some examples, autologous CD4⁺ T cells as feeder cells. Alternatively, feeder-free cultures can be performed, e.g., using the beads described above together with IL-2. Moreover culture-expansion can be accomplished with or without host APCs, e.g., DCs.

### Stimulating or Enhancing an Immune Response

As will be apparent to the skilled artisan based on the description herein, the present disclsoure also contemplates methods for enhancing an immune response in a subject by reducing or depleting Treg cells in the subject. In one example, reducing or depleting Treg cells in the subject is sufficient to induce an immune response, e.g., against a tumor and/or against an infectious agent. In another example, a method for inducing an immune response against a subject involves reducing or depleting Treg cells in the subject and administering a composition comprising an immunogenic compound.

As used herein, the term "immunogen" means any substance or organism that provokes an immune response (produces immunity) when introduced to a subject. In some embodiments, an immunogen can be used in therapeutic settings in a form of a vaccine. As used herein, and unless otherwise specified, the term "enhanced immune response" means that, when an immunogen is administered in combination with compound according to methods of this disclosure there is an increased immunological response, preferably T cell response and/or antibody response, measured using any standard methods known in the art or described herein, in a subject that receives such an administration as compared to a subject to which a compound is not administered.

Immunogenic compounds used may be a cancer antigen or a tumor antigen. Any cancer or tumor antigen known to one skilled in the art may be used in accordance with the immunogenic compositions including, but not limited to, KS 1/4 pan-carcinoma antigen (Perez and Walker, 1990), ovarian carcinoma antigen (CA125) (Yu *et al,* 1991), prostatic acid phosphate (Tailor *et al,* 1990), prostate specific antigen (Henttu and Vihko, 1989), melanoma-associated antigen p97 (Estin *et al,* 1989), melanoma antigen gp75 (Vijayasardahl *et al,* 1990), high molecular weight melanoma antigen (HMW-MAA) (Natali *et al,* 1987), prostate specific membrane antigen, carcinoembryonic antigen (CEA) (Foon *et al,* 1994), TAG-72 (Yokata *et al,* 1982), CO17-1A (Ragnhammar *et al,* 1993); GICA 19-9 (Herlyn *et al,* 1982), CD19 (Ghetie *et al,* 1994), human B-lymphoma antigen-CD20 (Reff *et al,* 1994), CD33 (Sgouros *et al,* 1993), GD2 (Saleh *et al,* 1993), ganglioside GD3 (Shitara *et al.,* 1993), ganglioside GM2 (Livingston *et al.,,* 1994), ganglioside GM3 (Hoon *et al.,* 1993), bladder tumor oncofetal antigen (Hellstrom *et al,* 1985), L20 (Hellstrom *et al,* 1986), human leukemia T cell antigen-Gp37 (Bhattacharya-Chatterjee *et al,* 1986), EGFR (Epidermal growth factor receptor), HER2 antigen, polymorphic epithelial mucin (PEM) (Hilkens *et al,* 1992), malignant human lymphocyte antigen-APO-1 (Bernhard *et al,* 1989), differentiation antigen (Feizi, 1985), MAGE-I, MAGE-3, BAGE, GAGE-I, GAGE-I. Additional tumor antigens are described, for example, in Novellino *et al.,* 2005.

In another example, the immunogen is a cancer cell or a lysate thereof.

In one example, the cancer is breast cancer.

In another example, the cancer is brain cancer, e.g., glioma.

In a further example, the cancer is gastric cancer.

In another example, the cancer is prostate cancer.

In another example, the cancer is melanoma.

In another example, the cancer is lymphoma, e.g., Hodgkin's lymphoma.

Immunogens used in methods of this invention may also be an infectious disease agent including, but not limited to, influenza virus hemagglutinin (Genbank Accession No. JO2132; Air, 1981), human respiratory syncytial virus G glycoprotein (Genbank Accession No. Z33429; Collins *et al,* 1984), core protein, matrix protein or any other protein of Dengue virus (Genbank Accession No. M19197; Hahn *et al,* 1988), measles virus hemagglutinin (Genbank Accession No. M81899; Rota *et al,* 1982), herpes simplex virus type 2 glycoprotein gB (Genbank Accession No. M14923; Bzik *et al,* 1986), poliovirus I VP1 (Emini *et al,* 1983), envelope glycoproteins of HIV-I (Putney *et al,* 1986), hepatitis B surface antigen (Itoh *et al,* 1986), diptheria toxin (Audibert *et al,* 1981), streptococcus 24M epitope (Beachey, 1985), hepatitis B virus core protein and/or hepatitis B virus surface antigen or a fragment or derivative thereof (see, e.g., GB 2034323).

In one example, the immunogenic compound is a DNA encoding a polypeptide antigen.

In one example, the immunogenic composition additionally comprises an adjuvant. Adjuvants are molecules and preparations that improve the immunogenicity of antigens. They can have immunopotentiating activity, but can also have, instead of or in addition to such activity, properties to alter the physical state of the immunogen. The effects of adjuvants are not antigen-specific. If they are administered together with a purified antigen, however, they can be used to selectively promote the response to the antigen. For example, the immune response is increased when protein antigens are precipitated by alum. Emulsification of antigens also prolongs the duration of antigen presentation. Suitable adjuvants include all acceptable immunostimulatory compounds, such as cytokines, toxins or synthetic compositions. Exemplary, often preferred adjuvants include, but are not limited, complete Freund's adjuvant (a non-specific stimulator of the immune response containing killed *Mycobacterium tuberculosis*), incomplete Freund's adjuvants, and aluminum hydroxide adjuvant. Other adjuvants that can also be used include MDP compounds, such as, for example, thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween 80 emulsion is also contemplated. Amphipathic and surface active agents, e.g., saponin and derivatives such as QS21 (Cambridge Biotech), form yet another group of adjuvants contemplated for use in embodiments of the present invention. Nonionic block copolymer surfactants can also be employed.

### Suppressing and Immune Response

The present invention also provides a method of treating or preventing a condition associated with reduced Treg cell numbers or activity, and/or inducing immunosuppression, and/or reducing CTL or Thelper cell activity in a subject, said method comprising:
(i) isolating a population of Treg cells by performing a method as described herein according to any embodiment; and
(ii) administering the cells at (i) to the subject.

Altrernatively, or in addition, the method comprises administering a population of cells as described herein according to any embodiment.

In one example, the subject suffers from or is at risk of developing a condition associated with reduced Treg numbers and/or activity and/or requires a reduction in CTL or Thelper cell activity (e.g., the subject suffers from or is at risk of developing an autoimmune disease) and/or the subject requires immunosuppression (e.g., is undergoing or about to undergo a transplant or suffers from graft-versus-host disease). Methods for determining a subject suffering from a condition will be apparent to the skilled artisan based on the description herein.

In one example, the subject suffers from type 1 diabetes.

In another example, the subject suffers from multiple sclerosis.

In a further example, the subject suffers from inflammatory bowel disease.

In a preferred example, the subject suffers from arthritis, e.g., rheumatoid arthritis.

Methods for identifying and/or isolating and/or culturing and/or formulating Treg cells for therarpy are described herein.

### Detection Assays

### Protein Detection Assays

In one embodiment, the method of the invention detects the presence of a protein, namely PI16. The amount, level or presence of a polypeptide is determined using any of a variety of techniques known to the skilled artisan such as, for example, a technique selected from the group consisting of, immunohistochemistry, immunofluorescence, an immunoblot, a Western blot, a dot blot, an enzyme linked immunosorbent assay (ELISA), radioimmunoassay (RIA), enzyme immunoassay, fluorescence resonance energy transfer (FRET), matrix-assisted laser desorption/ionization time of flight (MALDI-TOF), electrospray ionization (ESI), mass spectrometry (including tandem mass spectrometry, e.g. LC MS/MS), biosensor technology, evanescent fiber-optics technology or protein chip technology.

In one embodiment the assay used to determine the amount or level of a protein is a semi-quantitative assay.

In another embodiment the assay used to determine the amount or level of a protein is a quantitative assay.

Preferably, the protein is detected with an immunoassay. Preferably, using an assay selected from the group consisting of, immunohistochemistry, immunofluorescence, enzyme linked immunosorbent assay (ELISA), fluorescence linked immunosorbent assay (FLISA) Western blotting, RIA, a biosensor assay, a protein chip assay and an immunostaining assay (e.g. immunofluorescence). Preferably, the detection method is a flow cytometry method, e.g, detection of the number of Treg cells using fluorescence activated cell sorting (FACS), e.g., as is known in the art and/or described herein.

Standard solid-phase ELISA or FLISA formats are particularly useful in determining the concentration of a protein from a variety of samples.

In one form such an assay involves immobilizing a biological sample onto a solid matrix, such as, for example a polystyrene or polycarbonate microwell or dipstick, a membrane, or a glass support (e.g. a glass slide). A compound (e.g., an antibody) that specifically binds to a protein set out in any one or more of Tables 2, 4 or 5 is brought into direct contact with the immobilized biological sample, and forms a direct bond with any of its target protein present in said sample. This antibody is generally labeled with a detectable reporter molecule, such as for example, a fluorescent label (e.g. FITC or Texas Red) or a fluorescent semiconductor nanocrystal (as described in US 6,306,610) in the case of a FLISA or an enzyme (e.g. horseradish peroxidase (HRP), alkaline phosphatase (AP) or β-galactosidase) in the case of an ELISA, or alternatively a second labeled antibody can be used that binds to the first antibody. Following washing to remove any unbound antibody the label is detected either directly, in the case of a fluorescent label, or through the addition of a substrate, such as for example hydrogen peroxide, TMB, or toluidine, or 5-bromo-4-chloro-3-indol-beta-D-galaotopyranoside (x-gal) in the case of an enzymatic label. Such ELISA or FLISA based systems are particularly suitable for quantification of the amount of a protein in a sample, by calibrating the detection system against known amounts of a protein standard to which the antibody binds, such as for example, an isolated and/or recombinant polypeptide or immunogenic fragment thereof or epitope thereof.

In another form, an ELISA or FLISA comprises of immobilizing a compound (e.g., an antibody) on a solid matrix, such as, for example, a membrane, a polystyrene or polycarbonate microwell, a polystyrene or polycarbonate dipstick or a glass support. A sample is then brought into physical relation with said antibody, and the protein to which said compound binds is bound or 'captured'. The bound protein is then detected using a second labeled compound that binds to a different protein or a different site in the same protein. Alternatively, a third labeled antibody can be used that binds the second (detecting) antibody.

It will be apparent to the skilled person that the assay formats described herein are amenable to high throughput formats, such as, for example automation of screening processes or a microarray format as described in Mendoza *et al.,* 1999. Furthermore, variations of the above-described assay will be apparent to those skilled in the art, such as, for example, a competitive ELISA.

In an alternative embodiment, a polypeptide is detected within or on a cell, using methods known in the art, such as, for example, immunohistochemistry or immunofluorescence. Methods using immunofluorescence are preferable, as they are quantitative or at least semi-quantitative. Methods of quantitating the degree of fluorescence of a stained cell are known in the art and described, for example, in Cuello, 1984.

Biosensor devices generally employ an electrode surface in combination with current or impedance measuring elements to be integrated into a device in combination with the assay substrate (such as that described in US5,567,301). A compound that specifically binds to a protein o is preferably incorporated onto the surface of a biosensor device and a biological sample contacted to said device. A change in the detected current or impedance by the biosensor device indicates protein binding to said antibody. Some forms of biosensors known in the art also rely on surface plasmon resonance to detect protein interactions, whereby a change in the surface plasmon resonance surface of reflection is indicative of a protein binding to a ligand or antibody (US5,485,277 and US5,492,840).

Biosensors are of particular use in high throughput analysis due to the ease of adapting such systems to micro- or nano-scales. Furthermore, such systems are conveniently adapted to incorporate several detection reagents, allowing for multiplexing of diagnostic reagents in a single biosensor unit. This permits the simultaneous detection of several proteins or peptides in a small amount of body fluids.

Evanescent biosensors are also preferred as they do not require the pretreatment of a biological sample prior to detection of a protein of interest. An evanescent biosensor generally relies upon light of a predetermined wavelength interacting with a fluorescent molecule, such as for example, a fluorescent antibody attached near the probe's surface, to emit fluorescence at a different wavelength upon binding of the target polypeptide to the compound.

Micro- or nano-cantilever biosensors are also preferred as they do not require the use of a detectable label. A cantilever biosensor utilizes a compound capable of specifically detecting the analyte of interest that is bound to the surface of a deflectable arm of a micro- or nano-cantilever. Upon binding of the analyte of interest (e.g. a marker within a polypeptide) the deflectable arm of the cantilever is deflected in a vertical direction (i.e. upwards or downwards). The change in the deflection of the deflectable arm is then detected by any of a variety of methods, such as, for example, atomic force microscopy, a change in oscillation of the deflectable arm or a change in pizoresistivity. Exemplary micro-cantilever sensors are described in US20030010097.

To produce protein chips, the proteins, peptides, polypeptides, antibodies or ligands that are able to bind specific antibodies or proteins of interest are bound to a solid support such as for example glass, polycarbonate, polytetrafluoroethylene, polystyrene, silicon oxide, metal or silicon nitride. This immobilization is either direct (e.g. by covalent linkage, such as, for example, Schiff's base formation, disulfide linkage, or amide or urea bond formation) or indirect, Methods of generating a protein chip are known in the art and are described in for example US20020136821, US20020192654, US20020102617 and US6,391,625. To bind a protein to a solid support it is often necessary to treat the solid support so as to create chemically reactive groups on the surface, such as, for example, with an aldehyde-containing silane reagent. Alternatively, an antibody or ligand may be captured on a microfabricated polyacrylamide gel pad and accelerated into the gel using microelectrophoresis as described in, Arenkov *et al.,* 2000.

### Imaging Methods

As will be apparent to the skilled artisan from the foregoing, the present disclosure also contemplates imaging methods using a compound that binds to a protein as claimed . For imaging, a compound is generally conjugated to a detectable label, which can be any molecule or agent that can emit a signal that is detectable by imaging. However, a secondary labeled compound that specifically binds to the primary compound may also be used. Exemplary detectable labels include a protein, a radioisotope, a fluorophore, a visible light emitting fluorophore, infrared light emitting fluorophore, a metal, a ferromagnetic substance, an electromagnetic emitting substance a substance with a specific magnetic resonance (MR) spectroscopic signature, an X-ray absorbing or reflecting substance, or a sound altering substance.

The detection compound (and, if used the labeled secondary compound) can be administered either systemically or locally to an organ, or tissue (or tumor, in the case of a cancer) to be imaged, prior to the imaging procedure. Generally, the compound is administered in doses effective to achieve the desired optical image of a tumor, tissue, or organ. Such doses may vary widely, depending upon the particular compound employed, condition to be imaged, tissue, or organ subjected to the imaging procedure, the imaging equipment being used, and the like.

In some examples, the compound is used as *in vivo* optical imaging agents of tissues and organs in various biomedical applications including, but not limited to, imaging of tumors, tomographic imaging of organs, monitoring of organ functions, coronary angiography, fluorescence endoscopy, laser guided surgery, photoacoustic and sonofluorescence methods, and the like.

Examples of imaging methods include magnetic resonance imaging (MRI), MR spectroscopy, radiography, computerized tomography (CT), ultrasound, planar gamma camera imaging, single-photon emission computed tomography (SPECT), positron emission tomography (PET), other nuclear medicine-based imaging, optical imaging using visible light, optical imaging using luciferase, optical imaging using a fluorophore, other optical imaging, imaging using near infrared light, or imaging using infrared light.

In some examples, an imaging agent is tested using an *in vitro* or *in vivo* assay prior to use in humans, e.g., using a model described herein.

### Nucleic Acid Detection Assays

In another example, a Treg cell is detected and/or a Treg-associated condition is diagnosed/prognosed by detecting the level of expression of a nucleic acid. Exemplary assays for such detection include quantitative RT-PCR, NASBA, TMA or ligase-chain reaction.

Methods of RT-PCR are known in the art and described, for example, in Dieffenbach (ed) and Dveksler (ed) 1995.

Methods of TMA or self-sustained sequence replication (3SR) use two or more oligonucleotides that flank a target sequence, a RNA polymerase, RNase H and a reverse transcriptase. One oligonucleotide (that also comprises a RNA polymerase binding site) hybridizes to an RNA molecule that comprises the target sequence and the reverse transcriptase produces cDNA copy of this region. RNase H is used to digest the RNA in the RNA-DNA complex, and the second oligonucleotide used to produce a copy of the cDNA. The RNA polymerase is then used to produce a RNA copy of the cDNA, and the process repeated.

NASBA systems relies on the simultaneous activity of three enzymes (a reverse transcriptase, RNase H and RNA polymerase) to selectively amplify target mRNA sequences. The mRNA template is transcribed to cDNA by reverse transcription using an oligonucleotide that hybridizes to the target sequence and comprises a RNA polymerase binding site at its 5' end. The template RNA is digested with RNase H and double stranded DNA is synthesized. The RNA polymerase then produces multiple RNA copies of the cDNA and the process is repeated.

Clearly, the hybridization to and/or amplification of a nucleic acid using any of these methods is detectable using, for example, electrophoresis and/or mass spectrometry. In this regard, one or more of the probes/primers and/or one or more of the nucleotides used in an amplification reactions may be labeled with a detectable marker to facilitate rapid detection of a marker, for example, a fluorescent label (e.g. Cy5 or Cy3) or a radioisotope (e.g. ³²P). Alternatively, amplification of a nucleic acid may be continuously monitored using a melting curve analysis method, such as that described in, for example, US6174670.

### Samples

To the extent that the method of the present invention is performed *in vitro,* on an isolated tissue sample, rather than as an *in vivo* based screen, reference to "sample" should be understood as a reference to any sample of biological material derived from an animal such as, but not limited to, a body fluid (e.g., blood or synovial fluid or cerebrospinal fluid), cellular material (e.g. tissue aspirate), tissue biopsy specimens or surgical specimens. The "biological sample" or "tissue sample" includes extracts and/or derivatives and/or fractions of said sample, e.g., serum, plasma, peripheral blood mononuclear cells (PBMC), a buffy coat fraction. Preferably, the sample comprises Treg cells or is likely to comprise Treg cells.

The sample which is used according to the present invention may be used directly or may require some form of treatment prior to use. For example, a biopsy or surgical sample may require homogenization or other form of cellular dispersion prior to use. Furthermore, to the extent that the biological sample is not in liquid form, (if such form is required or desirable) it may require the addition of a reagent, such as a buffer, to mobilize the sample.

As will be apparent from the preceding description, such an assay may require the use of a suitable control, e.g. a normal or healthy individual or a typical population, e.g., for quantification.

As used herein, the term "normal individual" shall be taken to mean that the subject is selected on the basis that they do not have abnormal numbers of Treg cells in a sample derived therefrom, e.g., they have a level of Treg cells within the range detected in a population of healthy subjects.

A "healthy subject" is one that has not been diagnosed as suffering from a Treg-associated condition and/or is not at risk of developing a Treg-associated condition..

Alternatively, or in addition, a suitable control sample is a control data set comprising measurements of the marker being assayed for a typical population of subjects known not to suffer from a Treg-associated condition.

In one embodiment, a reference sample is not included in an assay. Instead, a suitable reference sample is derived from an established data set previously generated from a typical population. Data derived from processing, analyzing and/or assaying a test sample is then compared to data obtained for the sample population.

### In Vivo Assays

In another example, a population of cells isolated by a method as described herein according to any embodiment is determined by administering the cells to an animal model of a condition associated with Treg cells. For example, the cells are administered to an animal lacking Treg cells e.g., as a result of myeloablation or mice lacking FoxP3 expression e.g., as described in (Asano (1996); Suri-Payer (1998); and McHugh (2002). Cells that suppress, prevent, reduce or delay an autoimmune response are considered to have regulatory T cell function.

Alternatively, or in addition, cells isolated by a method as described herein according to any embodiment are administered to nude mice at the time of, prior to or after transfer of CD4⁺CD25⁻ (which induce autoimmune disease), e.g., as described in Sakaguchi *et al.,* (1995). Cells that suppress, prevent, reduce or delay an autoimmune response are considered to have regulatory T cell function.

Isolated cells can also be administered to NOD mice to test their ability to suppress, prevent, treat or delay diabetes (e.g., as described in Tang *et al.,* (2004)) and/or to a mouse model of GVHD (e.g., as described in Trenado (2002)) and/or to a mouse model of psoriasis (e.g., Wang *et al.,* 2008) and/or to a model of rheumatoid arthritis e.g., a SKG strain of mouse (Sakaguchi *et al*.), rat type II collagen arthritis model, mouse type II collagen arthritis model or antigen induced arthritis models in several species (Bendele, 2001)) and/or a model of multiple sclerosis (for example, experimental autoimmune encephalomyelitis (EAE; Bradl and Linington, 1996)) and/or inflammatory airway disease (for example, OVA challenge or cockroach antigen challenge (Chen *et al.,* 2007; Lukacs *et al.,* 2001) and/or models of inflammatory bowel disease (e.g., dextran sodium sulphate (DSS)-induced colitis or Muc2 deficient mouse model of colitis (Van der Sluis *et al.,* 2006) or CD45Rb adoptive transfer model of colitis (e.g., Kanai *et al.,* 2006)). These models are also useful for testing compounds for their ability to enhance or increase Treg function.

Compounds to be tested for their ability to suppress Treg function and/or numbers can be administered to a test subject and the number of Treg cells detected/isolated using standard methods or methods described herein. A reduction in the number of Treg cells compared to the number of Treg cells from an untreated subject indicates that the compound reduces Treg numbers.

Alternatively, or in addition, a compound is administered to a test subject at the time of, prior to or following administration of tumor cells. The presence/absence and/or size of any resulting tumor is then assessed and compared to subjects to which the cells but not the compound has been administered. A compound that reduces tumor size and/or prevents tumor formation is considered to reduce Treg function and permit induction of an immune response against the tumor cells. Exemplary methods are described in Jones *et al.,* 2002.

In another example, a test compound is administered at the time of or prior to administration of a composition comprising an immunogenic compound. The immune response is then measured against the immunogenic compound, e.g., antibody response (e.g., by ELISA/FLISA) or a T cell response (e.g., by ELISPOT or Fluorospot). Alternatively, or in addition, the compound and composition are administered to a subject suffering from or developing a condition treatable by an immune response, e.g., an infection or a tumor. Suitable models are known in the art and/or described herein.

### Kits

Also described are therapeutic/prophylactic/diagnostic kits comprising compounds of the present invention for use in the present detection/isolation/diagnostic/prognostic/treatment/prophylactic methods. Such kits will generally contain, in suitable container means, a compound of the present invention. The kits may also contain other compounds, e.g., for detection/isolation/diagnosis/imaging or combined therapy. For example, such kits may contain any one or more of a range of anti-inflammatory drugs and/or chemotherapeutic or radiotherapeutic drugs; anti-angiogenic agents; anti-tumor cell antibodies; and/or anti-tumor vasculature or anti-tumor stroma immunotoxins or coaguligands or vaccines.

In one example, the kit is for isolating a Treg cell and comprises a protein that binds to a protein identified in any one or more of Tables 2-6. Optionally, the compound is bound to or conjugated with a detectable label or a solid support or a magnetic particle. For example, the compound is labeled with a detectable label to facilitate FACS. The compound may also be labeled with a magnetic or paramagnetic particle to facilitate MACS.

In a further example, the kit is for treatment or prevention of a condition. In such kits the compound may be provided in solution or in a lyophilized form, optionally with a solution for resuspension. The protein may be conjugated to a therapeutic compound or the kit may include a therapeutic compound for conjugation thereto. As discussed above, the kit may also comprise additional therapeutic or prophylactic compounds.

The present invention is described further in the following non-limiting examples.

### Example 1 - Materials and Methods

### 1.1 Isolation, In Vitro Expansion and Characterization of Cord Blood T cell

### Populations

Cord blood was obtained with informed maternal consent. Mononuclear cells (MNC) were isolated from 60-100ml venous blood collected directly from cord blood post partum into pre-weighed blood collection bags (Fenwell) containing anticoagulant essentially as described in Bersatz (2007). Cord blood CD4⁺CD25⁺ (Treg) and CD4⁺CD25⁻ (Thelper) cells were isolated from MNC using a Dynabeads Regulatory CD4⁺CD25⁺ T cell kit (Invitrogen). The purity for each cell type was routinely greater than 90% by two color flow cytometry for CD4 and CD25 expression. *Ex vivo* expansion of isolated T cell populations (1x10⁶ cells/well in a 24 well plate) were performed in X-Vivo 15 media (BioWhitticker) supplemented with 20mM HEPES-, pH 7.4, 5% heat inactivated pooled human serum (Lonza), 2mM 1-glutamine and 500U/ml recombinant human interleukin-2 (rhIL2; R&D research) in the presence of Dynabeads CD3/CD28 T cell expander beads (Invitrogen) at a bead to cell ratio of 3:1. Cells were expanded for 8 days in the presence of Dynabeads prior to magnetic removal of the beads and culture in X-Vivo 15 media supplemented as above except with 100U/ml rhIL2. On the day of use the phenotype of expanded cells were characterized by surface expression of CD4 (PEcy5, ebioscience clone RPA-T4), CD25 (PE, BD clone MA251) and CD127 (PE, ebioscience clone ebioRDR73) in combination with intracellular detection of Foxp3 (Foxp3-Alexa 488, BD clone 259D/C7; Human FoxP3 Buffer Set) by three color flow cytometry on a Beckman Coulter Epics Elite ESP flow cytometer.

### RNA Preparation and Expression Array

Total RNA was isolated from expanded cells that were rested for approximately 60 hours prior to Ionomycin treatment (2 hours). Total RNA, including small molecular weight RNA, was isolated using QIAshredder and a miRNeasy mini kit (QIAGEN). Labeling and hybridization to Affymetrix Human Exon 1.0ST arrays was carried out essentially according to the manufacturer's protocols at the Biomolecular Resource Facility (John Curtin School of Medical Research, Australian National University). RNA quality was assayed for using an Agilent Systems Bioanalyzer prior to array experiments.

### RNA Expression Array Data Analysis

### Data Normalization

Probe level data was processed using the RMA model (Irrizary *et al,* 2003) with final transcript level estimates being obtained using probe-level modeling (Bolstad *et al.,* 2003) based on vs11.0 of an EntrezGene centric cdf (Dai *et al.,* 2004). All processing was conducted using the statistical software R(R Development Core Team) under the aroma Affymetrix framework (Bengtsson and Bengtsson, 2006; Bengtsson and Hossjer, 2006). Hybridization quality for each array was assessed using pseudo-image plots, NUSE, RLE (Bolstad *et al.,* 2003) and histograms of probe-level data. Log fold-change in a four way comparison between resting and stimulated treatments of CD4⁺CD25⁺ Treg cells vs CD4⁺CD25⁻ Thelper cells was estimated within individual donors after loess normalization. Final expression analysis was performed using array level weights and the R package limma (Smyth *et al.,* 2005; Wettenhall and Smyth, 2004). Raw p-values for each term were adjusted globally using an FDR cutoff of 0.05 (Hochberg and Benjamini, 1990).

### Selection of Significantly Expressed Genes

Significantly differentially expressed genes for each comparison were selected using a two-tiered approach. The first tier consisted of the most highly ranked genes selected using defined FDR cut-offs [FDR< 0.05 - 0.006 depending upon the individual comparison]. In the second tier, genes with a logFC greater than 0.7 and an FDR of less-than 0.05 were also selected except for the Treg .v. Thelper Resting comparison, where a logFC greater than 0.5 and an FDR less than 0.1 was used (Figure 4). The four lists were then combined, and logFC values for each comparison included.

### Clustering Gene Expression Behavior

Genes from the combined list with a significant adjusted p-value in either of the CD25⁺ vs. CD25⁻ comparisons (p<0.05 for Stimulated, p<0.1 for Resting) were divided into 6 mutually exclusive groups, denoted as groups T1 to T6 (Table 6) based upon their behavior across all four comparisons. Genes with no effect in either CD25⁺ vs. CD25⁻ comparison but with an activation effect in both CD25⁺ and CD25⁻ were additionally assigned to group Al.Clustering within each subgroup was performed using the agnes algorithm (Maechler *et al;* unpublished), based on significant log fold-change. For genes with a significant adjusted p-value in only one of the comparisons, the threshold of significance was raised to p<0.1 for all other comparisons. (All p-values < 10⁻⁷).

### Semi-Quantitative Real Time PCR

Random primed cDNA was prepared from total RNA using a QuantiTect reverse transcriptase kit (QIAGEN). cDNA was used in a 25ul qPCR reactions consisting of 1.25Units FastTaq DNA polymerase (Roche), 2.5mM MgC12, 200uM each primer, 100uM dNTP mix, 1.1x SYBR Green 1 (Molecular Probes) in 1x FastTaq PCR buffer. Gene specific primers pairs for qPCR were selected from Primerbank (Wang 2003). A primer set specific for RPL13a was used as an internal control. Gene specific primers pairs for qPCR were selected from Primerbank (Spandidos *et al.,* 2008). Cycling conditions consisted of 40 cycles of: 50 seconds at 94°C, 25 seconds at 60°C, and 50 seconds at 72°C followed by melt curve using a Rotorgene 6000 PCR machine (Corbet Research). Results were analyzed using Rotor-Gene 6000 and Q-gene software (Perikles Bioinformatics 19: 1439-1440, 2003).

### Treg Gene Expression Validation

To validate candidate genes identified in the expression array experiments, CD4⁺CD25⁺ or CD4⁺CD25⁻ cells purified from cord blood were cultured and stimulated as above. In addition, Adult peripheral blood was obtained from buffy coats provided by the Australian Red Cross Blood Service. Total blood was stained with a CD4 RosetteSep enrichment cocktail (StemCell Technologies Inc) and PBMC isolated by Ficoll density gradient centrifugation, resulting in a population of 85-95% pure CD4⁺ cells. The CD4⁺ enriched PBMC were then stained with CD25-PE, CD4-APCH7, CD45RO-PeCy7 and CD45RA-APC conjugated monoclonal antibodies (BD Biosciences). Lymphocytes were gated based on forward/side scatter followed by CD4 CD25 double positive expression or CD4 single positive expression. These two cell populations were further sub-divided based on CD45RA or CD45R0 positivity, excluding populations with intermediate CD45RA and CD45R0 expression. The four sorted populations were: CD4⁺CD25⁺CD45RA⁺CD45R0⁻ (naive Treg), CD4⁺CD25⁺CD45RA⁻CD45R0⁺ (activated/memory Treg), CD4⁺CD25⁻ CD45RA⁺CD45R0⁻(naïve Th), CD4⁺CD25⁻CD45RA⁻CD45R0⁺ (memory Th). Samples of cells were retained to assess FoxP3 protein by intracellular flow cytometry using an Alexa Fluor 488 Human FoxP3 Staining Set (eBioscience) and were 60-80 % FoxP3 positive. RNA was then purified from the four sorted populations using a miRNeasy Mini Kit (Qiagen). FoxP3 mRNA expression in the sorted populations was confirmed by quantitative RT-PCR.

### Validation of Cell Surface Molecules

For surface molecule analysis cord blood CD4⁺CD25⁺Treg or CD4⁺CD25⁻ Thelper cells were either freshly isolated or isolated and stimulated overnight in the presence of CD3/CD28 beads (bead to cell ratio, 1:1) and 100U/ml IL2, prior to 4 color flow cytometry using antibodies against CD4, CD25, FoxP3 and test antigen.

### Target Validation by Flow Cytometry

Purified and unconjugated antibodies against the following proteins were purchased from the indicated source: AQP3 (Santa Cruz), C18orf1 (Abnova), CD11b (Becton Dickinson Biosciences, BD), CD121 (BD), CD146 (eBioscience), CD33 (BD), CD49f (BD), CD66 (BD), CD79a (BD), CD79b (BD), CD8b (PE conjugated, BD), EPHB1 (Abnova), FCER1g (Santa Cruz), IFNgR2 (Abcam), LRP6 (Santa Cruz), LSR (Abnova), NPDC1 (Abcam), PERP (Abnova), PI16 (pAb, Abnova), PKD1L3 (Abcam), PTPRb (Santa Cruz), RGMB (Santa Cruz), TLR6 (eBioscience), TSPAN15 (Santa Cruz), VSIG1 (R&D Systems). For each antibody the appropriate isotype-control (species, Ig isotype and company) was used.

Analyses of the reactivity of the target antibodies were performed using a three-step "high sensitivity" staining protocol (Mavrangelos et al., 2004) on peripheral blood mononuclear cells (PBMC). Peripheral blood mononuclear cells were isolated from whole blood (obtained from healthy volunteers) by density centrifugation over Lymphoprep (Nycomed) and were washed twice with PBS-Azide. Blood was collected using lithium-heparin anticoagulant.

PBMC were incubated with the unconjugated primary antibody for 30min on ice, and then washed twice with PBS-azide. Biotinylated horse anti-mouse Ig reagent (VECTOR Laboratories) was added followed by 30min incubation and two PBS-azide washes. Normal mouse serum (Dako) was added (to block free Ig-binding sites on the anti-mouse Ig) and 10min later the SA-PE detection reagent (BD), as well as the directly conjugated antibodies (CD3, CD4, CD25, CD127 - depending on the screening step) were added. No wash step was included between the incubation of the mouse Ig and the addition of the final reagents. All incubations were carried out in melting ice. Cells were acquired on a FACSAria II flow cytometer and analyzed using FACSDiva software v5.0.2 (both BD). For data analysis, 100.000 viable cells (gated in FSC/SSC dot plots) were acquired for each sample.

In experiments where FoxP3 expression was analyzed, the surface staining was performed first, and the cells were then permeabilized and stained according to the instructions provided by the manufacturer (FoxP3 Buffer System, BD).

### RNA preparation and expression analysis by Custom TaqMan® Low Density Array.

Total RNA was isolated from expanded cord blood Thelper and nTreg cells that were rested for approximately 60 hours following an 8 day expansion protocol prior to either vehicle (DMSO) or Ionomycin treatment (2 hours). Total RNA, including small molecular weight RNA, was isolated using QIAshredder and a miRNeasy mini kit (QIAGEN). Total RNA (2µg) was converted to cDNA using a High Capacity cDNA Transcription Kit (Applied Biosysytems). Each cDNA synthesis reaction was combined with TaqMan® Universal PCR master mix and loaded equally into 4 sample fill-reservoirs of a Custom TaqMan® Low Density Array (Format 96b). Amplification and data acquisition was carried out on a &900HT Real-Time PCR System (Applied Biosystems). Donor matched control or stimulated Thelper and nTreg samples were loaded on the same array. Relative quantitation (RQ) of targets were performed using the comparative Ct (ΔΔCT) method using RQ manager (SDSv2.3 software, Applied Biosystems). The Custom TaqMan® Low Density Array was built using validated TaqMan® gene expression assays.

### In Vitro Production of Inducible T Regulatory (iTreg) Cells

The *in vitro* conversion of cord blood CD4⁺CD25⁻ cells to iTreg cells was carried by expanding cells in the presence of 5ng/ml of recombinant human TGFβ-1 (transforming growth factor beta-1, R&D Systems) essentially as described in Tran *et al.,* (2007) with the following modifications: Cord blood CD4⁺CD25⁻ (Thelper) cells were isolated from mononuclear cells (PBMC) using a Dynabeads Regulatory CD4⁺CD25⁺ T cell kit (Invitrogen) and expanded in complete X-Vivo 15 (Lonza) media (X-Vivo15 supplemented with 20mM HEPES-, pH 7.4, 5% heat inactivated pooled human serum and 2mM L-glutamine) and 500U/ml recombinant human interleukin-2 (rhIL2; R&D Systems) in the presence of Dynabeads CD3/CD28 T cell expander beads (bead to cell ratio of 3:1, Invitrogen). Cells were expanded for a total of eight days before beads were removed and the cells rested in complete X-Vivo-15 media supplemented with 100U/ml IL2. Control and TGFβ-1 treated cells were then analyzed for FoxP3 and surface markers.

### Example 2 - Isolation and Validation of Human Cord Blood Treg Cells

Following a single round of *ex vivo* expansion by cross-linking CD3 and CD28 using anti CD3 /CD28 beads, 100-200 fold expansion of the cord blood Treg cells was routinely obtained. These cells maintained a Treg phenotype upon expansion, with between 80- 90% of the expanded cells staining CD4⁺, CD25^{hi}, CD127^{-/dim} and FoxP3 positive (Figure 1A). Post expansion the cells retained function as they were able to robustly suppress the proliferation of CD4⁺CD25⁻ cells *in vitro* in an unmatched donor mixed Leukocyte suppression assay (Figure 1B). Expanded CD4⁺CD25⁺ and CD4⁺CD25⁻ cells were used or expression profiling experiments. In the case of the CD4⁺CD25⁻ cells, upregulation of CD25 was observed after expansion as expected after chronic TCR crosslinking, but importantly, these cells were not FoxP3 positive (Figure 1A)

### Example 3 - Human Treg Gene Expression Analysis

Analysis of differential gene expression in Treg vs Thelper both resting and stimulated revealed 1704 genes that were significantly differentially expressed based on the inclusion criteria used (pvalue and log fold change). The proportion of these that are up vs down regulated is 0.2:0.8. The behavior of these genes in the differential factorial analysis of the dataset is represented as a heat map in Figure 2. Genes were clustered together into the 7 groups (Table 6) based on clustered expression patterns, and the Treg specific gene signature is captured in T1-3, whereas the Treg and activation specific behavior is captured in T4-6. A signature of genes that are significantly differentially expressed but in response to stimulation of either Treg or Thelpers (T cell activation) is captured in A1.

**Table 7: Group definitions for the heatmap (Figure 2)**

| **Group** | **Inclusion Criteria:** | **Differentially expressed Genes** |
|---|---|---|
| **T1** | Effects in CD25+ vs. CD25- for both Stimulated & Resting | 388 |
| **T2** | Effects in CD25+ vs. CD25- for Resting only | 164 |
| **T3** | Effects in CD25+ vs. CD25- for Stimulated only No activation effects | 334 |
| **T4** | Effects in CD25+ vs. CD25- for Stimulated only Activation effects in both CD25+ and CD25- | 177 |
| **T5** | Effects in CD25+ vs. CD25- for Stimulated only Activation effects in CD25- only | 235 |
| **T6** | Effects in CD25+ vs. CD25- for Stimulated only Activation effects in CD25+ only | 406 |
| **Total** | | 1704 |
| **A1** | Stimulation effect in both CD25+ and CD25- | 649 |

### Example 4 - Cell Surface Molecule Identification

The list of genes from all T groups, groups T1 & T2 combined, and T3 to T6 combined were analyzed for the differential expression of cell surface expressed proteins which could be targets for therapeutic modulation of function or used as biomarkers. Exemplary cell surface molecules that were differentially expressed are described in Table 8 (and Tables 1-6). These results were confirmed for six biomarkers using qPCR, the results of which are shown in Figure 3.

| **Table 8** | **Differenitally** | **expressed genes** | | | | |
|---|---|---|---|---|---|---|
| **Entrez ID** | **Gene Name** | **Gene Description** | **TregVsThelp (Stim)** | **TregVsThelp (Resting)** | **Treg Activation** | **Thelp Activation** |
| 2615 | LRRC32 | leucine rich repeat containing 32 | 3.771 | 2.290 | 3.116 | 1.388 |
| 301 | ANXA1 | annexin A1 | -3.077 | -2.875 | 1.032 | 1.242 |
| 10800 | CYSLTR1 | cysteinyl leukotriene receptor 1 | -2.684 | -3.149 | 0.502 | 0.000 |
| 79895 | ATP884 | ATPase, class I, type 8B, member 4 | -3.020 | -2.716 | 0.000 | 0.000 |
| 3575 | IL7R | interleukin 7 receptor | -2.642 | -3.056 | -0.869 | -1.062 |
| 959 | CD40LG | CD40 ligand | -2.646 | -2.991 | 2.705 | 2.361 |
| 255231 | MCOLN2 | mucolipin 2 | -2.942 | -2.407 | 0.000 | 0.577 |
| 1493 | CTLA4 | cytotoxic T-lymphocyte-associated protein 4 | 2.112 | 2.755 | 1.126 | 1.714 |
| 2153 | F5 | coagulation factor V (proaccelerin, labile factor) | 2.269 | 2.401 | 0.897 | 1.000 |
| 225 | ABCD2 | ATP-binding cassette, sub-family D (ALD), member 2 | -2.306 | -2.228 | -0.891 | -0.987 |
| 3554 | IL1R1 | interleukin 1 receptor, type I | 2.724 | 1.708 | 1.688 | 0.000 |
| 3673 | ITGA22 | integrin, alpha 2 (CD49B, alpha 2 subunit of VLA-2 receptor) | -2.265 | -1.903 | 1.637 | 1.898 |
| 342372 | PKD1L3 | polycystic kidney disease 1-like 3 | 1.839 | 2.107 | 0.000 | 0.000 |
| 22914 | KLRK1 | killer cell lectin-like receptor subfamily K, member 1 | -1.716 | -2.091 | 0.000 | 0.000 |
| 201633 | TIGIT | T cell immunoreceptor with Ig and ITIM domains | 3.798 | 0.000 | 2.441 | 1.072 |
| 973 | CD79A | CD79a molecule, immunoglobulin-associated alpha | 1.797 | 1.914 | 0.000 | 0.000 |
| 11314 | CD300A | CD300a molecule | -1.437 | -2.180 | 0.000 | 0.000 |
| 3672 | ITGA1 | integrin, alpha 1 | -1.674 | -1.930 | 0.000 | 0.000 |
| 11001 | SLC27A2 | solute carrier family 27 (fatty acid transporter), member 2 | -1.993 | -1.586 | 1.593 | 1.646 |
| 4818 | NKG7 | natural killer cell group 7 sequence | -1.660 | -1.885 | 0.000 | 0.000 |
| 5787 | PTPRB | protein tyrosine phosphatase, receptor type, B | 1.686 | 1.856 | 0.000 | 0.000 |
| 29126 | CD274 | CD274 molecule | -1.676 | -1.802 | 0.000 | 0.000 |
| 55784 | MCTP2 | multiple C2 domains, transmembrane 2 | -1.966 | -1.502 | -0.612 | 0.000 |
| 4907 | NT5E | 5'-nucleotidase, ecto (CD73) | -1.778 | -1.609 | 0.993 | 0.000 |
| 84002 | B3GNT5 | UDP-GlcNAc:betaGal beta-1,3-N-acetylglucosaminyltransferase 5 | -3.335 | 0.000 | 1.891 | 4.471 |
| 160365 | CLECL1 | C-type lectin-like 1 | -1.841 | -1.468 | 0.000 | 0.000 |
| 3460 | IFNGR2 | interferon gamma receptor 2 (interferon gamma transducer 1) | 1.685 | 1.622 | 0.000 | 0.000 |
| 23365 | ARHGEF12 | Rho guanine nucleotide exchange factor (GEF) 12 | -1.764 | -1.445 | 0.000 | 0.000 |
| 11031 | RAB31 | RAB31, member RAS oncogene family | 1.537 | 1.667 | 0.000 | 0.000 |
| 928 | CD9 | CD9 molecule | -1.772 | -1.420 | 0.000 | 0.000 |
| 10371 | SEMA3A | sema domain, immunoglobulin domain (Ig), short basic domain, secreted, (semaphorin) 3A | -1.443 | -1.672 | 0.000 | 0.523 |
| 3570 | IL6R | interleukin 6 receptor | 1.315 | 1.796 | -0.699 | 0.000 |
| 54947 | LPCAT2 | lysophosphatidylcholine acyltransferase 2 | -1.437 | -1.664 | 0.000 | 0.000 |
| 10252 | SPRY1 | sprouty homolog 1, antagonist of FGF signaling (Drosophila) | -1.823 | -1.270 | 3.650 | 3.738 |
| 8828 | NRP2 | neuropilin 2 | -1.424 | -1.588 | 0.000 | 0.000 |
| 5924 | RASGRF2 | Ras protein-specific guanine nucleotide-releasing factor 2 | -1.427 | -1.566 | 0.000 | 0.000 |
| 219790 | RTKN2 | rhotekin 2 | 1.449 | 1.538 | 0.000 | 0.000 |
| 221476 | PI16 | peptidase inhibitor 16 | 1.334 | 1.633 | 0.000 | 0.000 |
| 1880 | GPR183 | G protein-coupled receptor 183 | -1.872 | -1.089 | 0.656 | 1.743 |
| 10000 | AKT3 | v-akt murine thymoma viral oncogene homolog 3 (protein kinase B, gamma) | -1.700 | -1.208 | 0.000 | 0.000 |
| 54674 | LRRN3 | leucine rich repeat neuronal 3 | -1.279 | -1.609 | 0.000 | 0.000 |
| 57476 | GRAMD1B | GRAM domain containing 1B | -1.567 | -1.310 | 0.000 | 0.000 |
| 6809 | STX3 | syntaxin 3 | -1.319 | -1.555 | -0.506 | -0.542 |
| 945 | CD33 | CD33 molecule | 1.350 | 1.505 | 0.000 | 0.000 |
| 79901 | CYBRD1 | cytochrome b reductase 1 | -1.172 | -1.671 | 0.000 | 0.000 |
| 92 | ACVR2A | activin A receptor, type IIA | -1.377 | -1.433 | 0.000 | 0.000 |
| 57493 | HEG1 | HEG homolog 1 (zebrafish) | -1.721 | -1.071 | 0.589 | 1.316 |
| 3717 | JAK2 | Janus kinase 2 (a protein tyrosine kinase) | -1.642 | -1.139 | 0.000 | 0.000 |
| 4162 | MCAM | melanoma cell adhesion molecule | 1.489 | 1.263 | 0.000 | 0.000 |
| 114801 | TMEM200A | transmembrane protein 200A | -1.086 | -1.638 | 0.000 | 0.000 |
| 4067 | LYN | v-yes-1 Yamaguchi sarcoma viral related oncogene homolog | -1.350 | -1.349 | 0.000 | 0.000 |
| 6272 | SORT1 | sortilin 1 | -1.527 | -1.126 | 0.000 | 0.000 |
| 55512 | SMPD3 | sphingomyelin phosphodiesterase 3, neutral membrane (neutral sphingomyelinase II) | 1.317 | 1.309 | -0.554 | 0.000 |
| 131450 | CD200R1 | CD200 receptor 1 | -1.233 | -1.383 | 0.000 | 0.000 |
| 55283 | MCOLN3 | mucolipin 3 | -1.387 | -1.204 | 0.000 | 0.000 |
| 3684 | ITGAM | integrin, alpha M (complement component 3 receptor 3 subunit) | 1.197 | 1.392 | 0.000 | 0.000 |
| 356 | FASLG | Fas ligand (TNF superfamily, member 6) | -2.588 | 0.000 | 1.187 | 3.273 |
| 10100 | TSPAN2 | tetraspanin 2 | -1.465 | -1.111 | 0.000 | 0.787 |
| 255324 | EPGN | epithelial mitogen homolog (mouse) | -1.335 | -1.238 | 0.000 | 0.000 |
| 360 | AQP3 | aquaporin 3 (Gill blood group) | 1.078 | 1.482 | 0.000 | 0.000 |
| 338596 | ST8SIA6 | ST8 alpha-N-acetyl-neuraminide alpha-2,8-sialyltransferase 6 | 1.079 | 1.471 | -0.541 | 0.000 |
| 8611 | PPAP2A | phosphatidic acid phosphatase type 2A | -1.309 | -1.234 | 0.000 | 0.000 |
| 10018 | BCL2L11 | BCL2-like 11 (apoptosis facilitator) | -0.928 | -1.567 | 1.798 | 1.487 |
| 3958 | LGALS3 | Lectin, galactoside-binding, soluble, 3 | 1.041 | 1.427 | 0.000 | 0.000 |
| 57091 | CASS4 | Cas scaffolding protein family member 4 | -1.040 | -1.426 | 0.000 | 0.000 |
| 8600 | TNFSF11 | Tumor necrosis factor (ligand) superfamily, member 11 | -1.676 | -0.782 | 0.621 | 1.589 |
| 3655 | ITGA6 | integrin, alpha 6 | 0.950 | 1.498 | -0.841 | 0.000 |
| 7133 | TNFRSF1B | Tumor necrosis factor receptor superfamily, member 1B | 1.441 | 0.977 | 0.754 | 0.000 |
| 535 | ATP6V0A1 | ATPase, H+transporting, lysosomal V0 subunit al | 1.215 | 1.155 | 0.000 | 0.000 |
| 51599 | LSR | lipolysis stimulated lipoprotein receptor | 1.440 | 0.923 | 0.000 | 0.000 |
| 84830 | C6orf105 | chromosome 6 open reading frame 105 | 1.069 | 1.268 | 0.000 | 0.000 |
| 634 | CEACAM1 | carcinoembryonic antigen-related cell adhesion molecule 1 (biliary glycoprotein) | 1.188 | 1.147 | 0.000 | 0.000 |
| 23555 | TSPAN15 | tetraspanin 15 | 1.081 | 1.247 | 0.000 | 0.000 |
| 8743 | TNFSF10 | Tumor necrosis factor (ligand) superfamily, member 10 | -1.224 | -1.099 | -1.120 | 0.000 |
| 23208 | SYT11 | synaptotagmin XI | -1.109 | -1.189 | 0.000 | 0.000 |
| 115727 | RASGRP4 | RAS guanyl releasing protein 4 | 1.373 | 0.915 | 0.956 | 0.000 |
| 55711 | FAR2 | fatty acyl CoA reductase 2 | -1.292 | -0.992 | 0.000 | 0.000 |
| 120425 | AMICA1 | adhesion molecule, interacts with CXADR antigen 1 | -0.974 | -1.283 | 0.000 | 0.000 |
| 4345 | CD200 | CD200molecule | -2.238 | 0.000 | 4.146 | 6.282 |
| 6976 | TRGV3 | Tcell receptor gamma variable 3 | -1.131 | -1.104 | -0.535 | 0.000 |
| 5733 | PTGER3 | prostaglandin E receptor 3 (subtype EP3) | -0.793 | -1.387 | 0.000 | 0.000 |
| 10207 | INADL | InaD-like (Drosophila) | -1.064 | -1.096 | 0.000 | 0.000 |
| 57111 | RAB25 | RAB25, member RAS oncogene family | 1.093 | 1.065 | 0.000 | 0.000 |
| 157506 | RDH10 | retinol dehydrogenase 10 (all-trans) | 0.000 | 2.087 | 1.217 | 2.724 |
| 1524 | CX3CR1 | chemokine (C-X3-C motif) receptor 1 | -0.942 | -1.132 | 0.808 | 0.000 |
| 5796 | PTPRK | protein tyrosine phosphatase, receptor type, K | -0.956 | -1.103 | 0.000 | 0.000 |
| 84636 | GPR174 | G protein-coupled receptor 174 | -0.692 | -1.347 | 0.579 | 0.000 |
| 56654 | NPDC1 | neural proliferation, differentiation and control, 1 | 1.067 | 0,970 | 0.000 | 0.000 |
| 3732 | CD82 | CD82 molecule | -1.042 | -0.993 | 0.000 | 0.000 |
| 5359 | PLSCR1 | phospholipid scramblase 1 | -1.443 | -0.583 | 0.000 | 0.585 |
| 1E+08 | LOC100132000 | Transmembrane protein ENSP00000382582 | -1.169 | -0.841 | 0.000 | 0.000 |
| 285704 | RGMB | RGM domain family, member B | 1.040 | 0.961 | 0.000 | 0.000 |
| 137835 | TMEM71 | transmembrane protein 71 | -1.168 | -0.828 | 0.000 | 0.000 |
| 84868 | HAVCR2 | hepatitis A virus cellular receptor 2 | -0.749 | -1.222 | 0.000 | 0.000 |
| 753 | C18orf1 | chromosome 18 open reading frame 1 | 1.270 | 0.693 | 0.000 | 0.000 |
| 55974 | RAG1AP1 | recombination activating gene 1 activating protein 1 | 0.781 | 1.158 | -0.738 | 0.000 |
| 2530 | FUT8 | fucosyltransferase 8 (alpha (1,6) fucosyltransferase) | -1.139 | -0.798 | 0.000 | 0.000 |
| 3382 | ICA1 | islet cell autoantigen 1, 69kDa | 0.849 | 1.087 | 0.000 | 0.000 |
| 253782 | LASS6 | LAG1 homolog, ceramide synthase 6 | -1.098 | -0.822 | 0.000 | 0.000 |
| 7097 | TLR2 | toll-like receptor 2 | -0.994 | -0.903 | 0.000 | 0.000 |
| 2838 | GPR15 | G protein-coupled receptor 15 | -0.541 | -1.330 | 0.000 | 0.000 |
| 79815 | NPAL2 | NIPA-like domain containing 2 | 0.897 | 0.973 | -0.580 | 0.000 |
| 6403 | SELP | selectin P (granule membrane protein 140kDa, antigen CD62) | 0.865 | 1.000 | 0.000 | 0.000 |
| 1235 | CCR6 | chemokine (C-C motif) receptor 6 | 0.793 | 1.069 | -0.806 | 0.000 |
| 8573 | CASK | calcium/calmodulin-dependent serine protein kinase (MAGUK family) | 0.863 | 0.984 | 0.000 | 0.000 |
| 11010 | GLIPR1 | GLI pathogenesis-related 1 | -0.778 | -1.058 | 0,000 | 0.000 |
| 166752 | FREM3 | FRAS1 related extracellular matrix 3 | 0.829 | 0.992 | 0.000 | 0.000 |
| 9455 | HOMER2 | homer homolog 2 (Drosophila) | -0.809 | -1.005 | 0.000 | 0.000 |
| 2047 | EPHB1 | EPH receptor B1 | 0.990 | 0.818 | 0.000 | 0.000 |
| 56253 | CRTAM | cytotoxic and regulatory T cell molecule | -1.800 | 0.000 | 0.809 | 2.400 |
| 9976 | CLEC2B | C-type lectin domain family 2, member 8 | -0.954 | -0.832 | 0.000 | 0.000 |
| 51301 | GCNT4 | glucosaminyl (N-acetyl) transferase 4, core 2 (beta-1,6-N-acetylglucosaminyltransferase) | 0.000 | 1.779 | -0.790 | 0.000 |
| 5141 | PDE4A | phosphodiesterase 4A, cAMP-specific (phosphodiesterase E2 dunce homolog, Drosophila) | -0.588 | -1.180 | 0.000 | -0.713 |
| 151888 | BTLA | B and T lymphocyte associated | -1.766 | 0.000 | 1.957 | 3.580 |
| 10333 | TLR6 | toll-like receptor 6 | 0.651 | 1.106 | -0.820 | 0.000 |
| 5168 | ENPP2 | ectonucleotide pyrophosphatase/phosphodiesterase 2 | -1.754 | 0.000 | 1.695 | 2.169 |
| 23654 | PLXNB2 | Plexin 82 | 0.923 | 0.816 | 0.000 | 0.000 |
| 26762 | HAVCR1 | hepatitis A virus cellular receptor 1 | -0.833 | -0.895 | 0.000 | 0.000 |
| 5054 | SERPINE1 | serpin peptidase inhibitor, clade E (nexin, plasminogen activator inhibitor type 1), member 1 | -1.725 | 0.000 | 0.633 | 2.354 |
| 57205 | ATP10D | ATPase, class V, type 10D | -0.883 | -0.838 | 0.000 | 0.000 |
| 3559 | IL2RA | interleukin 2 receptor, alpha | 0.611 | 1.102 | 0.552 | 0.970 |
| 8870 | IER3 | immediate early response 3 | -1.696 | 0.000 | 0.537 | 1.439 |
| 4033 | LRMP | lymphoid-restricted membrane protein | -0.939 | -0.750 | -0.788 | 0.000 |
| 4040 | LRP6 | low density lipoprotein receptor-related protein 6 | 0.607 | 1.078 | 0.000 | 0.000 |
| 1363 | CPE | carboxypeptidase E | 1.023 | 0.662 | 0.000 | 0.000 |
| 30061 | SLC40A1 | Solute carrier family 40 (iron-regulated transporter), member 1 | -0.835 | -0.848 | 0.000 | -0.509 |
| 8792 | TNFRSF11A | Tumor necrosis factor receptor superfamily, member 11a, NFKB activator | 0.969 | 0.694 | 0.000 | 0.000 |
| 57705 | WDFY4 | WDFY family member 4 | 0.875 | 0.778 | 0.000 | 0.000 |
| 926 | CD98 | CD8b molecule | 0.698 | 0.949 | 0.000 | 0.000 |
| 5734 | PTGER4 | prostaglandin E receptor 4 (subtype EP4) | -0.579 | -1.038 | 1.575 | 1.372 |
| 114836 | SLAMF6 | SLAM family member 6 | -1.615 | 0.000 | -0.630 | 0.652 |
| 2207 | FCER1G | Fc fragment of IgE, high affinity I, receptor for; gamma polypeptide | 1.604 | 0.000 | 0.000 | 0.000 |
| 340547 | VSIG1 | V-set and immunoglobulin domain containing 1 | 0.638 | 0.957 | -0.710 | 0.000 |
| 6812 | STXBP1 | syntaxin binding protein 1 | -0.940 | -0.624 | 0.000 | 0.000 |
| 64065 | PERP | PERP, TP53 apoptosis effector | 0.582 | 0.973 | 0.000 | 0.000 |
| 56913 | C1GALT1 | core 1 synthase, glycoprotein-N-acetylgalactosamine 3-beta-galactosyltransferase, 1 | -0.911 | -0.644 | 0.000 | 0.000 |
| 347902 | AMIG02 | adhesion molecule with Ig-like domain 2 | -0.936 | -0.598 | 1.034 | 1.425 |
| 29097 | CNIH4 | cornichon homolog 4 (Drosophila) | -0.994 | -0.527 | 0.000 | 0.000 |
| 9743 | RICS | Rho GTPase-activating protein | -0.885 | -0.624 | 0.000 | 0.000 |
| 25945 | PVRL3 | poliovirus receptor-related 3 | -0.964 | -0.543 | 0.627 | 1.165 |
| 8477 | GPR65 | G protein-coupled receptor 65 | -0.698 | -0.782 | 0.000 | 0.000 |
| 10855 | HPSE | heparanase | 0.610 | 0.854 | 0.000 | 0.613 |
| 55061 | SUSD4 | sushi domain containing 4 | 0.000 | -1.454 | 0.000 | 0.000 |
| 5743 | PTGS2 | prostaglandin-endoperoxide synthase 2 (prostaglandin G/H synthase and cyclooxygenase) | -1.453 | 0.000 | 4.352 | 5.552 |
| 54843 | SYTL2 | synaptotagmin-like 2 | -1.453 | 0.000 | 0.000 | 0.000 |
| 55103 | RALGPS2 | Ral GEF with PH domain and SH3 binding motif 2 | -0.835 | -0.594 | 0.000 | 0.000 |
| 8807 | IL18RAP | interleukin 18 receptor accessory protein | -1.429 | 0.000 | 1.534 | 1.950 |
| 92691 | TMEM169 | transmembrane protein 169 | 0.682 | 0.744 | -0.904 | -0.877 |
| 54947 | LPCAT2 | lysophosphatidylcholine acyltransferase 2 | -1.437 | -1.664 | 0.000 | 0.000 |

### Example 6 - Low Density Array Analysis of Gene Expression

Figures 5A to 5D show results of some low density array experiments to assess the expression of various biomarkers by stimulated nTreg cells compared to stimulated Thelper cells (Figures 5A and 5B) or resting nTreg cells compared to resting Thelper cells (Figure 5C and 5D).

A summary of the data obtained high density arrays and additional low density arrays is shown in Table 9.

**Table 9: Summary of results from array screening and low density array experiments**

| **Gene Name** | **Log Fold Change** | | **Log Fold Change** | | **Low Density Array Value** | |
|---|---|---|---|---|---|---|
| | **Treg vs Th(stim)** | **Treg vs Th(rest)** | **Treg Act.** | **Th Act.** | **stim** | **rest** |
| Upregulated genes | | | | | | |
| PI16 | 1.33 | 1.63 | 0 | 0 | 72.55 | 62.82 |
| CD121 | 2.72 | 1.71 | 1.69 | 0 | 128.51 | 401.83 |
| PKD1L3 | 1.84 | 2.11 | 0 | 0 | 23.26 | 15.06 |
| PTPRB | 1.69 | 1.86 | 0 | 0 | 26.47 | 18.49 |
| CD 146 | 1.49 | 1.26 | 0 | 0 | 7.06 | 6.57 |
| IL6R | 1.32 | 1.80 | -0.70 | 0 | 7.53 | 8.09 |
| C18orf1 | 1.27 | 0.69 | 0 | 0 | 2.04 | 1.59 |
| AQP3 | 1.08 | 1.48 | 0 | 0 | 2.36 | 2.26 |
| RGMB | 1.04 | 0.96 | 0 | 0 | 98.29 | 76.93 |
| CD49f | 0.95 | 1.50 | -0.84 | 0 | 3.87 | 4.08 |
| PERP | 0.58 | 0.97 | 0 | 0 | 3.56 | 4.28 |
| LSR | 1.44 | 0.92 | 0 | 0 | 17.66 | 10.36 |
| TIGIT | 3.80 | 0 | 2.44 | 1.07 | 30.23 | 29.82 |
| WDFY4 | 0.88 | 0.78 | 0 | 0 | 109.62 | 24.84 |
| ATP6V0A1 | 1.22 | 1.16 | 0 | 0 | 3.32 | 3.15 |
| ST8SIA6 | 1.08 | 1.47 | -0.54 | 0 | 7.05 | 4.30 |
| C6orf105 | 1.07 | 1.27 | 0 | 0 | 3.71 | 9.52 |
| NPAL2 | 0.90 | 0.97 | -0.58 | 0 | 2.32 | 2.26 |
| TMEM169 | 0.68 | 0.74 | -0.90 | -0.88 | 1.65 | 1.79 |
| FREM3 | 0.83 | 0.99 | 0 | 0 | | |
| EPH1 | 0.99 | 0.82 | 0 | 0 | 41.78 | 54.08 |
| FCER1G | 1.60 | 0 | 0 | 0 | 4.56 | 4.10 |
| TSPAN15 | 1.08 | 1.25 | 0 | 0 | 4.74 | 4.51 |
| PLXNB2 | 0.92 | 0.82 | 0 | 0 | 2.26 | 2.25 |
| VSIG1 | 0.64 | 0.96 | -0.71 | 0 | 1.67 | 1.62 |
| NPDC1 | 1.07 | 0.97 | 0 | 0 | 4.84 | 4.72 |
| CD79A | 1.80 | 1.91 | 0 | 0 | 15.40 | 13.95 |
| IFNgR2 | 1.69 | 1.62 | 0 | 0 | 8.54 | 7.92 |
| CD33 | 1.35 | 1.51 | 0 | 0 | 14.78 | 13.74 |
| CD11B | 1.20 | 1.39 | 0 | 0 | 3.81 | 3.27 |
| CD66 | 1.19 | 1.15 | 0 | 0 | 4.62 | 4.66 |
| CD79B | 0.74 | 0.81 | -0.70 | 0 | 2.01 | 1.94 |
| CD8B | 0.70 | 0.95 | 0 | 0 | 2.31 | 1.82 |
| TLR6 | 0.65 | 1.11 | -0.82 | 0 | 3.81 | 3.07 |
| LRP6 | 0.61 | 1.08 | 0 | 0 | 2.64 | 2.51 |
| | | | | | | |
| SMPD3 | 1.32 | 1.31 | -0.55 | 0 | 4.32 | 4.50 |
| | | | | | | |
| F5 | 2.27 | 2.40 | 0.90 | 1.00 | | |
| RAB31 | 1.54 | 1.67 | 0 | 0 | | |
| RTKN2 | 1.45 | 1.54 | 0 | 0 | | |
| RASGRP4 | 1.37 | 0.92 | 0.96 | 0 | | |
| RAB25 | 1.09 | 1.07 | 0 | 0 | | |
| CPE | 1.02 | 0.66 | 0 | 0 | | |
| CASK | 0.86 | 0.98 | 0 | 0 | | |
| ICA1 | 0.85 | 1.09 | 0 | 0 | | |
| TJP3 | 0.79 | 0.75 | 0 | 0 | | |
| RAG1AP1 | 0.78 | 1.16 | -0.74 | 0 | | |
| HPSE | 0.61 | 0.85 | 0 | 0.61 | | |

| Downregulated genes | | | | | | |
|---|---|---|---|---|---|---|
| ATP8B4 | -3.020 | -2.716 | 0.000 | 0.000 | -4.86 | -5.56 |
| MCOLN2 | -2.94 | -2.41 | 0 | 0.58 | -5.02 | -4.78 |
| CYSLTR1 | -2.684 | -3.149 | 0.502 | 0.000 | -6.73 | -7.35 |
| ITGA2 | -2.265 | -1.903 | 1.637 | 1.898 | -1.77 | -7.58 |
| GPR183 | -1.872 | -1.089 | 0.656 | 1.743 | -2.31 | -1.59 |
| CLECL1 | -1.84 | -1.47 | 0 | 0 | -1.78 | -2.27 |
| CRTAM | -1.800 | 0.000 | 0.809 | 2.400 | -3.65 | -4.13 |
| CD9 | -1.772 | -1.420 | 0.000 | 0.000 | -2.01 | -2.23 |
| ENPP2 | -1.754 | 0.000 | 1.695 | 2.169 | -7.27 | -13.33 |
| HEG1 | -1.72 | -1.07 | 0.59 | 1.32 | -2.51 | -1.48 |
| IER3 | -1.70 | 0 | 0.54 | 1.44 | -1.47 | -1.14 |
| CD274 | -1.676 | -1.802 | 0.000 | 0.000 | -1.74 | -3.31 |
| ITGA1 | -1.674 | -1.930 | 0.000 | 0.000 | -7.55 | -7.58 |
| NKG7 | -1.66 | -1.89 | 0 | 0 | -1.59 | -1.77 |
| SLAMF6 | -1.615 | 0.000 | -0.630 | 0.652 | -1.43 | -1.02 |
| SORT1 | -1.527 | -1.126 | 0.000 | 0.000 | -3.13 | -3.27 |
| TSPAN2 | -1.465 | -1.111 | 0.000 | 0.787 | -2.65 | -3.83 |
| LPCAT2 | -1.44 | -1.66 | 0 | 0 | -1.79 | -1.82 |
| PLSCR1 | -1.443 | -0.583 | 0.000 | 0.585 | -1.27 | -1.10 |
| SEMA3A | -1.443 | -1.672 | 0.000 | 0.523 | -17.53 | -40.00 |
| CD300A | -1.437 | -2.180 | 0.000 | 0.000 | -3.07 | -3.44 |
| NRP2 | -1.424 | -1.588 | 0.000 | 0.000 | -4.60 | -3.83 |
| MCOLN3 | -1.387 | -1.204 | 0.000 | 0.000 | -2.51 | -3.09 |
| ACVR2A | -1.377 | -1.433 | 0.000 | 0.000 | -3.43 | -3.68 |
| EPGN | -1.335 | -1.238 | 0.000 | 0.000 | -5.60 | -2.35 |
| STX3 | -1.32 | -1.56 | -0.51 | -0.54 | -1.90 | -2.07 |
| LRRN3 | -1.28 | -1.61 | 0 | 0 | -3.96 | -6.13 |
| CD200R1 | -1.233 | -1.383 | 0.000 | 0.000 | -2.57 | -2.29 |
| CYBRD1 | -1.172 | -1.671 | 0.000 | 0.000 | -2.27 | -3.04 |
| TMEM71 | -1.17 | -0.83 | 0 | 0 | -1.47 | -1.51 |
| LASS6 | -1.098 | -0.822 | 0.000 | 0.000 | -1.74 | -1.78 |
| TMEM200A | -1.086 | -1.64 | 0 | 0 | -4.87 | -4.98 |
| CD82 | -1.042 | -0.993 | 0.000 | 0.000 | -1.34 | -1.55 |
| CNIH4 | -0.994 | -0.527 | 0.000 | 0.000 | -1.02 | -1.11 |
| PVRL3 | -0.964 | -0.543 | 0.627 | 1.165 | -1.73 | -1.90 |
| PTPRK | -0.956 | -1.103 | 0.000 | 0.000 | -8.25 | -8.93 |
| CLEC2B | -0.954 | -0.832 | 0.000 | 0.000 | -1.25 | -1.53 |
| LRMP | -0.94 | -0.75 | -0.79 | 0 | -1.19 | -1.19 |
| AMIGO2 | -0.936 | -0.598 | 1.034 | 1.425 | -1.83 | -1.23 |
| C1GALT1 | -0.911 | -0.644 | 0.000 | 0.000 | -1.14 | -1.68 |
| ATP10D | -0.88 | -0.84 | 0 | 0 | -1.36 | -1.40 |
| RALGPS2 | -0.835 | -0.594 | 0.000 | 0.000 | -1.51 | -2.00 |
| PTGER3 | -0.793 | -1.387 | 0.000 | 0.000 | -3.28 | -4.22 |
| GPR174 | -0.69 | -1.35 | 0.58 | 0.00 | -2.01 | -2.64 |
| SUSD4 | 0.000 | -1.454 | 0.000 | 0.000 | -21.49 | -24.39 |
| | | | | | | |
| IL18RAP | -1.429 | 0.000 | 1.534 | 1.950 | -1.22 | |
| PPAP2A | -1.309 | -1.234 | 0.000 | 0.000 | | |
| AMICA1 | -0.974 | -1.283 | 0.000 | 0.000 | | |
| GLIPR1 | -0.778 | -1.058 | 0.000 | 0.000 | | |
| GPR65 | -0.698 | -0.782 | 0.000 | 0.000 | | |
| GPR15 | -0.541 | -1.330 | 0.000 | 0.000 | | |

### Example 7 - Validation of Biomarkers by Flow Cytometry

Biomarkers identified by microarray were screened with commercially available antibodies (where possible monoclonal antibodies (mAb) were chosen due to their increased specificity compared to polyclonal antibodies). Due to the broader specificity of polyclonal antibodies (pAb) and the relatively high background of rabbit and goat pAb, not all targets could be validated by flow cytometry. The targets for which the antibody reagents were robust and the backgrounds low are summarized in the tables below.

Screening Strategy:
1) Targets are screened for surface expression on T lymphocytes (test antibody/CD3).
2) If positive, targets are screened for Treg staining (test antibody/CD4+/CD25++/CD 127-).
3) If targets pass 2) correlation with FoxP3 is analyzed (test antibody/CD4+/CD25++/FoxP3).

Results of these screens are included in Tables 10-12.

**Table 10: Results of Stage 1 of the Screening Strategy**

| **Target** | **Type of Antibody** | **Staining Pattern Observed** |
|---|---|---|
| AQP3 | pAb | ∼20% of CD3⁺ lymphocytes |
| C18orf1 | pAb | ∼30% of CD3⁺ lymphocytes |
| CD11b | mAb | distinct population; on ∼12% of CD3⁺ lymphocytes |
| CD121 | mAb | distinct population; on ∼1% of CD3⁺ lymphocytes |
| CD146 | mAb | distinct population; consistent on 2-7% of CD3⁺ cells |
| CD33 | mAb | ∼0.2% of CD3⁺ lymphocytes |
| CD49f | mAb | ∼70% of CD3⁺ lymphocytes |
| CD66 | mAb | distinct population; on ∼20-30% of CD3⁺ lymphocytes |
| CD8B | mAb | distinct population; on ∼12% of CD3⁺ lymphocytes |
| IFNgR2 | mAb | ∼0.2-2% of CD3⁺ lymphocytes |
| LRP6 | mAb | ∼1-4% of CD3⁺ lymphocytes |
| LSR | pAb | ∼50% of CD3⁺ lymphocytes |
| PERP | pAb | ∼2-6% of CD3⁺ lymphocytes |
| PI16 | mAb | distinct population; consistent on ∼10% of CD3⁺ lymphocytes |
| PKD1L3 | pAb | on ~50% of CD3⁺ lymphocytes |
| PTPRB | pAb | distinct population; on ∼15% of CD3⁺ lymphocytes |
| TLR6 | mAb | on ∼50% of CD3⁺ lymphocytes |
| TSPAN15 | pAb | on ∼0.1% of CD3⁺ lymphocytes |

**Table 11: Results of Stage 2 of the Screening Strategy**

| **Target** | **Staining Pattern Observed** |
|---|---|
| AQP3 | 0.1 % of CD4+; 1.2% of CD4+CD25++; enriched on CD4+CD25++CD127- |
| C18orf1 | 0.7% of CD4+; 0.8% of CD4+CD25++; enriched on CD4+CD25++CD127- |
| CD121 | 0.3% of CD4+; 3.8% of CD4+CD25++; enriched on CD4+CD25++CD127- |
| CD146 | 0.6% of CD4+; 1.2% of CD4+CD25++; enriched on CD4+CD25++CD127- |
| CD49f | 8% of CD4+; 8.8% of CD4+CD25++; enriched on CD4+CD25++CD127- |
| LRP6 | 0.1% of CD4+; 0.1% of CD4+CD25++ |
| LSR | 0.6% of CD4+; 1.2% of CD4+CD25++; enriched on CD4+CD25++CD127- |
| PERP | 0.3% of CD4+; 0.7% of CD4+CD25++; enriched on CD4+CD25++CD127- |
| PI16 | 5% of CD4+; 12% of CD4+CD25++; enriched on CD4+CD25++CD127- |
| PKD1L3 | 5.4% of CD4+; 4.6% of CD4+CD25++ |
| PTPRB | 0.3% of CD4+; 4% of CD4+CD25++; enriched on CD4+CD25++CD127- |

**Table 12: Results of Stage 3 of the Screening Strategy**

| **Target** | **Staining Pattern Observed** |
|---|---|
| AQP3 | CD4+CD25++AQP3+ express FoxP3 |
| C18orf1 | CD4+CD25++C18orf1+ express FoxP3 |
| CD121 | CD4+CD25++CD121+ express FoxP3 |
| CD146 | CD4+CD25++CD146+ express FoxP3 |
| CD49f | CD4+CD25++CD49f+ express FoxP3 |
| PERP | CD4+CD25++PERP+ express FoxP3 |
| PI16 | CD4+CD25++PI16+ express FoxP3 |
| PKD1L3 | CD4+CD25++PKD1L3+ show weak FoxP3 expression |
| PTPRB | CD4+CD25++PTPRB+ express FoxP3 |

All targets that are enriched on CD4+CD25++CD127- Treg also express FoxP3.

### Example 8 - Analysis of PI16 expression on Treg Cells

To identify biomarker that could be used as a surrogate for FoxP3 expression, cell surface proteins were screened to identify those that were up-regulated on Treg cells. In particular, CD25 positive adult CD4⁺ cells were screened for co-expression of PI16 and FoxP3 using a polyclonal anti-PI16 antibody (Figure 6). This antibody stained approximately 15% of lymphocytes, the majority of which were also CD4⁺ (~12%). To confirm that PI16 was expressed by Treg cells, cells were stained with antibodies against CD4, CD25 and FoxP3 in addition to the PI16 antibody. As shown in Figure 6, more than 50% of CD25^{bright} PI16⁺ cells also expressed FoxP3 and PI16 was found to have a positive correlation with FoxP3.

The expression of PI16 was also tested on TGFβ induced Treg (iTreg; Figure 7). While TGFβ substantially up-regulated CD25 and FoxP3 expression, PI16 was not substantially expressed on these transiently induced cells, suggesting that PI16 expression may discriminate natural Treg from induced Treg.

Figure 8 also shows that PI16 is expressed by cells expressing a Treg phenotype, i.e., CD4⁺CD25^{hi}CD127⁻.

Figure 9 shows that antibody against PI16 binds to a population of memory Treg cells. Briefly, PBMC were labeled with antibodies to CD4, CD45RA, CD45R0, CD25 and the polyclonal anti-PI16 antibody. Staining of CD45RA is widely used to distinguish memory cells from naïve cells with CD45RA-negative cells belonging to the memory cell subset. Thus, PI16 appears to identify a population of CD4⁺ CD25⁺ CD45RA⁻ CD45R0⁺ memory Treg cells.

PI16 was also readily detectable on the surface of resting and stimulated nTreg cells (Figure 10). In particular, on resting Treg a significant portion (85%) of the CD4⁺CD25^{bright}FoxP3⁺ cells were PI16 positive, and the majority (∼70%) of the PI16⁺ cells co-express FoxP3, with only ∼15% FoxP3⁺PI16⁻ (Figure 10 Left Hand Panel ii). Resting CD25⁻ cells were not significantly FoxP3⁺, and there are ~ 20% PI16⁺ CD25⁻ cells (Figure 10A Left Hand Panel iii) detected. Upon isolation of CD4⁺CD25⁺ cells and overnight stimulation, approximately 80% of the PI16⁺ cells co express PI16 and FoxP3, although approximately 40% of the activated FoxP3⁺ cells are PI16 negative (Figure 10 Right Hand Panel c). Importantly, stimulated CD4⁺CD25⁻ cells do not express significant amounts of PI16 or FoxP3

### Example 9 - Production of Monoclonal Antibodies

A monoclonal antibody that specifically binds to a protein set forth in Table 4 is produced using methods known in the art. Briefly, a recombinant protein or a cell expressing said protein is administered to female Balb/c mice or Xenomouse™. Non-immune serum samples are taken from all mice prior to the first immunization. Groups of mice are initially immunized by the sub-cutaneous injection. Two weeks later, the mice are boosted by injection with sub-cutaneous injection. A test bleed is taken six days after the second injection, and is screened for reactivity as described below. About fourteen days later the mice are given a final boost, and four days later they are sacrificed and the spleens collected for preparing hybridomas.

For fusions, hybridoma cells are generated following the instructions of Macardle and Bailey (2006) using about 10⁸ mouse spleen cells and about 10⁷ SP2/0 myeloma cells (ratio about 10:1).

Hybridoma cells from selected wells are screened on PBMC and/or cells expressing the protein of interest and/or recombinant protein, expanded and cloned by single-cell sorting using a FACSAria II cell sorter equipped with an ACDU (BD Biosciences). Colonies arising from the sorted cells are screened by flow cytometry again.

Hybridoma cells from selected wells are expanded and re-cloned by single-cell sorting. Colonies arising from the sorted cells are screened by flow cytometry for the expression of antibodies with similar reactivity with PBMC and/or cells expressing the protein of interest and/or recombinant protein as the parent hybridoma.

### Example 10 - Determining the Level of Treg Cells in a Biological Sample

Monoclonal antibodies essentially as described in Example 9 and/or commercially available antibodies, e.g., for sources described herein are used in the production of a two-site ELISA to determine the level of a protein expressed on Treg cells in a biological sample.

Generally this method comprises capturing Treg cells with a monoclonal antibody against one protein described herein and detecting those cells with an antibody against a different protein or lysing cells and capturing with an antibody against one epitope in a protein and detecting with an antibody against a different epitope against the same protein.

A capture antibody absorbed to a microtitre plate at about 20°C for about 16 hours. Plates are then washed and blocked.

A test sample or a control sample comprising a known amount of Treg cells or protein is contacted to the immobilized protein. A further control is cord blood derived stimulated/unstimulated sorted Treg (e.g., isolated based on expression of FoxP3 and/or CD25 and/or CD4)

The detection monoclonal antibody is conjugated to, e.g., horseradish peroxidase (HRP) using a HRP conjugation kit (e.g., Alpha Diagnostics International, Inc., San Antonio, TX, USA).

Following washing of the microtitre plates, the HRP conjugated monoclonal antibody is added to each well of the plate and incubated. Plates are then washed and ABTS (Sigma Aldrich, Sydney, Australia) is added to each well. Reactions are stopped after an appropriate time, e.g., approximately 20 minutes. Absorbance values measured at 415 nm.

The amount of absorbance detected in negative control wells (cells or protein) is subtracted from the absorbance of each other well to determine the amount of detection antibody bound.

The amount of Treg cells or protein is also assessed in normal and/or healthy subjects and/or subjects known to suffer from, e.g., rheumatoid arthritis. Samples use include, for example, buffy coat fraction In this manner, an ELISA is produced to diagnose/prognose a Treg-associated condition, e.g., rheumatoid arthritis.

### Example 11 - Isolation of Treg Cells

Monoclonal antibodies as described in Example 9 are labeled with a fluorophore using standard techniques.

Peripheral blood mononuclear cells are resuspended in PBS in an optimally pre-titered cocktail of antibodies and incubated for about 20 minutes on ice. Labeled cells are washed in excess PBS and resuspended at about 5-10 x10⁶ cells/mL and held on ice for flow cytometric analysis and sorting. Propidium iodide (PI; about 1 µg/mL), is used as a viability dye for exclusion of non-viable cells. Fluorescence activated cell sorting is performed using standard methods to isolate cells expressing a marker described herein and CD4 and CD25.

Isolated cells are cultured with soluble CD3mAb, syngeneic APCs and IL-2 or with microbeads linked to anti-CD3 and anti-CD23 antibodies and IL-2 (e.g., as described in Levine *et al.,* 1997).

### Example 12 - Mixed Lymphocyte Reaction

CD4⁺CD25⁻ T-cells and/or dendritic stimulator APC are cultured in 96 well U-bottom plates. Treg cells isolated by the method described in Example 11 are added for standard assays, or in graded numbers for titration experiments. Wells are pulsed on days 3, 5, 6, and 7 with ³H-thymidine for the last 16 hours of culture. All timepoints have multiple replicates. Results are expressed in counts per minute. Reduced cell proliferation relative to cells cultured in the absence of Treg cells indicates that the Treg cells are functional.

### Example 13 - 5,6-carboxy fluorescein diacetate succinimidyl ester (CFSE) Suppressor Assay

CD4⁺ T cells are indirectly isolated from buffy coats using RosetteSep CD4⁺ T cell Enrichment Kit (Stem Cell Technologies, France) prior to Ficoll density gradient centrifugation or the CD4+ T cell Isolation Kit (Miltenyi Biotech, Germany) after Ficoll density gradient centrifugation. Purified CD4+ T cells are then further separated into CD4⁺ CD25^{bright} and a marker as described herein and CD4⁺ CD25⁻ cell populations by means of high speed FACS sorting (FACS Aria II, BD Biosciences).

CD4⁺ CD25⁻ T cells are labeled with 0.5 µM CFSE (Invitrogen, USA) in PBS/0.5% FCS for 15mins at 37°C. Excess CFSE is quenched with equal volumes of FCS for 10 minutes and then cells are washed twice with ice cold PBS. CD4⁺ CD25⁻ CFSE labeled T cells are cultured in 96-well round bottom plates at 1x10⁴ cells per well with 1x10⁵ irradiated PBMCs in the presence of varying amounts of Treg cells popualtiosn and CD3/CD28 Dynabeads (Invitrogen) in RPMI with 10% FCS. After 4 days proliferation of the CD4⁺ CD25⁻ CFSE labeled T cells are analysed by flow cytometry. Each CFSE signal peak represents one division cycle. The ability of Treg cells to suppress cell proliferation is assessed by comparing CFSE signal peaks of CD4⁺ CD25⁻ CFSE labeled T cells with and without the presence of a regulatory cell population.

### Example 14 - Treatment of GVHD

C57B1/6 (B6; H-2^{b}), BALB/c (H-2^{d}), (B6 x DBA/2 [D2])F1 (H-2^{bxd}), and C3H (H-2^{k}) are obtained. Unless otherwise stated, experiments are performed essentially as previously described in Cohen *et al.,* 1999. Briefly, 24 h after lethal irradiation of (B6 x D2)F1 (11 Gy) and B6 (10 Gy) or C3H (9.5 Gy) mice, recipients are transplanted with cells from B6 or BALB/c donor mice, respectively. The transplants are constituted of about 5 x 10⁶ T cell-depleted bone marrow (BM) cells, and in test cases Treg cells isolated as described in Example 11. Survival of mice is then determined, with prolonged survival in mice receiving Treg cells indicating that the cells are functional.

### Example 15 - Treatment of Rheumatoid Arthritis

DBA/1 Mice are immunized with collagen type II (CII) in complete Freund's adjuvant (CFA) and scores of arthritis recorded. Purified Treg cells are isolated and cultured as described in Example 11 and injected into the mice. Levels of anti-CII antibody and cytokines are determined in the supernatant using ELISA and Bio-Plex protein array system. Detection of the onset of arthritis and/or decreased serum levels of TNF-alpha and/or IL-6indicates that the isolated Treg cells are capable of treating or preventing autoimmune arthritis.

### Example 16 - Inducing an Immune Response

Mice are administered tumor cell lines, e.g., Neuro-2A or CT26 colorectal cancer cells. Following cell administration or at the time of administration, mice are administered a monoclonal antibody as described in Example 9. A subset of mice are also administered killed cancer cells. Immune response against tumor cells is also determined using ELISA and/or ELISPOT. Survival, tumor presence, the size of tumors and/or the degree of immune response against tumors in mice administered tumor cells without antibody treatment versus mice treated with antibody alone or antibody and cell vaccine is determined. A further increase in survival or immune response and/or reducing in tumor presence or size in mice administered cells and antibodies versus antibody alone or no antibody indicates that treatment permits a stronger response to vaccine treatment.

### References

- Air, Proc. Natl. Acad. Set USA 78: 7639-7643, 1981
- Arenkov et al., Anal. Biochem. 278:123-131, 2000
- Asano et al, J. Exp. Med. 184:387-396, 1996
- Audibert et al, Nature 289: 543, 1981
- F.M. Ausubel et al., (editors), Current Protocols in Molecular Biology, Greene Pub. Associates and Wiley-Interscience (1988, including all updates until present)
- Baecher-Allan et al, J. Immunol. 167:1245-1253, 2001
- Barnes et al., Anal. Biochem.102:255, 1980
- Beachey, Adv. Exp. Med. Biol. 185:193, 1985
- Bendele J Musculoskel Neuron Interact; 1(4):377-385, 2001
- Bengtsson and Bengtsson BMC Bioinformatics 7, 96, 2006
- Bengtsson and Hossjer BMC Bioinformatics 7, 100, 2006
- Beaucage, et al., Tetrahedron Letters 22: 1859-1862, 1981
- Berkner et al., BioTechniques 6: 616, 1988
- Bolstad et al., Bioinformatics (Oxford, England) 19, 185-193, 2003
- Bresatz et al., Immunol Methods 327, 53-62, 2007
- Bernhard et al, Science 245: 301-304, 1989
- Bhattacharya-Chatterjee et al, J of Immunospecificity. 141: 1398-1403, 1986
- Bluestone et al., Nat. Rev. Immunol., 3: 253, 2000
- Boerner et al., J Immunol, 147:86-95, 1991
- Boyer et al., Blood, 103: 3428-3430, 2004
- Bradl and Linington Brain Pathol., 6:303-311, 1996
- Brennan et al, Science, 229: 81 1985
- Bresslauer et al., Proc. Natl. Acad. Sci. USA, 83: 3746-3750, 1986
- Brown (editor), Essential Molecular Biology: A Practical Approach, Volumes 1 and 2, IRL Press (1991)
- Bzik et al, Virology 155:322-333, 1986
- Cao et al., Arthritis Res. Ther., 6: R35-R46, 2004
- Caron et al., J. Exp Med. 176:1 191-1 195 1992
- Caruthers, M. H., et al., "Methods in Enzymology," Vol. 154, pp. 287-314 (1988)
- Carter et al., Bio/Technology 10: 163-167, 1992
- Chen and Okayama, Mol. Cell Biol., 7: 2745-2752, 1987
- Chen et al., Nature, 446:203-207, 2007
- Chothia and Lesk J. Mol Biol. 196:901 -917, 1987
- J.E. Coligan et al., (editors) Current Protocols in Immunology, John Wiley & Sons (including all updates until present)
- Cima, et al., Biotechnol. Bioeng. 38:145 1991
- Clackson et al, Nature, 352:624-628 (1991)
- Cohen et al., Hum. Gene Ther. 10:2701-2707, 1999
- Cole et al., Monoclonal Antibodies and Cancer Therapy, Alan R. Liss, p. 77 (1985)
- Collins et al, Proc. Natl. Acad. Sci USA 11: 7683, 1984
- Crispin et al., J. Autoimmun., 21: 273-276, 2003
- Cunningham et al., Science, 244:1081-1085 1989
- Cuello, Immunohistochemistry, 1984 John Wiley and Sons, ASIN 0471900524
- Curiel et al., Nat. Med., 10: 942-949, 2004
- Dai et al., J Bioinform Comput Biol 1, 627-645, 2004
- Davies et al., 2007 Biotechnol Bioeng 74:288-294
- De Kleer et al., J. Immunol., 172: 6435-6443, 2004
- Dejaco et al., Immunology, 117: 289-300, 2005
- Dieffenbach and Dveksler (Eds) (In: PCR Primer: A Laboratory Manual, Cold Spring Harbour Laboratories, NY, 1995)
- Devereaux et al, Nucl. Acids Res. 12, 387-395, 1984
- Edelson, The Cancer Journal 4: 62, 1998
- Egholm et al., Am. Chem. Soc., 114: 1895, 1992
- Egholm et al., Nature, 365: 566, 1993
- Emini et al, Nature 304:699, 1983
- Estin et al, J Natl. Cancer Instil 81(6): 445-446, 1989
- Fecheimer et al., , Proc. Natl Acad. Sci. USA, 84: 8463-8467,1987
- Feizi, Nature 314: 53-57, 1985
- Flotte et al, J. Biol. Chem. 268: 3781-3790, 1993
- Foon et al, Proc. Am. Soc. Clin. Oncol. 13: 294, 1994
- Fraley et al., Proc. Natl Acad. Sci. USA, 76: 3348-3352,1979
- Gait (Ed) (In: oligonucleotide Synthesis: A Practical Approach, IRL Press, Oxford, 1984)
- Gefter et al, Somatic Cell Genet. 3, 231-236, 1977
- Ghetie et al, Blood 83: 1329-1336, 1994
- Gillies et al, J. Immunol. Methods 125:191-202, 1989
- Gilligan et al, Anal Chem, 74(9): 2041 - 2047, 2002
- Glover and B.D. Hames (editors), DNA Cloning: A Practical Approach, Volumes 1-4, IRL Press (1995 and 1996)
- Goforth et al., Cancer Immunol Immunother. 2008
- Gonzales-Scarano et al, Virology 120: 42, 1982
- Goodman et al., Goodman and Gilman's The Pharmacological Basis of Therapeutics, 8th Ed., Macmillan Publishing Co., 1990
- Gopal, Mol. Cell Biol., 5: 1188-1190,1985
- Graham et al., Gen Virol. 36:59, 1977
- Graham and Van Der Eb, Virology, 52: 456-467,1973
- Gregori et al., J Immunol. 167: 1945-1953, 2001
- Gruber et al, J. Immunol., 152:5368 1994
- Guss et al., EMBO J. 5: 1567-1575, 1986
- Hahn et al, Virology 162: 167-180, 1988
- Ham et al., Meth. Enz. 58:44, 1979
- Ed Harlow and David Lane (editors) Antibodies: A Laboratory Manual, Cold Spring Harbour Laboratory, (1988)
- G. Hartmann and S. Endres, Manual of Antisense Methodology, Kluwer (1999)
- Hellstrom et al, Cancer. Res. 45: 2210-2188, 1985
- Hellstrom et al, Cancer Res. 46: 3917-3923, 1986
- Henttu and Vihko, Biochem. Biophys. Res. Comm. 160(2): 903-910, 1989
- Herlyn et al, J Clin. Immunol 2: 135, 1982
- Hermonat et al., Proc. Natl. Acad. Sci. USA 81: 6466-6470, 1984
- Hilkens et al, Trends in Bio. Chem. Sci. 17: 359, 1992
- Hochberg and Benjamini Stat Med 9, 811-818, 1990
- Hollinger et al, Proc. Natl. Acad. Sci. USA, 90:6444-6448 1993
- Hoogenboom and Winter J Mol Biol, 227:381, 1991
- Hoon et al., Cancer Res. 53: 5244-5250, 1993
- Irizarry et al., Biostatistics 4, 249-264, 2003
- Itoh et al, Nature 308: 19, 1986
- Jakobovits et al., Nature Biotechnology 25, 1134 - 1143 2007
- Jespers et al, Bio/technology 12:899-903, 1988
- Jones et al., Nature, 321:522-625, 1989
- Jones et al., Cancer Immun. 22;2:1, 2002
- Kabat et al., Sequences of Proteins of Immunological Interest, 5th Ed. Public Health Service, National Institutes of Health, Bethesda, MD. (1991)
- Kam et al., PNAS USA 102: 11600-11605 2005
- Kamarch, Methods Enzymol, 151:150-165, 1987
- Kanai et al., Inflamm Bowel Dis., 12: 89-99, 2006
- Kingsley et al, J. Immunol. 168:1080, 2002
- Klein et al., Proc. Natl. Acad. Sci USA, 100: 8886-8891, 2003
- Koenecke et al., Eur J Immunol. 39):3091-3096, 2009
- Kohler and Milstein, Nature 256, 495-497, 1975
- Kohler and Milstein, Eur. J. Immunol. 6, 511-519, 1976
- Kostelny et al, J. Immunol., 148(5):1547-1553 1992
- Kumar et al, Immuno. Letters 65, 153-159, 1999
- Largaespada et al, Curr. Top. Microbiol. Immunol, 166, 91-96. 1990
- Largaespada et al, Oncogene, 7, 811 -819, 1992
- Largaespada et al, J. Immunol. Methods. 197(1-2), 85-95, 1996
- Lee et al., Mol. Immunol. 17, 749-756 1980
- Levine et al., J. Immunol., 159: 5921-5930, 1997
- Lindmark et al., J Immunol Meth. 62: 1 -13, 1983
- Liu et al., Scand. J. Immunol., 59: 198-202, 2004
- Livingston et al., , JClin. Oncol. 12: 1036-1044, 1994
- Longhi et al., J. Hepatol., 41: 31-37, 2004
- Lonberg et al., Nature 368 (1994): 856-859;
- Lonberg, N. "Transgenic Approaches to Human Monoclonal Antibodies." Handbook of Experimental Pharmacology 113 (1994): 49-101
- Lukacs et al., J. Exp. Med., 194: 551-555, 2001
- Macardle and Bailey Cell Biology: A Laboratory Handbook. Chapter 57: Preparation of Monoclonal Antibodies. 2006;
- Marks et al, J. Mol. Biol., 222:581-597 (1991)
- Marks et al, Bio/Technology, 10:779-783 (1992)
- Mather Biol. Reprod. 23:243-251, 1980
- Mather et al., Annals N. Y. Acad. Sci. 383:44-68, 1982
- Mavrangelos et al., J Immunol Methods; 289:169-178, 2004
- McCafferty et al, Nature, 348:552-654 (1990)
- McHugh et al., J. Immunol., 168: 5979-5983, 2002
- Mendoza et al., Biotechniques 27(4): 778-788, 1999
- Messing Methods Enzymol, 101, 20-78, 1983
- Millstein et al, Nature, 305:537-539, 1983
- Miltenyi et al., Cytometry 11:231-238, 1990
- Morrison et al., Proc. Natl Acad. Sci USA 81:6851-6855, 1984
- Morrison, Science 229:1202, 1985
- Mottet et al., J. Immunol., 170: 3939-3943, 2003
- Nakamura et al, J. Exp. Med. 194:629-644, 2001
- Narang, et al., Meth. Enzymol 68: 90, 1979
- Narang, editor, "Synthesis and Applications of DNA and RNA," Academic Press, New York, 1987
- Natali et al, Cancer 59: 55-63, 1987
- Needleman and Wunsch, J. Mol. Biol. 48, 443-453, 1970
- Nelson et al., Electrophoresis 21: 1155-1163, 2000
- Nicolau and Sene, Biochim. Biophys. Acta, 721: 185-190,1982
- Nielsen et al, J. Chem. Soc. Perkin Trans., 1: 3423, 1997
- Novellino et al., Cancer Immunol Immunother. 54(3):187-207, 2005
- Okazaki et al., JMB, 336: 1239-49, 2004
- Oi et al, BioTechniques 4:214, 1986
- Orum et al., Nucl. Acids Res., 21: 5332, 1993
- Perbal, A Practical Guide to Molecular Cloning, John Wiley and Sons (1984)
- Perez and Walker, J. Immunol. 142: 3662-3667, 1990
- Perikles Bioinformatics 19: 1439-1440, 2003
- Pluckthun, Immunol. Revs., 130:151-188 (1992)
- Presta Curr Op Struct Biol, 2:593-59, 1992
- Putney et al, Science 234: 1392-1395, 1986
- Queen et al., Proc. Natl Acad. Sci. USA 86: 10029 10033, 1989
- Ragnhammar et al, Int. J. Cancer 53: 751-758, 1993
- Reff et al, Blood 83: 435-445, 1994
- Riechmann et al., Nature, 332:323-329, 1988
- Rippe et al., , Mol. Cell Biol., 10: 689-695,1990
- Rosenfeld et al., Science 252: 431-434, 1991
- Rosenfeld et al., Cell 68: 143-155, 1992
- Rota et al, Virology 188: 135-142, 1982
- Sakaguchi et al., J. Immunol., 155: 1151-1164, 1995
- Sakaguchi et al, Immunol. Rev. 182:18- 32, 2001
- Sakaguchi et al., Nature, 426: 454-460
- Saleh et al, J.Immunol., 151, 3390-3398, 1993
- Salomon et al, Immunity 12:431-440, 2000
- Sambrook et al., Molecular Cloning: A Laboratory Manual, Cold Spring Harbour Laboratory Press (1989)
- Santa Lucia, Proc. Natl. Acad. Sci. USA, 95: 1460-1465, 1995
- Scopes (In: Protein purification: principles and practice, Third Edition, Springer Verlag, 1994)
- Sgouros et al, J Nucl. Med. 34: 422-430, 1993
- Shalaby et al, J. Exp. Med., 175: 217-225 1992
- Shitara et al., , Cancer Immunol. Immunother. 36: 373- 380, 1993
- Shevach, Annu. Rev. Immunol. 18:423-449, 2000
- Shevach, Nature Rev. Immunol 2:389, 2002
- Shield et al, 2002, J Biol Chem 277:26733-26740
- Shinkawa et al., 2003, J Biol Chem 278:3466-3473
- Shopes, B. J. Immunol. 148:2918-2922 1992
- Singh and Wengel, Chem. Commun. 1247, 1998
- Skerra et al, Curr. Opinion in Immunol., 5:256-262 (1993)
- Smyth et al., Bioinformatics (Oxford, England) 21, 2067-2075, 2005
- Spandidos et al.,_BMC Genomics, 9:633, 2008
- Suresh et al, Methods in Enzymology, 121:210, 1986
- Suri-Payer et al, J. Immunol. 157:1799-1805, 1996
- Suri-Payer et al., J. Immunol., 160: 1212-1218, 1998
- Stephens et al, Eur. J. Immunol. 31:1247-1254, 2001
- Stevenson et al., Anti- Cancer Drug Design 3:219-230 1989
- Taams et al, Eur. J. Immunol. 31:1122-1131, 2001
- Tailor et al, Nucl. Acids Res. 18(16): 4928, 1990
- Tang et al., J. Exp. Med., 199: 1455-1465,2004
- Thompson et al, Nucl. Acids Res. 22, 4673-4680, 1994
- Tomizuka et al., Proceedings of the National Academy of Sciences USA 97(2) (2000): 722-727
- Tratschin et al., Mol. Cell. Biol. 5: 3251-3260, 1985
- Tratschin et al., J. Virol. 51: 611-619, 1984
- Traunecker et al, EMBO J., 10:3655-3659 1991
- Trenado et al., J. Clin.lnvest., 112: 1688-1696, 2002
- Tur-Kaspa et al., , Mol. Cell Biol., 6: 716-718, 1986
- Tutt et al, J. Immunol. 147: 60 (1991)
- Umana et al, , Nat. Biotechnol 17:176-180, 1999
- Urlaub et al., Proc. Natl. Acad. Sci USA 77:4216, 1980
- Vacanti, et al., J. Ped. Surg. 23:3-9 1988
- Vacanti, et al., Plast. Reconstr. Surg. 88:753-9 1991
- Van der Sluis et al., Gastroenterology 131: 117-129, 2006
- Van Maurik J. Immunol. 169: 5401-5404, 2002
- Venken et al., J. Immunol. Methods, 322: 1-11, 2007
- Verhoeyen et al., Science, 239:1534-1536, 1988
- Vijayasardahl et al, J Exp. Med. 171(4): 1375-1380, 1990
- Wang and Seed Nucleic Acids Res 31, e 154, (2003)
- Wang et al., Immunity 20: 107-118, 2004
- Wang et al., J Clin Invest. 118(7): 2629-2639, 2008
- Waterhouse et al, Nuc. Acids. Res., 21 :2265-2266 (1993)
- Weissinger et al., Proc. Natl. Acad. Sci USA, 88, 8735-8739, 1991
- Weissinger et al, J. Immunol. Methods 168, 123-130, 1994
- Wettenhall and Smyth Bioinformatics (Oxford, England) 20, 3705-3706, 2004
- Willerford et al, Immunity 3:521-530, 1995
- Wing and Sakaguchi, Nature Immunology, 11: 7-13, 2010;
- Wolff et al., Cancer Research 53:2560-2565 1993
- Wondisford et al., Mol. Endocrinol. 2: 32-39, 1988
- Wu and Wu, J. Biol. Chem., 262: 4429-4432, 1987
- Yang et al., , Proc. Natl Acad. Sci USA, 87: 9568-9572,1990
- Yaniv Nature 297: 17-18, 1982
- Yokata et al, Cancer Res. 52: 3402-3408, 1982
- Yu et al, Cancer Res. 51(2): 468-475, 1991
- Zola (Ed), "Monoclonal Antibodies: A Manual of Techniques", CRC Press, 1987.

## Claims

1. An *in vitro* method for detecting a regulatory T (Treg) cell (i.e., cells expressing CD4, CD25 and FoxP3) comprising determining the level of expression of a nucleic acid encoding PI16 or a PI16 protein in/on a T cell in a sample, wherein an increased level of expression of the nucleic acid or protein compared to another cell type is indicative of a Treg cell.

2. The method of claim 1 additionally comprising determining the level of expression of an additional nucleic acid or protein set forth in Table 2 and/or Table 3 in/on a T cell, wherein an increased level of expression of a nucleic acid or protein set forth in Table 2 compared to another cell type, or a reduced level of expression of a nucleic acid or protein set forth in Table 3 compared to another cell type is indicative of a Treg cell.

3. The method according to claim 2, wherein the additional protein is selected from the group consisting of or the nucleic acid encodes a protein selected from the group consisting of WDFY4,
AQP3, PTPRB, PLXNB2, PKD1L3, LSR, RGMB, EPHB1, TMEM169 and NPAL2.

4. The method according to any one of claims 1 to 3 additionally comprising isolating the detected Treg cell from the sample.

5. The method of claim 4 additionally comprising formulating the isolated Treg cell into a pharmaceutical composition.

6. The method according to any one of claims 1 to 3, further comprising diagnosing and/or
prognosing a regulatory T (Treg) cell-associated condition in a subject wherein detection of Treg cell(s) or failure to detect Treg cells in a sample from a subject indicates that the subject suffers from a Treg-associated condition wherein detection of Treg cell(s) or failure to detect Treg cells indicates that the subject suffers from a Treg-associated condition.

7. The method according to claim 6 additionally comprising:
(i) determining or estimating the number of Treg cells in the sample or a portion thereof;
(ii) comparing the number of Treg cells at (i) to the number of Treg cells in a sample from a normal and/or healthy subject;
wherein an increased or decreased number of Treg cells at (i) compared to the number of Treg cells in a sample from a normal and/or healthy subject indicates that the subject suffers from a Treg-associated condition.

8. The method according to claim 6 or 7, wherein the Treg cell-associated condition is an autoimmune condition or inflammation or cancer.

9. An isolated population of cells enriched for regulatory T (Treg) cells (i.e., cells expressing CD4,
CD25 and FoxP3) expressing nucleic acid encoding PI16 or PI16 protein.

10. The isolated population of cells of claim 9 expressing one or more additional the nucleic acids or proteins set forth in Table 2 and/or 4 and/or expressing a reduced level of one or more of the nucleic acids or proteins set forth in Table 3.

11. The isolated population of cells of claim 9 or 10 expressing CD4 and CD25, and expressing low or undetectable levels of CD127.

12. The isolated population of cells of any one of claims 9 to 11, wherein the Treg cells are natural Treg cells and/or memory Treg cells.

13. An isolated population of cells according to any one of claims 9 to 12 or a population of cells enriched for Treg cells isolated by performing the method according to claim 4 or 5 for use in treating or preventing a condition associated with reduced regulatory T (Treg) cell numbers or activity, and/or inducing immunosuppression, and/or reducing CTL or Thelper cell activity in a subject.

14. The isolated population for use according to claim 13, wherein the Treg cell-associated condition is an autoimmune condition or inflammation or cancer.

## Patentansprüche

1. In-vitro-Verfahren zur Detektion einer regulierenden T- (Treg-) Zelle (das sind Zellen, die CD4, CD25 und FoxP3 exprimieren), das das Bestimmen des Ausmaßes des Expression einer Nucleinsäure, die für PI16 kodiert, oder eines PI16-Proteins in/auf einer T-Zelle in einer Probe umfasst, worin ein höheres Ausmaß der Expression der Nucleinsäure oder des Proteins als bei einem anderen Zelltyp ein Indikator für eine Treg-Zelle ist.

2. Verfahren nach Anspruch 1, das zusätzlich das Bestimmen des Ausmaßes der Expression einer zusätzlichen Nucleinsäure oder eines zusätzlichen Proteins, die in Tabelle 2 und/oder Tabelle 3 dargelegt sind, in/auf einer T-Zelle umfasst, worin ein höheres Ausmaß der Expression der Nucleinsäure oder des Proteins, die in Tabelle 2 dargelegt sind, als bei einem anderen Zelltyp oder ein geringeres Ausmaß der Expression der Nucleinsäure oder des Proteins, die in Tabelle 3 dargelegt sind, als bei einem anderen Zelltyp ein Indikator für eine Treg-Zelle ist.

3. Verfahren nach Anspruch 2, worin das zusätzliche Protein aus der aus WDFY4, AQP3, PTPRB, PLXNB2, PKD1L3, LSR, RGMB, EPHB1, TMEM169 und NPAL2 bestehenden Gruppe ausgewählt ist oder die Nucleinsäure für ein aus dieser Gruppe ausgewähltes Protein kodiert.

4. Verfahren nach einem der Ansprüche 1 bis 3, das zusätzlich das Isolieren der detektierten Treg-Zelle aus der Probe umfasst.

5. Verfahren nach Anspruch 4, das zusätzlich das Formulieren der isolierten Treg-Zelle zu einer pharmazeutischen Zusammensetzung umfasst.

6. Verfahren nach einem der Ansprüche 1 bis 3, das ferner das Diagnostizieren und/oder Prognostizieren eines mit einer regulierenden T- (Treg-) Zelle verbundenen Leidens bei einem Individuum umfasst, worin die Detektion von einer oder mehreren Treg-Zellen in einer Probe von einem Individuum oder die Tatsache, dass darin keine Treg-Zellen detektiert wurden, ein Indikator dafür ist, dass das Individuum an einem mit Treg verbundenen Leiden leidet, worin die Detektion von einer oder mehreren Treg-Zellen in einer Probe von einem Individuum oder die Tatsache, dass keine Treg-Zellen detektiert wurden, ein Indikator dafür ist, dass das Individuum an einem mit Treg verbundenen Leiden leidet.

7. Verfahren nach Anspruch 6, das zusätzlich Folgendes umfasst:
(i) das Bestimmen oder Schätzen der Anzahl an Treg-Zellen in der Probe oder einem Teil davon;
(ii) das Vergleichen der Anzahl an Treg-Zellen nach (i) mit der Anzahl an Treg-Zellen in einer Probe von einem normalen und/oder gesunden Individuum;
worin eine im Vergleich zu der Anzahl an Treg-Zellen in einer Probe von einem normalen und/oder gesunden Individuum höhere oder geringere Anzahl an Treg-Zellen nach (i) ein Indikator dafür ist, dass das Individuum an einem mit Treg verbundenen Leiden leidet.

8. Verfahren nach Anspruch 6 oder 7, worin das mit Treg-Zellen verbundene Leiden ein Autoimmunleiden, eine Entzündung oder Krebs ist.

9. Isolierte Population von Zellen, angereichert an regulierenden T- (Treg-) Zellen (das sind Zellen, die CD4, CD25 und FoxP3 exprimieren), die Nucleinsäure, die für PI16 kodiert, oder PI16-Protein exprimieren.

10. Isolierte Population von Zellen nach Anspruch 9, die eine oder mehrere zusätzliche Nucleinsäuren oder ein oder mehrere Proteine, die in Tabelle 2 und/oder 4 dargelegt sind, exprimiert und/oder die eine reduzierte Menge einer oder mehrerer der Nucleinsäuren oder eines oder mehrerer der Proteine, die in Tabelle 3 dargelegt sind, exprimiert.

11. Isolierte Population von Zellen nach Anspruch 9 oder 10, die CD4 und CD25 exprimieren und die geringe oder undetektierbare Ausmaße an CD127 exprimieren.

12. Isolierte Population von Zellen nach einem der Ansprüche 9 bis 11, worin die Treg-Zellen natürliche Treg-Zellen und/oder Gedächtnis-Treg-Zellen sind.

13. Isolierte Population von Zellen nach einem der Ansprüche 9 bis 12 oder um Treg-Zellen angereicherte Population von Zellen, die durch Anwendung eines Verfahrens nach Anspruch 4 oder 5 isoliert wurden, zur Verwendung bei der Behandlung oder Prävention eines mit reduzierten regulierenden T- (Treg-) Zellanzahlen oder reduzierter Treg-Zellenaktivität verbundenen Leidens und/oder bei der Auslösung von Immunsuppression und/oder bei der Reduktion von CTL- oder T-Helferzellen-Aktivität bei einem Individuum.

14. Isolierte Population zur Verwendung nach Anspruch 13, worin das mit Treg-Zellen verbundene Leiden ein Autoimmunleiden, eine Entzündung oder Krebs ist.

## Revendications

1. Méthode *in vitro* pour détecter une cellule T régulatrice (Treg), (c'est-à-dire des cellules exprimant CD4, CD25 et FoxP3) comprenant la détermination du niveau d'expression d'un acide nucléique codant PI16 ou une protéine PI16 dans/sur une cellule T dans un échantillon, où un niveau accru d'expression de l'acide nucléique ou de la protéine comparé à un autre type de cellule est indicatif d'une cellule Treg.

2. Méthode selon la revendication 1, comprenant de plus la détermination du niveau d'expression d'un acide nucléique ou protéine additionnel exposé dans le Tableau 2 et/ou le Tableau 3 dans/sur une cellule T, où un niveau accru d'expression d'un acide nucléique ou protéine exposé dans le Tableau 2 comparé à un autre type de cellule ou bien un niveau réduit d'expression d'un acide nucléique ou d'une protéine exposé dans le Tableau 3 comparé à un autre type de cellule est indicatif d'une cellule Treg.

3. Méthode selon la revendication 2, dans laquelle la protéine additionnelle est sélectionnée dans le groupe consistant en, ou l'acide nucléique code pour une protéine sélectionnée dans le groupe consistant en WDFY4, AQP3, PTPRB, PLXNB2, PKD1L3, LSR, RGMB, EPHB1, TMEM169 et NPAL2.

4. Méthode selon l'une quelconque des revendications 1 à 3, comprenant de plus l'isolation de la cellule Treg détectée de l'échantillon.

5. Méthode selon la revendication 4, comprenant de plus la formulation de la cellule Treg isolée en une composition pharmaceutique.

6. Méthode selon l'une quelconque des revendications 1 à 3, comprenant en outre le diagnostic et/ou le pronostic d'un état associé à une cellule T régulatrice chez un sujet, où la détection d'une ou de cellule(s) Treg ou la non-détection de cellules Treg dans un échantillon d'un sujet indique que le sujet souffre d'un état associé à la Treg où la détection de cellule(s) Treg ou la non-détection de cellules Treg indique que le sujet souffre d'un état associé à la Treg.

7. Méthode selon la revendication 6, comprenant de plus :
(i) déterminer ou estimer le nombre de cellules Treg dans l'échantillon ou une portion de celui-ci ;
(ii) comparer le nombre de cellules Treg à (i) au nombre de cellules Treg dans un échantillon d'un sujet normal et/ou en bonne santé ;
où un nombre accru ou diminué de cellules Treg à (i) comparé au nombre de cellules Treg dans un échantillon d'un sujet normal et/ou en bonne santé indique que le sujet souffre d'un état associé à la Treg.

8. Méthode selon la revendication 6 ou 7, dans laquelle l'état associé à la cellule Treg est une condition auto-immune ou une inflammation ou un cancer.

9. Population isolée de cellules enrichies pour des cellules T régulatrices (Treg) (c'est-à-dire des cellules exprimant CD4, CD25 et FoxP3) exprimant l'acide nucléique codant pour PI16 ou la protéine PI16.

10. Population isolée de cellules de la revendication 9, exprimant un ou plusieurs acides nucléiques ou protéines additionnels exposés dans le Tableau 2 et/ou 4 et/ou exprimant un niveau réduit d'un ou de plusieurs des acides nucléiques ou protéines exposés dans le Tableau 3.

11. Population isolée de cellules de la revendication 9 ou 10 exprimant CD4 et CD25, et exprimant des niveaux bas ou non détectables de CD127.

12. Population isolée de cellules selon l'une quelconque des revendications 9 à 11, où les cellules Treg sont des cellules Treg naturelles et/ou des cellules Treg de mémoire.

13. Population isolée de cellules selon l'une quelconque des revendications 9 à 12 ou une population de cellules enrichies pour des cellules Treg isolées en exécutant la méthode selon la revendication 4 ou 5 pour utilisation dans le traitement ou la prévention d'une condition associée à un nombre de cellules T régulatrices (Treg) ou activité réduit, et/ou induisant l'immuno-suppression, et/ou la réduction de l'activité CTL ou de cellule Thelper chez un sujet.

14. Population isolée pour utilisation selon la revendication 13, où la condition associée à la cellule Treg est une condition auto-immune ou une inflammation ou un cancer.
